# EUROPEAN PATENT SPECIFICATION

(11) **EP 4 320 124 B1**
(45) Date of publication and mention of the grant of the patent: **17.06.2026**
(21) Application number: 22721082.0
(22) Date of filing: 07.04.2022
(51) Int. Cl.: C07D 421/12, C07D 421/14, C07D 517/04, A61P 9/10, A61P 29/00, A61P 35/00, A61P 37/00

(54) **DICHALCOGENIDE PRODRUGS**
DICHALCOGENID-PRODRUGS
PROMÉDICAMENTS DE DICHALCOGÉNURE

(30) Priority: 07.04.2021 EP 21167187
(43) Date of publication of application: 14.02.2024
(73) Proprietor: Ludwig-Maximilians-Universität München, 80539 München (DE)
(72) Inventor: THORN-SESHOLD, Oliver, 81375 München (DE); ZEISEL, Lukas, 81373 München (DE); FELBER, Jan, 80337 München (DE)
(74) Representative: Vossius & Partner Patentanwälte Rechtsanwälte mbB
(86) International application number: PCT/EP2022/059280
(87) International publication number: WO 2022/214598

(56) References cited:
- US-A1- 2017 037 401
- FELBER JAN G ET AL: "Cyclic 5-membered disulfides are not selective substrates of thioredoxin reductase, but are opened nonspecifically by thiols", CHEMRXIV, 24 December 2020 (2020-12-24), pages 1 - 10, XP055828957, Retrieved from the Internet <URL:https://chemrxiv.org/engage/api-gateway/chemrxiv/assets/orp/resource/item/60c75361bb8c1a8a533dc01e/original/cyclic-5-membered-disulfides-are-not-selective-substrates-of-thioredoxin-reductase-but-are-opened-nonspecifically-by-thiols.pdf> [retrieved on 20210729]
- MAFIREYI TENDAI J. ET AL: "A Diselenide Turn-On Fluorescent Probe for the Detection of Thioredoxin Reductase", vol. 59, no. 35, 17 June 2020 (2020-06-17), DE, pages 15147 - 15151, XP055808960, ISSN: 1433-7851, Retrieved from the Internet <URL:https://onlinelibrary.wiley.com/doi/full-xml/10.1002/anie.202004094> DOI: 10.1002/anie.202004094
- JARKKO RAUTIO ET AL: "Prodrugs: design and clinical applications", NATURE REVIEWS DRUG DISCOVERY, vol. 7, no. 3, 1 March 2008 (2008-03-01), GB, pages 255 - 270, XP055227338, ISSN: 1474-1776, DOI: 10.1038/nrd2468

## Description

### Technical Field

The present application relates to a compound having the formula (I). The compound having the formula (I) is capable of releasing a molecular cargo in the presence of a reductant and is thus suitable for diagnosing or quantifying the presence or activity of the reductant; and for treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury.

In particular, the molecular cargo can be a therapeutic or diagnostic agent. In particular, the reductant can be a biological oxidoreductase and/or its redox effector protein which may be catalytically active. The invention further relates to a pharmaceutical or diagnostic composition comprising the same.

### Prior Art

Dithiol/disulfide-exchange redox reactions are critical in a great number of biological pathways. Often, these are coordinated through conserved, specialised networks of oxidoreductases that perform redox reactions upon or using disulfides and/or thiols. The thioredoxin reductase - thioredoxin (TrxR-Trx) system, and the glutathione reductase - glutathione - glutaredoxin (GR-GSH-Grx) system, are two of the central redox systems; other key reductive enzymes/proteins include but are not limited to peroxiredoxins, glutathione peroxidases, glutathione-S-transferases, methionine sulfoxide reductase, and protein disulfide isomerases (PDIs) (Lee, S. et al. Antioxid Redox Signal 2013, 18, 1165-1207. https://doi.org/10.1089/ars.2011.4322). Such redox systems drive reactions vital to cellular metabolism, and can also regulate protein activity, protein-protein interactions, and protein localisation by reversible dithiol/disulfide-type reactions (Jones, D. P. et al. Antioxidants & Redox Signaling 2015, 23, 734-746. https://doi.org/10.1089/ars.2015.6247).

To better investigate and understand biological processes, it is of great interest to develop specific methods to image or quantify the activity of biological species, or otherwise to respond to their activity. Therefore, it was an object of the present invention to provide compounds which have high selectivity for an oxidoreductase or redox effector protein, and which can be reduced by the disulfide reductive activity of that enzyme and thus release a molecular cargo. This has been a longstanding goal of research in the field of cellular dithiol/disulfide-exchange redox reactions. In this field, a significant challenge for chemical probe development is to ensure that a probe is specific for being reduced by the targeted species and is not significantly reduced by any other reducing species in the cell. Therefore, to selectively probe one of the species TrxR, GR, Trx, or Grx, a compound must not be activated by any of the others or by GSH. This is difficult because these reductants can perform similar chemical reactions, and because the most active of these reductants have the lowest cellular concentrations (TrxR and GR have nM cellular concentrations; Trx and Grx have µM concentrations) while the least active reductant GSH is the most concentrated (mM).

These reducing species all act upon disulfides, so most reduction-activated chemical probes in the prior art have used disulfides as reduction-sensing motifs. Such disulfides have been reviewed elsewhere (see Felber, J. et al. ChemRxiv 2020, https://doi.org/10.26434/chemrxiv.13483155.v1; Felber, J. et al. ChemRxiv 2021, https://doi.org/10.26434/chemrxiv.14270444; and references therein).

Probes based on dichalcogenides other than disulfides (such as selenenylsulfides -RSeSR-, and diselenides -RSeSeR-) are very rare in the literature. The reports that exist do not teach towards features that determine their reductant selectivity, and the performance reported for the compounds that do exist is often contradictory or inconclusive. The prior art in cargo-releasing dichalcogenide reduction probes are **E1-E2:**

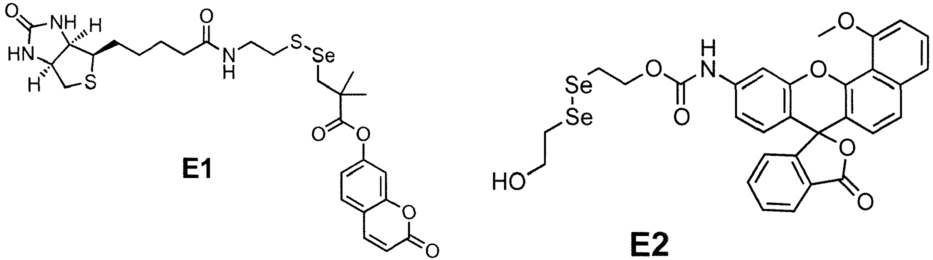

Suarez *et al.* reported linear selenenylsulfide-based reductively-activated fluorogenic probe **E1** (Suarez; Chemistry - A European Journal 2019 25, 15736-15740). This probe was stated to be a biological sensor for H₂S, developed to have "remarkable reactivity and selectivity" for H₂S. **E1** was not tested against any redox enzymes or proteins, nor were controls run for thiolysis of the phenolic ester by 50 µM Na₂S. The report also teaches a linear topology of the selenenylsulfide.

Mafireyi *et al.* reported linear diselenide-based reductively-activated fluorogenic probe **E2** (Mafireyi; Angewandte Chemie International Edition 2020 59, 15147-15151). This is an aniline carbamate linear diselenide (therefore will have slow rates of cyclisation-driven release due to the poor leaving-group nature of the aniline). While it was claimed as selective for TrxR1, no other redox proteins were actually tested; and in fact a low concentration of GSH gave strong signal turn-on within 20 minutes (**Fig S1e** in that paper). Therefore this report does not teach towards cyclic dichalcogenides; also it does not teach towards phenol-releasing probes; and it does not teach about stability against GSH or how to ensure it.

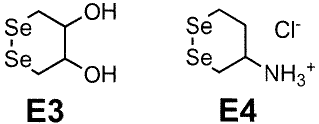

The molecules **E3** and **E4** were reported by Li (Li et al.; Nature Communications 2019 10, 2745) and were investigated for their reduction in an *in vitro* screen to provide a far-limit case as part of investigations into interactions between disulfides and TrxR. It was reported that **E4** was resistant to TrxR, and that **E3** was processed by TrxR, therefore the report teaches that there is no clear relationship between structure and capacity to act as a TrxR substrate in a cell-free setting ("the reduction... is a little bit complicated"). The molecules **E3-E4** are completely reversibly reducible/oxidisable, and therefore do not indicate how an irreversibly-triggered molecule would perform in this assay; also there is no suggestion that such six-membered cyclic diselenides could be applied in cells for any purpose. This report also mentions that phenol-releasing carbamates (TRFS2 in that report) "hydrolyzed spontaneously in aqueous buffer,".

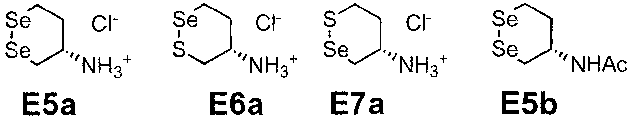

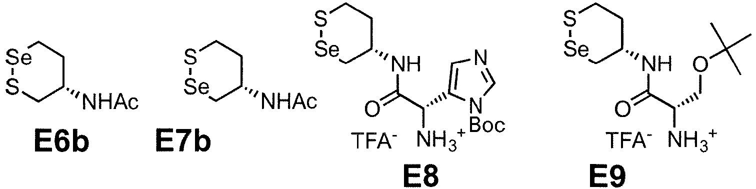

Cyclic 6-membered selenenylsulfides that are known in the literature are reported by Arai et *al.* (Arai et al.; Chem. Eur. J. 2019 25, 12751-12760), including the interrelated series of molecules **E6a, E6b, E7a, E7b, E8, E9,** as well as diselenides **E5a, E5b.** These molecules were intended as *mimics* of the SecCys active site of mammalian TrxR, that could be studied by chemical methods to gain understanding of the mechanism by which TrxR operates as an enzyme. Again it will be noted that the molecules are completely reversibly reducible/oxidisable.

Therefore there remain numerous unsolved challenges, including to: (i) determine what, if any, utility dichalcogenide species other than disulfides can have in a biological setting; (ii) design probe or prodrug systems that release a molecular cargo upon reductive triggering and are selective for being triggered by a particular cellular reductant, i.e. are shown to resist reduction by the other major cellular reductants (e.g. GSH, TrxR, Trx, Grx, or GR etc. as appropriate); (iii) ensuring that the probe/prodrug design is hydrolytically stable; and (iv) designing probe or prodrug systems that release a molecular cargo upon reductive triggering and are selective for being triggered under pathological conditions. If these challenges can be solved, highly valuable probes or prodrugs to report on the activity of those reductants, or to respond to this activity by releasing a drug, would be created.

Under pathological conditions, homeostasis in these redox systems and in their target proteins is significantly dysregulated. This has been particularly shown in diseases such as cancer, inflammatory diseases such as autoimmune disorders, under acute stress such as reperfusion injury, and under other chronic conditions such as cardiovascular disease (Mohammadi, F. et al. Cancer Chemotherapy and Pharmacology 2019. https://doi.org/10.1007/s00280-019-03912-4, Whayne, T. F. et al. Can. J. Physiol. Pharmacol. 2015, 93, 903-911. https://doi.org/10.1139/cjpp-2015-0105). Such dysregulation has also been studied in the context of disease-associated biomarkers, such as hypoxia. Hypoxia is a pathology-associated feature, prevalent in cancer and inflammation, that is tightly correlated with significant biochemical changes including oxidoreductase dysregulation, many of which rely on the hypoxia-dependent transcription factor HIF-1. Oxidoreductase or redox effector protein dysregulation may be reflected in a combination of changes to their expression level, enzymatic activity, redox poise (the ratio of oxidised to reduced form of the oxidoreductase or redox effector protein), localisation, and/or other parameters. For example, under pathological conditions, the TrxR-Trx and GR-GSH-Grx systems act as repair systems, counteracting acute cellular damage by oxidants, and re-normalising redox imbalances e.g. from oxygen depletion (hypoxia) or from the switching of metabolic pathways due to cellular stress (e.g. the Warburg Effect in cancer; Arnér, E. S. J. et al. Seminars in Cancer Biology 2006, 16, 420-426. https://doi.org/10.1016/j.semcancer.2006.10.009, Karlenius, T. C. et al. Cancers 2010, 2, 209-232. https://doi.org/10.3390/cancers2020209); therefore cells affected by pathologies that require such repair and renormalisation are often found to upregulate expression levels and activity of these oxidoreductase and redox effector protein systems, and shift their redox poise (Jiang, J. et al. Nanoscale 2014, 6, 12104-12110. https://doi.org/10.1039/C4NR01263A). This dysregulation has been particularly studied in the context of cancer, where for the example of Trx it has been shown that cycling hypoxia, which is a hallmark of most tumors, upregulates Trx expression levels (Karlenius, T. C. et al. Biochemical and Biophysical Research Communications 2012, 419, 350-355. https://doi.org/10.1016/j.bbrc.2012.02.027); that Trx overexpression contributes to hallmarks of cancer including increased proliferation and angiogenesis, and evasion of apoptosis (Powis, G. et al. Current Opinion in Pharmacology 2007, 7, 392-397. https://doi.org/10.1016/j.coph.2007.04.003); and that Trx overexpression in tumors contributes to chemoresistance and is associated to poor patient survival (Biaglow, J. E. et al. Cancer Biology & Therapy 2005, 4, 13-20. https://doi.org/10.4161/cbt.4.1.1434). Dysregulated function is also particularly studied in the context of inflammatory diseases, since many central players in inflammation (NFκB, TNF-α, Keap1, Nrf2) are regulated by these oxidoreductases or redox effector proteins, and processes from inflammasome activation to immune cell chemotaxis have also been shown to depend on these oxidoreductases or redox effector proteins (Bertini, R. et al. J Exp Med 1999, 189, 1783-1789. https://doi.org/10.1084/jem.189.11.1783, Yoshihara, E. et al. Front Immunol 2014, 4, 514-514. https://doi.org/10.3389/fimmu.2013.00514).

The disease-correlated nature of these dysregulated states of oxidoreductases and redox effector proteins - particularly of Trx, TrxR, and PDIs - makes them promising targets for diagnostic or therapeutic applications using diagnostic or therapeutic drugs that target these oxidoreductases or redox effector proteins. For example, the TrxR-inhibiting drug auranofin is FDA-approved for use in the inflammatory disease, rheumatoid arthritis (Lee, S. et al. Antioxid Redox Signal 2013, 18, 1165-1207. https://doi.org/10.1089/ars.2011.4322; Arnér, E. S. J. et al. Seminars in Cancer Biology 2006, 16, 420-426. https://doi.org/10.1016/j.semcancer.2006.10.009). In this context, a probe or prodrug system to release a molecular cargo, that is selectively triggered by one of these oxidoreductases or redox effector proteins, would be very valuable. Similarly, substantial efforts have been made to use the disease-correlated feature, hypoxia, both as a diagnostic marker and for therapeutic exploitation, for example by using diagnostic and therapeutic drugs that target hypoxia, which has been extensively reviewed (Airley, R. et al. The Pharmaceutical Journal 2000, 264, 666-673; Phillips, R. M. Cancer Chemotherapy and Pharmacology 2016, 77, 441-457. https://doi.org/10.1007/s00280-015-2920-7).

Therefore it was a further object of the present invention to provide compounds which may have high selectivity for an oxidoreductase or redox effector protein in a pathologically dysregulated state, and can be reduced by the disulfide reductive activity of that oxidoreductase or effector protein and thus release a molecular cargo after reduction. It was a further object of the present invention to provide compounds which may have high selectivity for pathologically dysregulated redox states and/or for cells in hypoxia and/or cells with an activated hypoxic response pathway.

In conclusion therefore, objects of the present invention were to provide compounds which are capable of releasing a molecular cargo after reductive activation, and which
(A) are suitable for diagnosing, quantifying, or responding to reductive activity of one or more selected oxidoreductases or redox effector proteins; and/or
(B) are suitable for diagnosing, treating, ameliorating or preventing a disorder associated with redox activity; and/or
(C) are suitable for diagnosing, treating, ameliorating or preventing a disorder selected from a neoplastic disorder; atherosclerosis; an autoimmune disorder; an inflammatory disease; a chronic inflammatory autoimmune disease; ischaemia; and reperfusion injury, particularly cancer; and/or
(D) are suitable for diagnosing, treating, ameliorating or preventing a disorder associated with a dysregulated redox state and/or with hypoxia and/or with activation of the hypoxic response pathway.

Note that it is possible, and in many cases it is desirable, that one or more of these objectives should be fulfilled by the same compound; for example, a single compound of the invention may fulfil objectives A and B simultaneously; while another compound of the invention may fulfil objectives A, B, C and D simultaneously, etc.

### Summary of the Invention

The invention is summarized in the following items:
1. A compound having the formula (I)

   A-L-B (I)

   wherein
   A is represented by 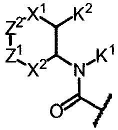
   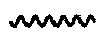 denotes the attachment point of A to L;
   L is a bond or a self-immolative spacer according to item 4;
   B is represented by 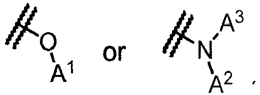
   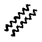 denotes the attachment point of B to L;
   A¹ is selected such that A¹-OH is a therapeutic, diagnostic or theranostic agent which contains an -OH group that is attached to a 5- or 6-membered aromatic or heteroaromatic ring;
   A² and A³ are independently selected such that A²-NH-A³ is a therapeutic, diagnostic or theranostic agent which contains an -NH₂ or -NH- moiety;
   K¹ is selected from -C₁₋₄-alkyl optionally substituted by W¹;
   K² is selected from -H, -O-R^{j}, and -O-R^{k}; or
   K¹ and K² are bonded together and K¹ and K² represent -X³-Y-X-, wherein X³ is bonded to N and X is bonded to C;
   X is selected from -N(R^{a})-, -N(R^{b})-, -CR^{c}₂- and -O-;
   X¹ is -(CR^{d}₂)ₘ-;
   X² is -(CR^{e}₂)ₙ-;
   X³ is -CR^{f}₂-;
   Y is -(CR^{g}₂)ₚ-;
   Z¹ and Z² are independently selected from S or Se such that either Z¹ is Se and Z² is S, or Z¹ is S and Z² is Se, or Z¹ and Z² are both Se;
   W is independently from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or - (C₂₋₄-alkylene)- group via the N atom;
   W¹ is independently selected from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or -(C₂₋₄-alkylene)- group via the N atom;
   R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W;
   R^{b} is an acyl group of a monopeptide selected from -proteinogenic amino acids attached via a carboxy group;
   R^{c} groups are independently selected from -H and -C₁₋₄-alkyl;
   R^{d} groups are independently selected from -H and -C₁₋₄-alkyl;
   R^{e} groups are independently selected from -H and -C₁₋₄-alkyl;
   R^{f} groups are independently selected from -H and -C₁₋₄-alkyl;
   R^{g} groups are independently selected from -H and -C₁₋₄-alkyl;
   R^{h} is independently selected from -H, -C₁₋₄-alkyl and -CH₂CH₂OH;
   Rⁱ is independently selected from -H, -C₁₋₄-alkyl and -CH₂CH₂OH;
   R^{j} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W¹,
   R^{k} is an acyl group of a monopeptide selected from -proteinogenic amino acids attached via a carboxy group;
   R^{x} groups are independently selected from phenyl- and 4-methoxyphenyl-;
   m is 0, 1 or 2;
   n is 1 or 2, provided that m+n is 2 or 3;
   p is 0, 1, or 2, provided that when K¹ and K² are bonded together and K¹ and K² represent -X³-Y-X- and X represents -N(R^{a})- or -N(R^{b})-, then p = 1 or 2; or any pharmaceutically acceptable salt, solvate or ester thereof.
2. The compound according to item 1, wherein either Z¹ is Se and Z² is S, or Z¹ is S and Z² is Se.
3. The compound according to item 1 or 2, wherein L is a bond.
4. The compound according to item 1 or 2, wherein L is a self-immolative spacer selected from 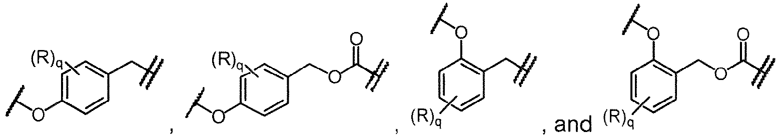 wherein
   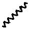 denotes the attachment point to A;
   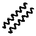 denotes the attachment point to B;
   R is independently selected from halogen, -O(R^{r}), -N(R^{s})(R^{t}), -NO₂, -CN, and a heterocyclic group selected from azetidinyl, pyrrolidinyl, piperidinyl or morpholino, wherein the heterocyclic group is attached to the phenyl ring *via* the N atom;
   q is 0, 1, 2, 3 or 4;
   R^{r} is independently selected from -H, -C₁₋₄-alkyl and -(C₂₋₄-alkylene)-O-(C₁₋₄-alkyl), wherein -C₁₋₄-alkyl or -(C₂₋₄-alkylene)- can be optionally substituted by W;
   R^{s} is independently selected from -H, -C(O)-C₁₋₄-alkyl and -C₁₋₄-alkyl; and
   R^{t} is independently selected from -H, -C(O)-C₁₋₄-alkyl and -C₁₋₄-alkyl.
5. The compound according to item 4, wherein L is a self-immolative spacer selected from 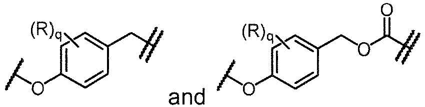 wherein R and q are as defined in item 4.
6. The compound according to any one of items 1 to 5, wherein X¹ and X² are -CH₂-.
7. The compound according to any one of items 1 to 6, wherein K¹ and K² are bonded together and K¹ and K² represent -X³-Y-X-.
8. The compound according to any one of items 1 to 6, wherein K¹ is -C₁₋₄-alkyl optionally substituted by W¹ and K² is -H, -O-R^{j} or -O-R^{k}.
9. The compound according to any one of items 1 to 8, wherein A¹-OH or A²-NH-A³ is selected from a diagnostically acceptable dye, a therapeutically acceptable DNA-alkylating agent, a therapeutically acceptable tubulin-inhibiting agent, and a therapeutically acceptable topoisomerase-inhibiting agent.
10. The compound according to any one of items 1 to 9, wherein A'-OH or A²-NH-A³ is selected from 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-*N,O*-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin γ1, calicheamicin δ1, calicheamicin ε, calicheamicin θ, calicheamicin T, diclofenac, aceclofenac, mefenamic acid, clonixin, piroxicam, meloxicam, tenoxicam, lornoxicam, baricitinib, filgotinib, tofacitinib, upadacitinib, ruxolitinib, peficitinib, decemotinib, solcitinib, itacitinib, fostamatinib, SHR0302, leuco-methylene blue, leuco-methyl methylene blue, leuco-dimethyl methylene blue, leuco-toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methyl-coumarin, 7-amino-4-chloromethylcoumarin, 3-*O*-methylfluorescein, 3-*O*-ethyl-5-carboxyfluorescein, 2,7-difluoro-3-O-methylfluorescein, 3-*N*-acetyl-rhodamine, 3-*N-*acetyl-dimethylsilarhodamine, 2,7-dibromo-3-*N*-acetyl-dimethylcarborhodamine, 3-*N-*acetyl-6-carboxyrhodamine, 2,7-difluoro-3-*N*-acetylrhodol, 3-*O*-(*N*,*N*-dimethyl-2-aminoethyl)-6-carboxyfluorescein, 2,7-dichloro-3-*O*-(*N*,*N*-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, and 6-bromo-2-(5-bromo-2-hydroxyphenyl)quinazolin-4-one.
11. A pharmaceutical or diagnostic composition comprising the compound according to any one of items 1 to 10, or a pharmaceutically acceptable salt, solvate, or ester thereof, and optionally a pharmaceutically acceptable carrier or excipient.
12. The pharmaceutical or diagnostic composition according to item 11, further comprising a second pharmaceutically active agent selected from a vascular disrupting agent, a cytotoxic chemotherapeutic agent and an immunomodulator.
13. The pharmaceutical or diagnostic composition according to item 12, wherein the second pharmaceutically active agent is selected from combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.
14. A compound according to any one of items 1 to 10, or a pharmaceutically acceptable salt, solvate, or ester thereof, for use in medicine.
15. A compound according to any one of items 1 to 10, or a pharmaceutically acceptable salt, solvate, or ester thereof, for use in the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.
16. The compound for use according to item 15, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.
17. Use of a compound according to any one of items 1 to 10, or a pharmaceutically acceptable salt, solvate, or ester thereof, for the manufacture of a medicament for the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury.
18. Use of a compound according to item 17, or a pharmaceutically acceptable salt, solvate, or ester thereof, wherein the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.
21. A method of predicting the suitability of a compound having the formula (I) as defined in any one of items 1 to 10, or a pharmaceutically acceptable salt, solvate, or ester thereof, for treating a patient who is suffering from a disorder selected from a neoplastic disorder; atherosclerosis; an autoimmune disorder; an inflammatory disease; a chronic inflammatory autoimmune disease; ischaemia; and reperfusion injury, wherein the method comprises:
   (i) obtaining a sample from the patient;
   (ii) contacting the sample with a compound having the formula (I) as defined in any one of items 1 to 10, or a pharmaceutically acceptable salt, solvate, or ester thereof, wherein A¹ is selected such that A'-OH is a diagnostic or theranostic agent or A² and A³ are independently selected such that A²-NH-A³ is a diagnostic or theranostic agent; and
   (iii) detecting the presence or absence of A'-OH or A²-NH-A³.
22. An *in vitro* method of determining an inhibitory activity of a candidate inhibitor or candidate drug upon an oxidoreductase and/or a redox effector protein, wherein the method comprises:
   (i) contacting a compound having the formula (I) as defined in any one of items 1 to 8, or a pharmaceutically acceptable salt, solvate, or ester thereof, wherein A¹ is selected such that A¹-OH is a diagnostic or theranostic agent or A² and A³ are independently selected such that A²-NH-A³ is a diagnostic or theranostic agent, with the oxidoreductase and/or the redox effector protein as well as the candidate inhibitor or candidate drug; and
   (ii) detecting the presence or absence of A¹-OH or A²-NH-A³.

### Description of the figures

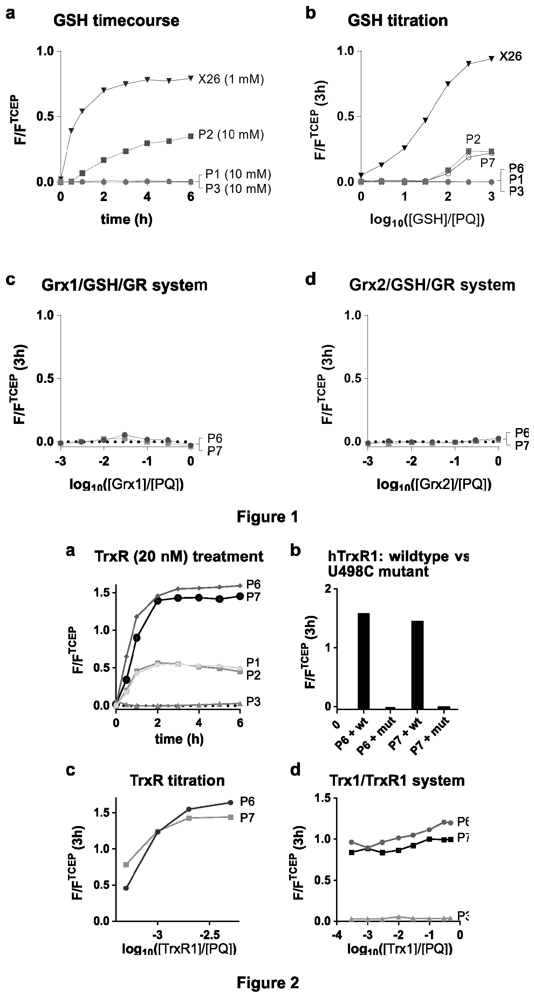

**Figure 1** shows cell-free assays evaluating: how the compounds of the invention can resist releasing their cargos when challenged with the ubiquitous cellular non-enzymatic reductant GSH up to supraphysiologically high concentrations; how the compounds of the invention can also resist releasing their cargos when challenged by full enzyme/effector redox systems comprised of GR (powered by NADPH), and GSH, and either Grx1 or Grx2; and therefore illustrates that compounds of the invention can resist reductive triggering even by chemocompatible reductants (and therefore have the potential for enzyme- or protein-selective triggering, as will be shown later). The timecourse graph in panel a shows how the monothiol reductant glutathione (GSH) time-dependently triggers the release of fluorescent cargo **PQ-OH,** as measured by fluorescence signal. Prior art dichalcogenide **X26** is rapidly and strongly affected by subphysiological GSH (1 mM), whereas compounds of the invention can majorly (**P2**) or entirely (**P1**, **P3**) resist even supraphysiological GSH concentrations (10 mM). The concentration dependence of timecourse data is summarised in the GSH titration plot, which shows the signal generation by those probes (at 10 µM) at the 3 h timepoint, depending on the ratio of GSH to probe concentration (expressed as a fraction of the maximal signal F^{TCEP}); this quantifies how compounds of the invention have very low sensitivity (**P2**, **P7**) or are completely insensitive (**P1**, **P3, P6**) to GSH up to even supraphysiological concentrations (10 mM). The two GR/GSH/Grx plots show that probes of the invention **P6** and **P7** (at 10 µM) resist triggering by these thiol-based reductant systems up to physiological Grx concentrations, as well as by the oxidoreductase GR that is also present. Thus, the results of Figure 1 show that compounds of the general formula (I) can be stable to physiological levels of biological monothiol reductants (GSH) and to selected dithiol-type oxidoreductases and redox effector proteins.
**Figure 2** shows the resistance or activation of compounds of the invention that release the fluorescent cargo **PQ-OH,** evaluated in cell-free assays, with components of the Trx/TrxR system. The timecourse graph shows that compounds of the general formula (I) (**P1**, **P2, P6, P7**) can release their cargo rapidly upon exposure to the oxidoreductase TrxR (with NADPH) and can therefore be suitable as TrxR-responsive proagents, while other compounds compounds of the general formula (I) (**P3**) are stable to this challenge and therefore can be suitable as redox-responsive proagents for other redox activities. The wildtype/mutant assay shows that compounds of the invention which are TrxR-sensors (**P6**, **P7**) are selective for the wildtype TrxR enzyme, and do not respond to the mutant with a point mutation of the key active site selenocysteine to cysteine; this also shows how compounds of the invention can have excellent selectivity for physiological reducing species. The TrxR titration shows that compounds of the invention which are TrxR-sensors (**P6**, **P7,** each 10 µM) are excellently responsive to even subphysiological concentrations of TrxR (shown: down to 5 nM) highlighting how compounds of the invention will be useful for sensitive as well as selective response to physiological reductant species. The Trx1 titration ("Trx1/TrxR1 system") shows that compounds of the invention can be selective for single components within redox systems and can therefore be useful to probe or respond to individual elements of redox networks; in this example, compounds of the general formula (I) (**P6** and **P7** at 10 µM) have identical release of cargo when challenged by increasing concentrations of redox effector Trx1 in the presence of a fixed concentration its oxidoreductase TrxR1, because the probes are functionally TrxR1-selective.

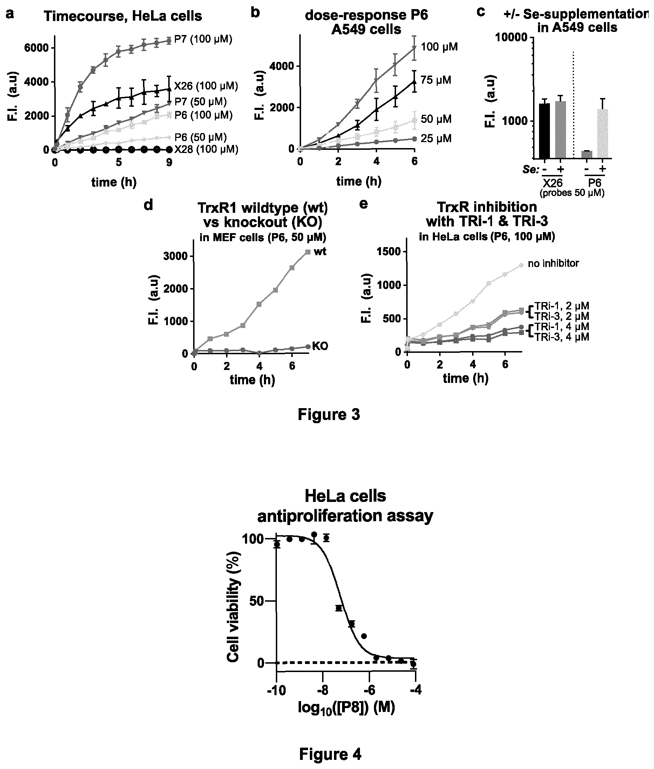

**Figure 3** shows the fluorescence signal generation by compounds of the invention that release the fluorescent cargo **PQ-OH** upon reductive triggering, when administered to various cell lines (HeLa, A549, MEF wildtype, MEF with TrxR1 knockout) under various conditions (chemical inhibition of TrxR, knockout of TrxR1, cell culture with selenium starvation or supplementation). The HeLa cell timecourse illustrates that compounds of the invention **P6** and **P7** can generate strong time- and dose-dependent fluorescent signal increase, while the control compound **X28** (non-reducible cyclohexyl control) does not generate signal, illustrating that the carbamate system used in the compounds of the invention is not responsible for their signal generation (i.e. the carbamate is robust to cellular hydrolysis). The A549 cell results show that this time- and dose-dependent fluorescence increase (for **P6**) is reproducible in another cell line, indicating that compounds of the invention can be usefully applied in diverse cell lines. The selenium supplementation assay shows that for the case of the TrxR1-selective compound **P6,** signal generation of **P6** is strongly suppressed by selenium starvation indicating that its cellular processing depends on one or more selenium-dependent reductases; the TrxR1 knockout assay shows that the signal of **P6** is almost entirely suppressed by knockout, and the inhibitor assay shows that chemical TrxR inhibition with TRi compounds also suppresses **P6** signal generation. Taken together with its stability against the GR/GSH/Grx systems *in vitro* (Fig 1) and its strong activation by the selenium-dependent reductase TrxR1 *in vitro* only when it incorporates selenium (Fig 2), this shows that **P6** is processed by native (selenium-incorporating) TrxR1 in cells, making it an oxidoreductase-selective proagent. This illustrates that compounds of the invention can have highly sensitive and selective response to a major cellular oxidoreductase or redox effector protein, making the compounds of the general formula (I) useful for selective cargo release depending on the presence and activity of that reducing species.
**Figure 4** shows result of antiproliferation assays in HeLa cervical cancer cell line, for **P8,** a therapeutic compound of the general formula (I) with the same selenenylsulfide trigger motif as fluorogenic probe **P1,** but which releases a DNA alkylator (CBI derivative) following reduction. **P8** has EC₅₀ ca. 60 nM, indicating that cellular TrxR activity can activate **P8** and release an amount of the of active DNA-alkylating agent which then performs a desirable therapeutically effective action. This shows that by choosing alternative cargos to release, the compounds of the invention can achieve alternative desirable actions.

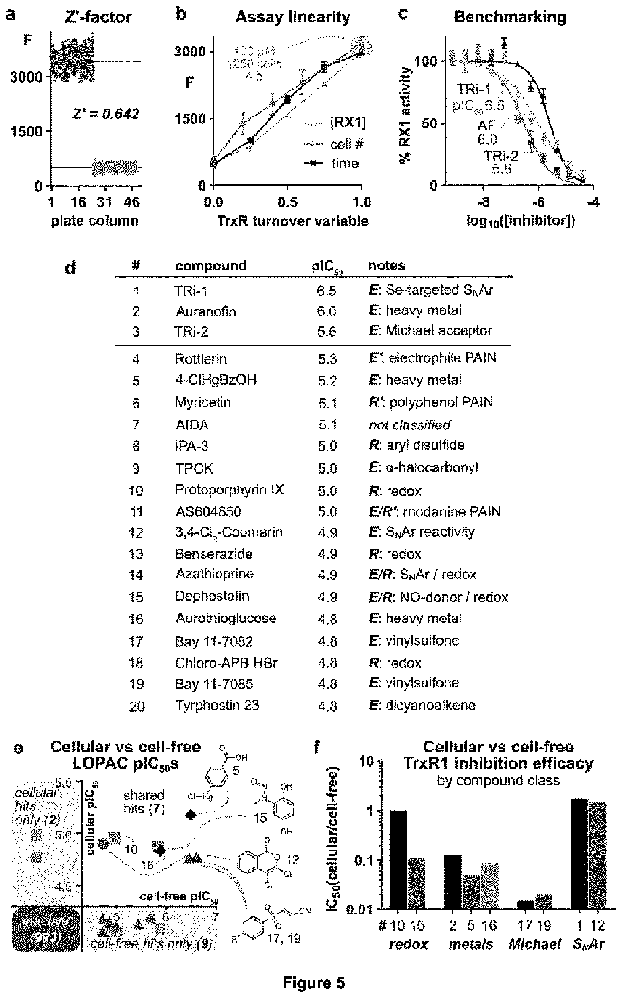

**Figure 5** shows results of a high-throughput screen with **P6** that quantifies cellular TrxR inhibition by pharmaceutically active compounds. Wide assay dynamic range and excellent data precision lead to an outstanding Z' value (cells vs no cells controls). The signal-turnover relationship of **P6** is linear over the whole dynamic range (parameters varied: incubation time, **P6** concentration, cell count; standard parameter values normalised to 1). Dose-response plots obtained in 6 µL automatic **P6** HTS for reference inhibitors show robust signal decrease and IC₅₀. Potencies for all 18 hits from LOPAC₁₂₈₀ **P6** qHTS plus TRi-1 and TRi-2 are annotated for reactivity and compound class (E: electrophile, R: redox-active; pIC₅₀ = log₁₀(IC₅₀ [M])). Potency distribution among compounds tested in current cellular as well as previous cell-free qHTS. Ratios of cellular to cell-free potencies of shared hits suggest that S_{N}Ar-based TrxR-inhibitors may translate well through development. [Data in panels b,c are shown as mean ± SD from three independent experiments.]

### Definitions

Unless stated otherwise the following definitions apply.

The term "**alkyl**" refers to a saturated straight or branched hydrocarbon chain. In any of the below definitions, C₁₋₄-alkyl is not particularly limited, and refers to a saturated straight or branched hydrocarbon chain, which has 1 to 4 carbon atoms, preferably 1 to 3 carbon atoms, more preferably 1 or 2 carbon atoms, even more preferably 1 carbon atom. Preferably C₁₋₄-alkyl can be methyl, ethyl, n-propyl, or i-propyl, more preferably it can be methyl or ethyl, most preferably it can be methyl.

In any of the below definitions, C₂₋₄-**alkylene** is not particularly limited, and refers to a saturated straight or branched hydrocarbon chain, which has 2 to 4 carbon atoms, preferably 2 or 3 carbon atoms, more preferably 2 carbon atoms. Preferably C₂₋₄-alkylene can be ethylene, or n-propylene, more preferably it can be ethylene.

An **aromatic ring** is any not particularly limited and refers to any aromatic carbocyclic ring, which has 5 to 7 carbon atoms, preferably 5 or 6 carbon atoms, more preferably the aromatic ring is phenyl. The aromatic ring can optionally be annulated to one or more carbocyclic or heterocyclic rings. Examples of annulated rings include but are not limited to naphthyl, quinolyl, isoquinolyl, quinoxalyl, benzopyranonyl, benzo[1,3]dioxolyl, benzothiazolyl, 2,3-dihydro-1*H-*benzo[e]indole, xanthenyl or indolyl, preferably naphthyl, quinolyl or xanthenyl.

The **heteroaromatic ring** is any not particularly limited and refers to any aromatic heterocyclic ring, which has 5 to 7 ring atoms, preferably 5 or 6 ring atoms. The heteroaromatic ring can optionally be annulated to one more carbocyclic or heterocyclic rings. Examples of the heteroaromatic ring include thiophenyl, pyridinyl, pyrazolyl, pyrimidinyl, purinyl, pyrrolo, furanyl, oxazolyl, thiazolyl, imidazolyl, benzofuranyl, benzothiophenyl, benzothiazolyl, benzoxazolyl or naphthyridinyl, preferably pyrrolo, pyridinyl, thiophenyl or furanyl.

An **aliphatic moiety** is a saturated or unsaturated, linear, branched or cyclic moiety containing between 1 to 50 carbon atoms and optionally 0 to 19 heteroatoms, preferably 0 to 15 heteroatoms, wherein the heteroatoms are typically chosen from O, N, S, Se, Si, Hal, B or P, preferably chosen from O, N, Hal, S, Si or P, more preferably O, N, Hal, S, Si, even more preferably chosen from O, N or Hal.

A **carbocyclic ring** is a cyclic structure containing from 4 to 50 carbon atoms. Examples of the carbocyclic ring include cyclobutane, cyclopentane, cyclohexane, benzene, cycloheptane, naphthalene or anthracene, preferably cyclohexane, cyclopentane, benzene or naphthalene, more preferably benzene or naphthalene.

A **heterocyclic ring** is a cyclic structure containing at least one heteroatom selected from N, O, Si, S, B, P, and Se, and containing at least one carbon atom. Examples of the heterocyclic ring include thiophene, pyridine, pyrazole, pyrimidine, pyrrole, furan, oxazole, thiazole, imidazole, quinoline, isoquinoline, benzofuran, benzothiophene, benzothiazole, benzoxazole, naphthyridine, piperidine, piperazine, pyrrolidine, tetrahydrothiophene, tetrahydrofuran or pyran, preferably pyridine, furan, thioazole, quinoline, isoquinoline, benzothiazole, piperidine, piperazine or pyran, more preferably pyridine, quinoline, piperidine or piperazine.

**Halogen** refers to -F, -Cl, -Br or -I, preferably -F or -CI.

If a moiety is referred to as being "**optionally substituted**" by a substituent it can in each instance include one or more of the indicated substituents.

**Oxidoreductase** (also referred to as disulfide reductase or disulfide oxidoreductase) refers to an enzyme whose biochemical reactivity includes reducing disulfides to the corresponding thiols. Examples thereof include thioredoxin reductase 1 (TrxR1), TrxR2, thioredoxin glutathione reductase (TrxR3/TGR), methionine-*R*-sulfoxide reductase (MsrB1), or glutathione disulfide reductase (GR). A **disulfide effector protein** (or redox effector protein or simply effector protein) is a redox-active protein, typically with a dithiol active site such as a thioredoxin-like domain but alternatively also with a monothiol or selenol active site, that is reduced directly or indirectly by an upstream catalytic disulfide oxidoreductase and fulfils redox-active functions. Examples thereof include thioredoxins (Trxs) such as Trx1 and Trx2; thioredoxin-related proteins such as thioredoxin-related protein of 14 kDa (TRP14) and thioredoxin-related transmembrane proteins (TMXs) such as TMX1, TMX2, TMX3, and TMX4; thioredoxin-like protein 1 (TXNL1); human macrothioredoxin (hMTHr); glutaredoxins such as Grx1, Grx2, Grx3, Grx4, Grx5, GrxA, and Grx6; glutathione peroxidases (GPxs) such as GPx1, GPx4, GPx2; protein disulfide isomerases (PDIs) such as PDIA1, PDIA2, PDIA3, PDIA4, PDIA5, PDIA6, EndoPDI, PDIp, endoplasmic reticulum resident protein 18 (ERp18), ERp44, ERp57, ERp72, ERdj5, disulfide bond formation protein A (DsbA), DsbB, DsbC.

The term "**cancer**", as used herein, refers to a group of proliferative diseases characterized by uncontrolled division of abnormal cells in a subject. Non-limiting examples of cancers include acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma.

The term "**cargo**", as used herein, represents a therapeutic, diagnostic or theranostic agent to be delivered to a site of enzymatic activity or disease (e.g. a tumour, a site of inflammatory or autoimmune disease). Cargos used in the compounds of the invention may be small molecules. Examples include agents for treating, ameliorating, preventing or diagnosing a neoplastic disorder, an inflammatory disorder (e.g. a non-steroidal anti-inflammatory drug (NSAID), a disease-modifying anti-rheumatic drug (DMARD), or mesalamine), atherosclerosis; an autoimmune disorder; a chronic inflammatory autoimmune disorder; ischaemia; and reperfusion injury as well as kinase inhibitors. Examples also include diagnostically acceptable dyes, therapeutically acceptable DNA-alkylating agents, therapeutically acceptable topoisomerase-inhibiting agents or therapeutically acceptable tubulin-inhibiting agents.

Non-limiting examples of **antineoplastic** agents include 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-*N,O*-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin γ1, calicheamicin δ1, calicheamicin ε, calicheamicin θ, or calicheamicin T.

Non-limiting examples of **NSAIDs** include diclofenac, aceclofenac, mefenamic acid, clonixin, piroxicam, meloxicam, tenoxicam, or lornoxicam.

Non-limiting examples of **DMARDs** include methotrexate.

Non-limiting examples of **kinase inhibitors** include baricitinib, filgotinib, tofacitinib, upadacitinib, ruxolitinib, peficitinib, decemotinib, solcitinib, itacitinib, fostamatinib, or SHR0302. Particularly of interest are inhibitors of the Janus Kinase (JAK) family of kinases, such as of JAK1 or JAK3.

Cargos used in the compounds of the invention may be diagnostic agents (e.g., a fluorescent agent or a radiotracer agent), that may be used to detect and/or determine the stage of a targeted pathology such as a tumor, or to measure or localise redox activity. Non-limiting examples of fluorescent diagnostic agents include leuco-methylene blue, leuco-methyl methylene blue, leuco-dimethyl methylene blue, leuco toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methyl-coumarin, 7-amino-4-chloromethylcoumarin, 3-*O*-methylfluorescein, 3-*O*-ethyl-5-carboxy-fluorescein, 2,7-difluoro-3-*O*-methylfluorescein, 3-*N*-acetyl-rhodamine, 3-*N*-acetyl-dimethyl-silarhodamine, 2,7-dibromo-3-*N*-acetyl-dimethylcarborhodamine, 3-*N*-acetyl-6-carboxy-rhodamine, 2,7-difluoro-3-*N*-acetylrhodol, 3-*O*-(*N,N*-dimethyl-2-aminoethyl)-6-carboxy-fluorescein, 2,7-dichloro-3-*O*-(*N*,*N*-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, or 6-bromo-2-(5-bromo-2-hydroxyphenyl)quinazolin-4-one.

Radiotracer agents are agents useful for imaging which incorporate a radioactive isotope, non-limiting examples of which are radioactive isotopes of carbon, cobalt, fluorine, gallium, iodine, or technetium, preferably carbon, fluorine or iodine. Compounds of the present invention which have been labelled with a radioactive isotope can be employed as diagnostic applications.

The term "**pharmaceutically acceptable salt**" refers to a salt of a compound of the present invention. Suitable pharmaceutically acceptable salts include acid addition salts which may, for example, be formed by mixing a solution of compounds of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, hemisulfate, heptanoate, hexanoate, hydrabamine, hydrobromide, hydrochloride, hydroiodide, 2-hydroxy-ethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, succinate, tannate, tartrate, teoclate, tosylate, triethiodide, undecanoate, valerate, and the like (see, for example, S. M. Berge et al.; J. Pharm. Sci. 1977, 66, 1-19).

When the compounds of the present invention are provided in crystalline form, the structure can contain solvent molecules. The solvents are typically pharmaceutically acceptable solvents and include, among others, water (hydrates) or organic solvents. Examples of possible **solvates** include hydrates, ethanolates and iso-propanolates.

The term "**pharmaceutically acceptable ester**" refers to an ester of a compound of the present invention. Suitable pharmaceutically acceptable esters include acetyl, butyryl, and acetoxymethyl esters.

The term "**protecting group**", as used herein (represented in schemes as moieties -PG), represents a group intended to protect a hydroxy or an amino group from participating in one or more undesirable reactions during chemical synthesis. The term "***O*-protecting group**", as used herein, represents a group intended to protect a hydroxy or carbonyl group from participating in one or more undesirable reactions during chemical synthesis. The term "***N*-protecting group**", as used herein, represents a group intended to protect a nitrogen containing (e.g., an amino or hydrazine) group from participating in one or more undesirable reactions during chemical synthesis. Exemplary *O*- and *N*-protecting groups include, but are not limited to: alkanoyl, aryloyl, or carbamyl groups such as formyl, acetyl, propionyl, pivaloyl, tert-butylacetyl, 2-chloroacetyl, 2-bromoacetyl, trifluoroacetyl, trichloroacetyl, phthalyl, o-nitrophenoxyacetyl, *α*-chlorobutyryl, benzoyl, 4-chlorobenzoyl, 4-bromobenzoyl, triiso-propylsilyloxymethyl, 4,4'-dimethoxytrityl, isobutyryl, phenoxyacetyl, 4-isopropylphenoxy-acetyl, dimethylformamidino, and 4-nitrobenzoyl. Other *O*-protecting groups include, but are not limited to: substituted alkyl, aryl, and aryl-alkyl ethers (e.g., trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; *tert*-butyl ether; *p*-chlorophenyl, p-methoxyphenyl, p-nitrophenyl, benzyl, p-methoxybenzyl, and nitrobenzyl); silyl ethers (e.g., trimethylsilyl; triethylsilyl; triisopropylsilyl; dimethylisopropylsilyl; *tert*-butyldimethylsilyl; *tert*-butyldiphenylsilyl; tribenzylsilyl; triphenylsilyl; and diphenymethylsilyl); carbonates (e.g., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl). Other *N*-protecting groups include, but are not limited to, chiral auxiliaries such as protected or unprotected, *L*- or *D*-amino acids such as alanine, leucine, phenylalanine, and the like; sulfonyl-containing groups such as benzenesulfonyl, *p*-toluenesulfonyl, and the like; carbamate forming groups such as benzyloxycarbonyl, *p*-chlorobenzyloxycarbonyl, *p*-methoxybenzyloxycarbonyl, *p*-nitrobenzyloxycarbonyl, 2-nitrobenzyloxycarbonyl, *p*-bromobenzyloxycarbonyl, 3,4-dimethoxybenzyloxycarbonyl, 3,5-dimethoxybenzyl oxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1 - (*p*-biphenylyl)-lmethylethoxycarbonyl, *a,a*-dimethyl-3,5-dimethoxybenzyloxycarbonyl, benzhydryloxy carbonyl, tbutyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, aryl-alkyl groups such as benzyl, triphenylmethyl, benzyloxymethyl, and the like and silyl groups such as trimethylsilyl, and the like. Useful *N*-protecting groups are formyl, acetyl, benzoyl, pivaloyl, *tert*-butylacetyl, alanyl, phenylsulfonyl, benzyl, *tert*-butyloxycarbonyl (Boc), and benzyloxycarbonyl (Cbz).

The term "**proteinogenic amino acids"** herein comprises the 20 principal naturally-occurring *L*-amino acids (glycine, alanine, valine, leucine, isoleucine, phenylalanine, proline, methionine, serine, threonine, cysteine, tyrosine, tryptophan, asparagic acid, glutamic acid, asparagine, arginine, histidine, lysine, asparagine and glutamine) which are to be attached, as the monopeptide, via their carboxyl terminus to the specified amine nitrogen of the compound, creating amides. Preferably the proteinogenic amino acid is selected from leucine, serine and lysine.

The term "**self-immolative spacer",** also called "traceless linker" or "self-immolative linker", as used herein, represents a multivalent (e.g., divalent) group covalently linking a dichalcogenide-containing group which will be further defined below (as the moiety A), to a cargo B, in such a way that reductive cleavage of the dichalcogenide bond in the moiety A is followed by a sequence of reactions which result in the cleavage of a bond between the self-immolative linker group and the cargo B, thereby releasing the cargo. Illustrative examples thereof include:

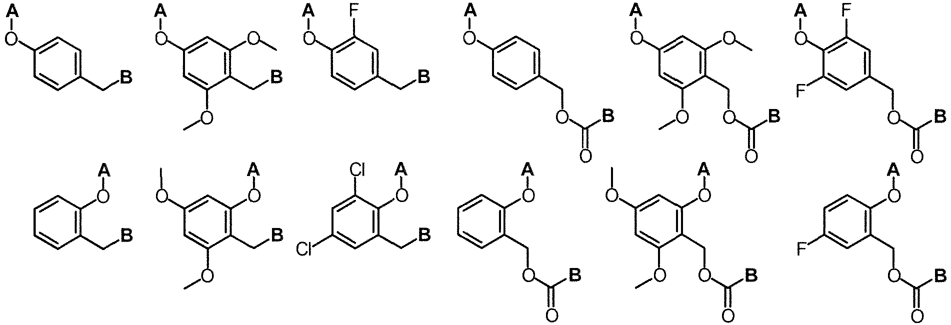

The stereochemistry at the ring atoms bridging the two rings of the bicyclic dichalcogenide structure of the compounds of formula (I) of the invention is not particularly limited; any absolute configuration of the two bridging ring tertiary carbon atoms is within the scope of the compounds of formula (I). Either carbon can be separately either R, or S, or a mixture of R and S in any proportions (including racemic and undefined proportions); therefore the dichalcogenide ring may be considered to be cis-fused to the carbamate-containing ring with any given stereochemistry, or *trans*-fused to the carbamate-containing ring with any given stereochemistry, or present as a mixture in any proportions of *cis*-fused and *trans*-fused with any given stereochemistries (including racemic and undefined proportions). Preferably, the dichalcogenide ring is either *cis*-fused or *trans*-fused.

The term "**vascular disrupting agent**" refers to a compound that reduces tumor blood flow upon administration. Such agents are typically microtubule-depolymerising agents, and are well represented in the literature (Gill, Pharmacology & Therapeutics 2019, 202, 18-31); as known to those skilled in the art, these include combretastatin A-4 (CA4), 3'-aminocombretastatin A-4, BNC105, ABT-751, ZD6126, combretastatin A-1, and prodrugs of the same, including but not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P).

The term "**cytotoxic chemotherapeutic agent**" refers to compounds that have antineoplastic and/or antitumoral efficacy and which are typically useful in the treatment of cancer, optionally as part of combination therapies. Such agents include paclitaxel, docetaxel, nab-paclitaxel, epothilone, mertansine, irinotecan, etoposide, teniposide, mitoxantrone, doxorubicin, daunorubicin, epirubicin, cisplatin, carboplatin, oxaliplatin, melphalan, chlorambucil, busulfan, methotrexate, permetrexed, cyclophosphamide, 5-fluorouracil, capecitabine, gemcitabine, cytarabine, fludarabine, mercaptopurine, vinolrebine, vinblastine, vincristine, dolastatin, monomethyl auristatin E, monomethyl auristatin F, bleomycin, dacarbazine, pyrrolobenzodiazepine, temozolomide, carmustine, mitomycin and procarbazine.

In the context of the invention the term "**immunomodulator**" refers to therapeutically active compounds that interact with the immune system, which may for example be useful in the treatment of cancer or autoimmune diseases, optionally as part of combination therapies. Such agents include anti-PD-1 antibody such as pembrolizumab and nivolumab, anti-PD-L1 antibody such as avelumab, anti-CTLA4 antibody such as ipilimumab, cytokine agonists such as proleukin and interferon alfa-2a/interferon alfa-2b, mesalamine, baricitinib, filgotinib, tofacitinib, upadacitinib, ruxolitinib, peficitinib, decemotinib, solcitinib, itacitinib, fostamatinib, and SHR0302.

### Detailed description of the invention

### Compound having the formula (I)

The present invention provides a compound having the formula (I)

A-L-B (I)

A is represented by 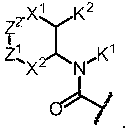
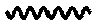 denotes the attachment point from A to L.
Z¹ and Z² are independently selected from S or Se such that either Z¹ is Se and Z² is S, or Z¹ is S and Z² is Se, or Z¹ and Z² are both Se. In a preferred embodiment, either Z¹ is Se and Z² is S, or Z¹ is S and Z² is Se. In another preferred embodiment, Z¹ and Z² are Se. More preferably, Z¹ is Se and Z² is S.

In a first embodiment, K¹ is selected from -C₁₋₄-alkyl optionally substituted by W¹. Preferably when K¹ is -C₁₋₄-alkyl optionally substituted by W', K¹ is -Me, -Et, -(CH₂)₂OH, -(CH₂)₂PPh₃⁺, -(CH₂)₂C(O)-1-(4-methylpiperazine), -(CH₂)₂C(O)-morpholino, -(CH₂)₃C(O)-morpholino or - (CH₂)₃C(O)-1-(4-methylpiperazine), more preferably K¹ is -Me, -Et, -(CH₂)₂C(O)-1-(4-methylpiperazine), or -(CH₂)₂C(O)-morpholino.

In the first embodiment, K² is selected from -H, -O-R^{j}, and -O-R^{k}; preferably K² is selected from -H, -OMe, -O(CH₂)₂O(CH₂)₂OH, -O(CH₂)₂PPh₃⁺, or -O(CH₂)₃C(O)-1-(4-methylpiperazine); more preferably K² is -H, -O(CH₂)₂PPh₃⁺, or -O(CH₂)₃C(O)-1-(4-methylpiperazine); more preferably K² is -H.

In a second embodiment, K¹ and K² are bonded together and -K¹-K²- is -X³-Y-X-, such that X³ is bonded to N and X is bonded to C.

X is selected from -N(R^{a})-, -N(R^{b})-, -CR^{c}₂- and -O-; preferably -N(R^{a})- or -CR^{c}₂; more preferably -CH₂-, -NH-, -N(Me)-, -N((CH₂)₂OH)-, -N((CH₂)₂PPh₃⁺)-, -N((CH₂)₃C(O)-1-(4-methylpiperazine))-.

X¹ is -(CR^{d}₂)ₘ-, preferably -(CH₂)- or -(CHMe)- or -(CMe₂)-, more preferably -(CH₂)-.

X² is -(CR^{e}₂)ₙ-, preferably -(CH₂)-.

X³ is -CR^{f}₂-, preferably -(CH₂)-.

Y is -(CR^{g}₂)ₚ-, preferably -(CH₂)- or -(CH₂)-(CH₂)-, more preferably -(CH₂)-.

W is selected from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group. The heterocyclic group is selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or -(C₂₋₄-alkylene)- group via the N atom. Preferably, W is selected from -OH, -C(O)-N((CH₂)₂OH)₂, -N(CH₃)₂, -PPh₃⁺, -C(O)-1-(4-methylpiperazine), -azetidin-1-yl, or -morpholino. More preferably, W is selected from -OH, -N(CH₃)₂, -PPh₃⁺, and -C(O)-1-(4-methylpiperazine).

Disclosed is that W is selected from -OH, -C(O)-N(R^{h})(Rⁱ), -N(R^{h})(Rⁱ), -PR^{x}₃⁺, -(morpholine-4-carbonyl), -(piperazine-1-carbonyl), -(4-methylpiperazine-1-carbonyl), -(4-ethylpiperazine-1-carbonyl), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein the heterocyclic group is attached to the -C₁₋₄-alkyl or -(C₂₋₄-alkylene)-group via the N atom. Preferably, W is selected from -OH, -C(O)-N((CH₂)₂OH)₂, -N(CH₃)₂, -PPh₃⁺, -(4-methylpiperazine-1-carbonyl), -azetidin-1-yl, or -morpholino. More preferably, W is selected from -OH, -N(CH₃)₂, -PPh₃⁺, and -(4-methylpiperazine-1-carbonyl).

W¹ is selected from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group. The heterocyclic group is selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or -(C₂₋₄-alkylene)- group via the N atom. Preferably, W¹ is selected from -OH, -C(O)-N((CH₂)₂OH)₂, -N(CH₃)₂, -PPh₃⁺, -(4-methylpiperazine-1-carbonyl), -azetidin-1-yl, or -morpholino. More preferably, W¹ is selected from -OH, -N(CH₃)₂, -PPh₃⁺, -C(O)-1-(4-methylpiperazine), and -C(O)-4-(morpholine).

Disclosed is that W¹ is selected from -OH, -C(O)-N(R^{h})(Rⁱ), -N(R^{h})(Rⁱ), -PR^{x}₃⁺, -(morpholine-4-carbonyl), -(piperazine-1-carbonyl), -(4-methylpiperazine-1-carbonyl), -(4-ethylpiperazine-1-carbonyl), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein the heterocyclic group is attached to the -C₁₋₄-alkyl or -(C₂₋₄-alkylene)-group via the N atom. Preferably, W¹ is selected from -OH, -C(O)-N((CH₂)₂OH)₂, -N(CH₃)₂, -PPh₃⁺, -(4-methylpiperazine-1-carbonyl), -azetidin-1-yl, or -morpholino. More preferably, W¹ is selected from -OH, -N(CH₃)₂, -PPh₃⁺, and -(4-methylpiperazine-1-carbonyl).

R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -(C₂₋₄-alkylene) or -(C₁₋₄-alkyl) can be optionally substituted by W. Preferably R^{a} is selected from -H and -C₁₋₄-alkyl, wherein -(C₁₋₄-alkyl) can be optionally substituted by W. More preferably, R^{a} is selected from -H, -CH₃, -C₂H₅, and -CH₂CH₂W.

R^{b} is an acyl group of a monopeptide selected from proteinogenic amino acids attached via a carboxy group. The attachment point is not particularly limited, the proteinogenic amino acid can be connected via the carboxy group at the α-carbon or via a carboxy group of the side chain forming an amide group with the ring nitrogen atom, preferably the proteinogenic amino acid is connected via the carboxy group at the α-carbon. The proteinogenic amino acid is not particularly limited and can be any amino acid as defined above; preferably leucine, serine or lysine; more preferably serine. In the case of serine, for example, R^{b} is -C(O)-C(H)(NH₂)-CH₂OH.

R^{c} groups are independently selected from -H and -C₁₋₄-alkyl, preferably H.

R^{d} groups are independently selected from -H and -C₁₋₄-alkyl, preferably H or Me.

R^{e} groups are independently selected from -H and -C₁₋₄-alkyl, preferably H.

R^{f} groups are independently selected from -H and -C₁₋₄-alkyl, preferably H.

R^{g} groups are independently selected from -H and -C₁₋₄-alkyl, preferably H.

R^{h} is selected from -H, -C₁₋₄-alkyl and -CH₂CH₂OH; preferably, from -H, -CH₃ and -CH₂CH₂OH.

Rⁱ is selected from -H, -C₁₋₄-alkyl and -CH₂CH₂OH; preferably, from -H, -CH₃ and -CH₂CH₂OH.

R^{j} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W¹. Preferably R^{j} is selected from -H and -C₁₋₄-alkyl, wherein -(C₁₋₄-alkyl) can be optionally substituted by W¹. More preferably, R^{j} is selected from -H, -CH₃, -C₂H₅, and -CH₂CH₂W¹.

R^{k} is an acyl group of a monopeptide selected from -proteinogenic amino acids attached via a carboxy group. The attachment point is not particularly limited, the proteinogenic amino acid can be connected via the carboxy group at the α-carbon or via a carboxy group of the side chain forming an amide group with the ring nitrogen atom, preferably the proteinogenic amino acid is connected via the carboxy group at the α-carbon. The proteinogenic amino acid is not particularly limited and can be any amino acid as defined above; preferably leucine, serine or lysine; more preferably serine. In the case of serine, for example, R^{k} is -C(O)-C(H)(NH₂)-CH₂OH.

R^{x} groups are independently selected from -phenyl and 4-methoxyphenyl-;
m is 0, 1 or 2 and n is 1 or 2, provided that m+n is 2 or 3. Preferably m is 1 and n is 1.
p is 0, 1, or 2, provided that when K¹ and K² are bonded together and represent -X³-Y-X-,
and X represents -N(R^{a})- or -N(R^{b})-, then p = 1 or 2. Preferably p is 1 or 2. More preferably p is 1.
L is a bond or a self-immolative spacer according to claim 1.

In one embodiment, L is a bond. In another embodiment, L is a self-immolative spacer. A self-immolative spacer is any linking group which is capable of covalently linking A and B and which can release the cargo H-B when the dichalcogenide bond in A is cleaved and the resulting dichalcogenol species cyclizes *via* nucleophilic attack at the carbonyl group of A. The self-immolative spacer is not particularly limited and can be chosen from any known self-immolative spacer. Examples are to be found in e.g. Blencowe, C. A. et al. Polym. Chem. 2011, 2, 773-790. https://doi.org/10.1039/C0PY00324G. Preferred examples thereof include, but are not limited to, the moieties shown in the Definitions section, and, in particular, para-hydroxybenzylic 1,4-elimination self-immolative spacers and para-hydroxybenzylic 1,6-elimination self-immolative spacers:

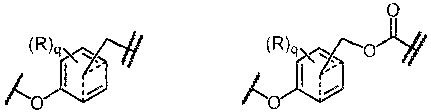

In these formulae (R)_{q}- can be attached at any available position on the benzene ring. 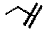 and 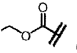 can be attached at either of the carbon atoms which are indicated by the dashed lines.

More preferably the self-immolative spacer is a *para*-hydroxybenzylic 1,6-elimination spacer:

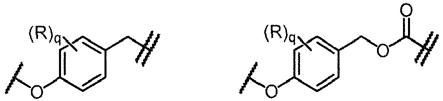

In these formulae
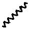 denotes the attachment from A to L.
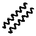 denotes the attachment point from L to B.

If L is a bond, then the cargo **B,** which is a therapeutic, diagnostic or theranostic agent, can contain an -OH group that is attached to a 5- or 6-membered aromatic or heteroaromatic ring, preferably an -OH group that is attached to a 6-membered aromatic ring, more preferably an -OH group that is attached to phenyl ring. The 5- or 6-membered aromatic or heteroaromatic ring or the phenyl ring can be part of a larger structure as defined below. In these embodiments L is suitable for binding to A *via* the O atom, giving a carbamate.

If L is 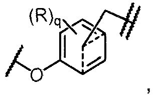 then the cargo, which may be a therapeutic, diagnostic or theranostic agent (such as an aniline or amine) that contains an -NH₂ or -NH- moiety; or an -OH group that is attached to a 5- or 6-membered aromatic or heteroaromatic ring that can be part of a larger structure as defined below; preferably, an -OH group that is attached to a 5- or 6-membered aromatic ring that can be part of a larger structure as defined below; more preferably, an -OH group that is attached to a phenyl ring that can be part of a larger structure as defined below.

In these embodiments the -NH₂ or -NH- moiety or the OH group is suitable for binding to L *via* the respective N or O atom.

If L is 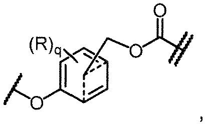 then the cargo, which is a therapeutic, diagnostic or theranostic agent, can contain an -NH₂ or -NH- moiety; or an -OH group that is attached to a 5- or 6-membered aromatic or heteroaromatic ring; preferably an -NH₂ or -NH- moiety which is attached to an aromatic ring that can be part of a larger structure as defined below, or an -OH group that is attached to a 5- or 6-membered aromatic ring that can be part of a larger structure as defined below; more preferably an -OH group that is attached to a phenyl ring that can be part of a larger structure as defined below.

In these embodiments the -NH₂ or -NH- moiety or the OH group is suitable for binding to L *via* the respective N or O atom.

q is 0, 1, 2, 3 or 4, preferably q is 0 or 1.

Each group R is independently selected from -halogen -O(R^{r}), -N(R^{s})(R^{t}), -NO₂, -CN, and a heterocyclic group selected from azetidinyl, pyrrolidinyl, piperidinyl or morpholino, wherein the heterocyclic group is attached to the aromatic ring *via* the N atom. Preferably, group R is a halogen (such as F or Cl) in *ortho* position relative to the bond to A; also preferably, group R is -O(R^{r}) in *ortho* position relative to the bond to B.

R^{r} is selected from -H and -C₁₋₄-alkyl, preferably -C₁₋₄-alkyl, more preferably R^{r} is methyl.

R^{s} is selected from -H, -C(O)-C₁₋₄-alkyl and -C₁₋₄-alkyl, preferably -H or -C₁₋₄-alkyl, more preferably -H or -CH₃.

R^{t} is selected from -H, -C(O)-C₁₋₄-alkyl and -C₁₋₄-alkyl, preferably -H or -C₁₋₄-alkyl, more preferably -H or -CH₃.

Most preferred examples of self-immolative spacers that link groups A and B are

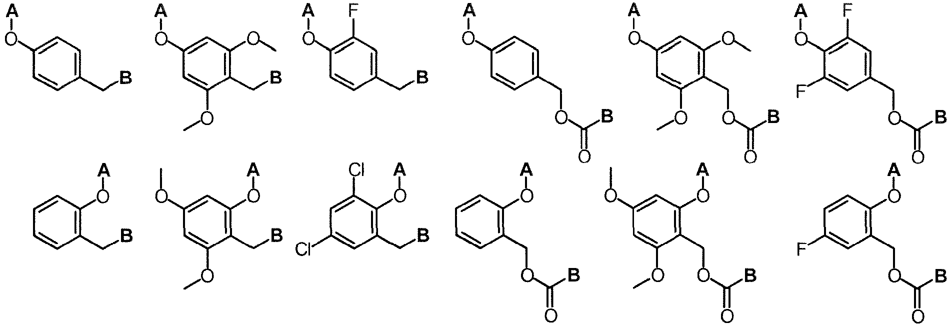

B is 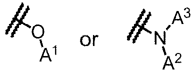 in which 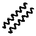 denotes the attachment point from L to B.

A¹ is selected such that A'-OH is a therapeutic, diagnostic or theranostic agent which contains an -OH group that is attached to a 5- or 6-membered aromatic or heteroaromatic ring, preferably an -OH group that is attached to a 5- or 6-membered aromatic ring, more preferably an -OH group that is attached to a phenyl ring. The bond attaching L to that O atom replaces its bond to hydrogen that is present in the agent A'-OH. The 5- or 6-membered aromatic or heteroaromatic ring can be part of a larger structure which forms the therapeutic, diagnostic or theranostic agent A'-OH. In particular, the 5- or 6-membered aromatic or heteroaromatic ring can be substituted, or can be fused to a further ring or rings such as aromatic or heteroaromatic rings, preferably phenyl, pyridinyl, naphthyl, quinolyl, isoquinolyl, indolyl, pyrrolyl, thiophenyl, pyridinyl, pyrazolyl, pyrimidinyl, furanyl, oxazolyl, thiazolyl, imidazolyl, purinyl, pyrrolo, benzofuranyl, benzothiophenyl, benzothiazolyl, benzoxazolyl or naphthyridinyl, quinoxalyl, benzopyranonyl, benzo[1,3]dioxolyl, benzothiazolyl, 2,3-dihydro-1*H*-benzo[*e*]indole, xanthenyl or indolyl, more preferably phenyl, pyridinyl, benzopyranonyl, naphthyl, benzo[1,3]dioxolyl, 2,3-dihydro-1*H*-benzo[*e*]indole, quinolyl or xanthenyl.

A¹ can be any hydrocarbon moiety which contains a 5- or 6-membered aromatic or heteroaromatic ring, wherein the hydrocarbon moiety has from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

A² and A³ are independently selected such that A²-NH-A³ is a therapeutic, diagnostic or theranostic agent which contains an -NH₂ or -NH- moiety. A² and A³ can be independently selected from 5- or 6-membered aromatic or heteroaromatic rings and aliphatic moieties, and A² may optionally be H. In one embodiment, A² and A³ may optionally be connected forming a carbocyclic or heterocyclic ring that can be saturated, unsaturated or aromatic and which has from 5 to 7 ring atoms. Each 5- or 6-membered aromatic, heteroaromatic, carbocyclic or heterocyclic ring can be part of a larger structure which forms the therapeutic, diagnostic or theranostic agent A²-NH-A³. In particular, the 5- or 6-membered aromatic or heteroaromatic ring or the carbocyclic or heterocyclic ring can be substituted or can be fused to a further ring or rings such as aromatic or heteroaromatic rings, preferably phenyl, pyridinyl, naphthyl, quinolyl, isoquinolyl, indolyl, pyrrolyl, thiophenyl, pyridinyl, pyrazolyl, pyrimidinyl, furanyl, oxazolyl, thiazolyl, imidazolyl, purinyl, pyrrolo, benzofuranyl, benzothiophenyl, benzothiazolyl, benzoxazolyl or naphthyridinyl, quinoxalyl, benzopyranonyl, benzothiazolyl, xanthenyl or indolyl. The aliphatic moiety can be substituted.

A² can be hydrogen or any hydrocarbon moiety which has from 1 to 50 carbon atoms, preferably from 1 to 20 carbon atoms, more preferably 1 to 10 carbon atoms, even more preferably 1 to 4 carbon atoms. The hydrocarbon moiety can optionally contain 1 or more heteroatoms, preferably 1 to 10 heteroatoms, more preferably 1 to 5 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal. In one embodiment A² is a C₁₋₄ alkyl, such as methyl.

A³ can be any hydrocarbon moiety which contains from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

If A² and A³ are bound together to form a carbocyclic or heterocyclic ring, the carbocyclic or heterocyclic ring can contain from 3 to 50 carbon atoms, preferably from 5 to 50 carbon atoms, more preferably 6 to 50 carbon atoms. The hydrocarbon moiety can optionally contain 1 or more heteroatoms, preferably 1 to 20 heteroatoms, more preferably 1 to 15 heteroatoms, wherein the heteroatoms can be selected from O, N, S, Se, Si, Hal, B or P, preferably selected from O, N, Hal, S, Si or P, more preferably selected from O, N, Hal, S, Si, even more preferably selected from O, N or Hal.

The nature of the therapeutic agent, diagnostic agent or theranostic agent is not particularly limited, as long as it contains an -OH, -NH₂ or -NH- moiety which after removal of the H is suitable for binding to the self-immolative spacer L, or, if L is a bond, to A, via the O or N atom. As can be seen from the schemes below, the O or N atom partakes in the reaction when the moiety A of a compound having the formula (I) is cleaved by an oxidoreductase or redox effector protein. The rest of the therapeutic agent, diagnostic agent or theranostic agent is not involved in this reaction, so that a wide variety of therapeutic agents, diagnostic agents and theranostic agents are suitable for use in the present invention.

Examples of suitable therapeutic agents, diagnostic agents and theranostic agents include, but are not limited to diagnostically acceptable dyes, therapeutically acceptable DNA-alkylating agents, therapeutically acceptable topoisomerase-inhibiting agents or therapeutically acceptable tubulin-inhibiting agents. In one embodiment, diagnostically acceptable dyes are preferred, which includes compounds or structurally and functionally related derivatives selected from, but not limited to the following classes of dyes: xanthenes including e.g. alkyl-fluoresceins, alkyl-carbofluoresceins, alkyl-silarhodamines, phenylquinazolinones, coumarins, firefly-luciferins, black hole quencher dyes, or any compound derived by substitution of the same. How acceptable compounds may be derived by substitution will be clarified in the subsequent examples. In another embodiment, therapeutically acceptable agents are preferred, which includes theranostic agents that include structurally and functionally related derivatives of the leuco-methylene blue class; DNA-alkylating agents that include compounds or structurally and functionally related derivatives selected from, but not limited to the following classes: nitrogen mustards or seco-cyclopropabenzaindoles; topoisomerase-inhibiting agents that include compounds or structurally and functionally related derivatives selected from, but not limited to the following classes of compounds: anthracyclins or camptothecins; antiproliferative agents that include compounds or structurally and functionally related derivatives selected from, but not limited to the following classes: monomethyl auristatins, the Amaryllidaceae alkaloids; or any compounds derived by substitution of the same.

Examples of therapeutic agents, diagnostic agents and theranostic agents include, but are not limited to, agents for treating, ameliorating, preventing or diagnosing a neoplastic disorder, an inflammatory disorder (e.g. a non-steroidal anti-inflammatory drug (NSAID), a disease-modifying anti-rheumatic drug (DMARD), or mesalamine), atherosclerosis; an autoimmune disorder; a chronic inflammatory autoimmune disorder; ischaemia; and reperfusion injury as well as kinase inhibitors.

Preferred are agents for which the conjugation of the active agent H-B into the compound A-L-B results in a substantial reduction of diagnostic signal or therapeutic potency; particularly preferred are therapeutically acceptable agents for which the conjugation of the active agent H-B into the compound A-L-B totally or near-totally prevents the compound from exhibiting the diagnostic signal or therapeutic potency that is associated with the free agent H-B. This preferred total or near-total prevention of the activity of agent H-B when conjugated as A-L-B is possible in several ways, non-limiting examples of which are given below in the section

### Utility, Diseases, and Examples.

How suitable diagnostic, therapeutic or theranostic agents may be derived by substitution of specific agents will be understood by reference to the following non-limiting examples:
A'-OH can be Haugland's precipitating fluorophore 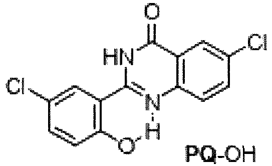
A'-OH or A²-NH-A³ can be a diagnostic agent of the xanthene class: 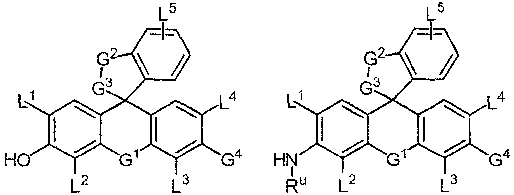
L¹, L², L³ or L⁴ are independently selected from -H, -halogen, -NO₂, -CN, -OMe, -O-CF₃. L⁵ is attached at any free position on the indicated benzene ring and is selected from -H, -C₁₋₄-alkyl, -halogen, -C(O)-C₁₋₄-alkyl, -C(O)-O-C₁₋₄-alkyl, -C(O)-N(-C₁₋₄-alkyl)₂, -S(O)₂-OH, -S(O)₂-O-C₁₋₄-alkyl, -O-C₁₋₄-alkyl.
G¹ is selected from -O-, -S-, -Se-, -C(-C₁₋₄-alkyl)₂-, -Si(-C₁₋₄-alkyl)₂-.
G² is selected from -CH₂-, -C(O)-, -S(O)₂-, -P(O)₂-.
G³ is selected from -O-, -N(-C₁₋₄-alkyl)-.
G⁴ is selected from -OH, -O-C₁₋₄-alkyl, -NH₂, -NH-C₁₋₄-alkyl, -N(-C₁₋₄-alkyl)₂, -N-C(O)-C₁₋₄-alkyl, wherein any of the C₁₋₄-alkyl in G⁴ can be optionally substituted by W. R^{u} is selected from -H, -C₁₋₄-alkyl.

When A¹ is selected such that A'-OH is a diagnostic agent of the phenylquinazolinone class: 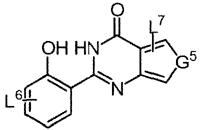 G⁵ is selected from -CH₂-, -CH=CH-, -O-, -S-, -Se-.

One or more substituent L⁶ and one or more substituent L⁷ are independently selected from -H, -C₁₋₄-alkyl, -halogen, -O-C₁₋₄-alkyl, -CF₃, -NO₂.

When A¹ is selected such that A'-OH is a diagnostic agent of the umbelliferone class:

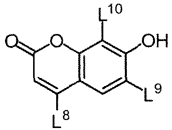

L⁸ is selected from -H, -C₁₋₄-alkyl, -halogen, -O-C₁₋₄-alkyl, -CF₃, -NO₂.

L⁹ is selected from -H, -C₁₋₄-alkyl, -halogen, -O-C₁₋₄-alkyl, -CF₃, -NO₂.

L¹⁰ is selected from -H, -C₁₋₄-alkyl, -halogen, -O-C₁₋₄-alkyl, -CF₃, -NO₂.

When A¹, A² or A³ are selected such that A'-OH or A²-NH-A³ is a therapeutic agent of the seco-cyclopropabenzaindole (CBI) class of DNA-alkylating anti-cancer drugs:

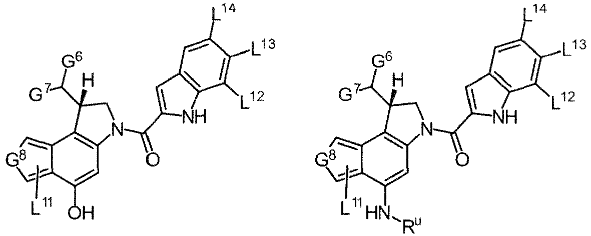

G⁶ is selected from -Cl, -Br, -I, -OMs, -OTs, -OTf, -ONs.

G⁷ is selected from -H, -C₁₋₄-alkyl, -CF₃.

G⁸ is selected from -CH=CH-, -O-, -S-.

One or more substituent L¹¹ is independently selected from -H, -F, -C₁₋₄-alkyl, -CF₃, -OMe, -O-C₁₋₄-alkyl.

L¹² and L¹³ are independently selected from -H, -F, -C₁₋₄-alkyl, -CF₃, -OMe, -O-C₁₋₄-alkyl, wherein any of the C₁₋₄-alkyl in L¹² and L¹³ can be optionally substituted by W.

L¹⁴ is selected from -O-C₁₋₄-alkyl optionally substituted by W.

When A² and A³ are selected such that A²-NH-A³ is a diagnostic or theranostic agent of the leuco-methylene blue class:

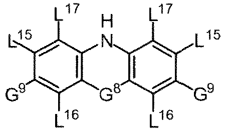

G⁸ is selected from -S-, -Se-, -Si(-C₁₋₄-alkyl)₂-, -Ge(-C₁₋₄-alkyl)₂-.

G⁹ is selected from -NH₂, -NH(-C₁₋₄-alkyl), -N(-C₁₋₄-alkyl)₂.

L¹⁵, L¹⁶ and L¹⁷ are independently selected from -H, -C₁₋₄-alkyl, -halogen, -O-C₁₋₄-alkyl, -CF₃, -NO₂.

When A¹ is selected such that A'-OH is a therapeutic agent of the camptothecin class of topoisomerase-inhibiting anti-cancer drugs:

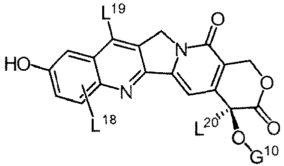

G¹⁰ is selected from -H, -C(O)-C_{alkyl}.

One or more substituent L¹⁸ are independently selected from -H, -F, -CF₃, -CH₂-CF₃, -C₁₋₄-alkyl, -CH₂-N(-C₁₋₄-alkyl)₂.

L¹⁹ is selected from -H, -F, -CF₃, -CH₂-CF₃, -C₁₋₄-alkyl, -CH₂-N(-C₁₋₄-alkyl)₂.

L²⁰ is selected from -CF₃, -CH₂-CF₃, -C₁₋₄-alkyl.

When A¹ or A² and A³ are selected such that A'-OH or A²-NH-A³ is a therapeutic agent of the nitrogen mustard class of DNA-alkylating agents:

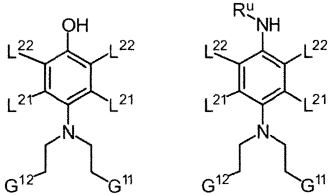

G¹¹ and G¹² are independently selected from -Cl, -Br, -I, -OMs, -OTs, -OTf, -ONs.

L²¹ and L²² are independently selected from -H, -C₁₋₄-alkyl, -halogen, -O-C₁₋₄-alkyl, -CF₃, -NO₂.

When A¹ is selected such that A'-OH is a diagnostic agent of the firefly luciferin class:

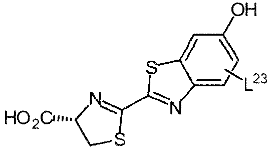

One or more substituent L²³ is selected from -H, -C₁₋₄-alkyl, -halogen, -O-C₁₋₄-alkyl, -CF₃, -NO₂.

When A¹ is selected such that A'-OH is a therapeutic agent of the Amaryllidaceae class of anti-proliferative alkaloids: 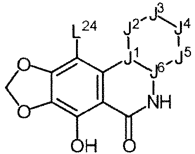 L²⁴ is selected from -H, -C₁₋₄-alkyl, -halogen, -O-C₁₋₄-alkyl, -CF₃, -NO₂.

J¹ and J⁶ are independently selected from -CH- or =C-.

J², J³, J⁴ and J⁵ are independently selected from -CH₂-, -CH(-OH)-, -CH(-O-(C(O)-C₁₋₄-alkyl))-, -CH(-O-(galactoside)-, -CH(-O-(glucoside)- or =CH-.

When A¹ is selected such that A'-OH is a diagnostic agent of the Black hole quencher class:

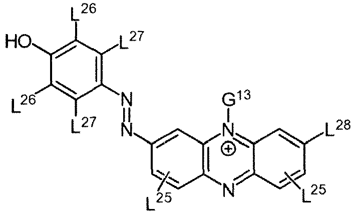

One or more substituent L²⁵ is independently selected from -H, -C₁₋₄-alkyl, -halogen, -O-C₁₋₄-alkyl, -CF₃, -NO₂.

L²⁶ and L²⁷ are independently selected from -H, -C₁₋₄-alkyl, -halogen, -O-C₁₋₄-alkyl, -CF₃, -NO₂.

L²⁸ is selected from -H, -C₁₋₄-alkyl, -halogen, -O-C₁₋₄-alkyl, -N(-C₁₋₄-alkyl)₂, -CF₃, -NO₂. G¹³ is selected from phenyl, pyridine-2-yl, pyridine-3-yl, pyridine-4-yl, 2-methoxyphenyl, 3-methoxyphenyl, 4-methoxyphenyl, 3,5-dimethoxyphenyl, 2,4-dimethoxyphenyl, 2,3,4-trimethoxyphenyl, pyrazin-2-yl.

When A² and A³ are selected such that A²-NH-A³ is a therapeutic agent of the anthracycline class:

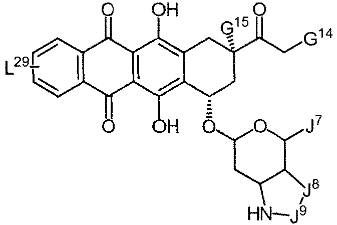

G¹⁴ is selected from H, -C₁₋₄-alkyl, -OH, -NH₂, -O-C₁₋₄-alkyl, -NH(-C₁₋₄-alkyl), -N(-C₁₋₄-alkyl)₂, -CF₃.

G¹⁵ is selected from H, -C₁₋₄-alkyl, -OH, -NH₂.

J⁷ is selected from H, -C₁₋₄-alkyl, -OH, -NH₂, -O-C₁₋₄-alkyl, -CF₃.

J⁸ is selected from H, -C₁₋₄-alkyl, -OH, -O-(tetrahydro-2H-pyran-2-yl), -NH₂, -O-C₁₋₄-alkyl, -CF₃, -CH₂-, -C(-C₁₋₄-alkyl)₂-, -O-, -S-.

If J⁸ is selected from H, -C₁₋₄-alkyl, -OH, -O-(tetrahydro-2H-pyran-2-yl), -NH₂, -O-C₁₋₄-alkyl, -CF₃, then J⁹ is selected from -CH₂-, -C(-C₁₋₄-alkyl)₂-.

If J⁸ is selected from -CH₂-, -C(-C₁₋₄-alkyl)₂-, -O-, -S-, then J⁹ is selected from -CH₂-, -C(-C₁₋₄-alkyl)₂-.

One or more substituent L²⁹ is independently selected from -O-C₁₋₄-alkyl, -OH, -halogen, -NH₂,-O-C₁₋₄-alkyl, -CF₃.

When A² and A³ are selected such that A²-NH-A³ is a therapeutic agent of the auristatin class:

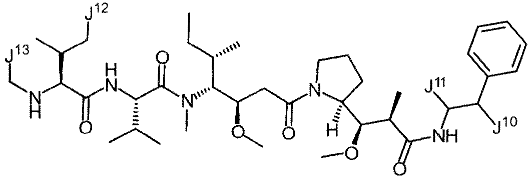

J¹⁰ is selected from H, -C₁₋₄-alkyl, -OH, -NH₂, -O-C₁₋₄-alkyl.

J¹¹ is selected from H, -C₁₋₄-alkyl, -OH, -NH₂, -O-C₁₋₄-alkyl, -C(O)-H, C(O)-O-C₁₋₄-alkyl, thiazo-2-yl, thiazo-4-yl, oxazo-2-yl, oxazo-4-yl.

J¹² is selected from H, -C₁₋₄-alkyl.

J¹³ is selected from H, -C₁₋₄-alkyl.

Illustrative embodiments of the compound having the formula (I) include

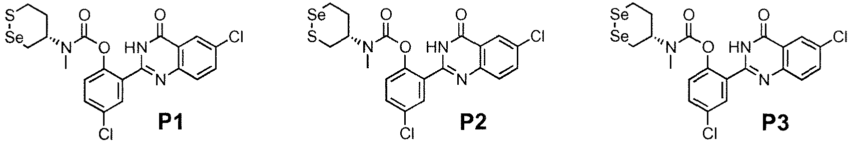

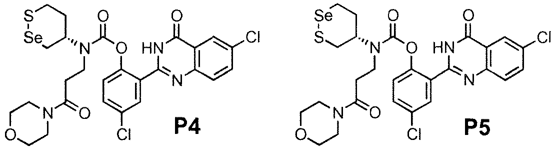

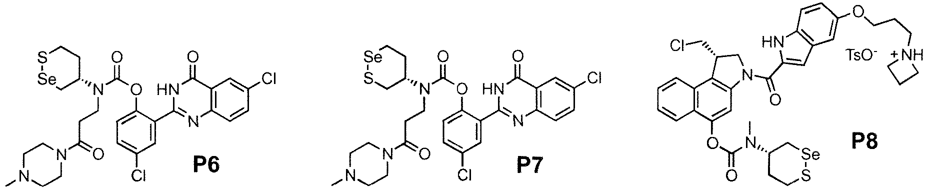

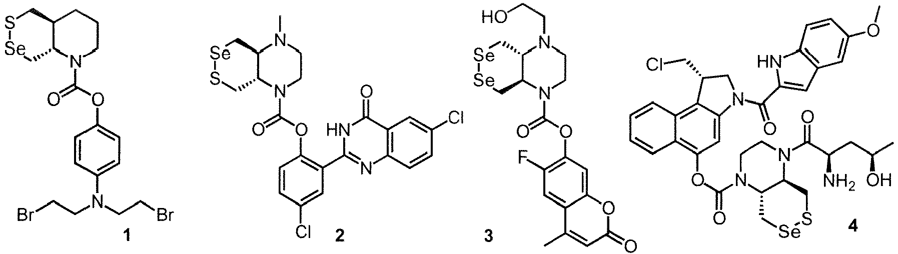

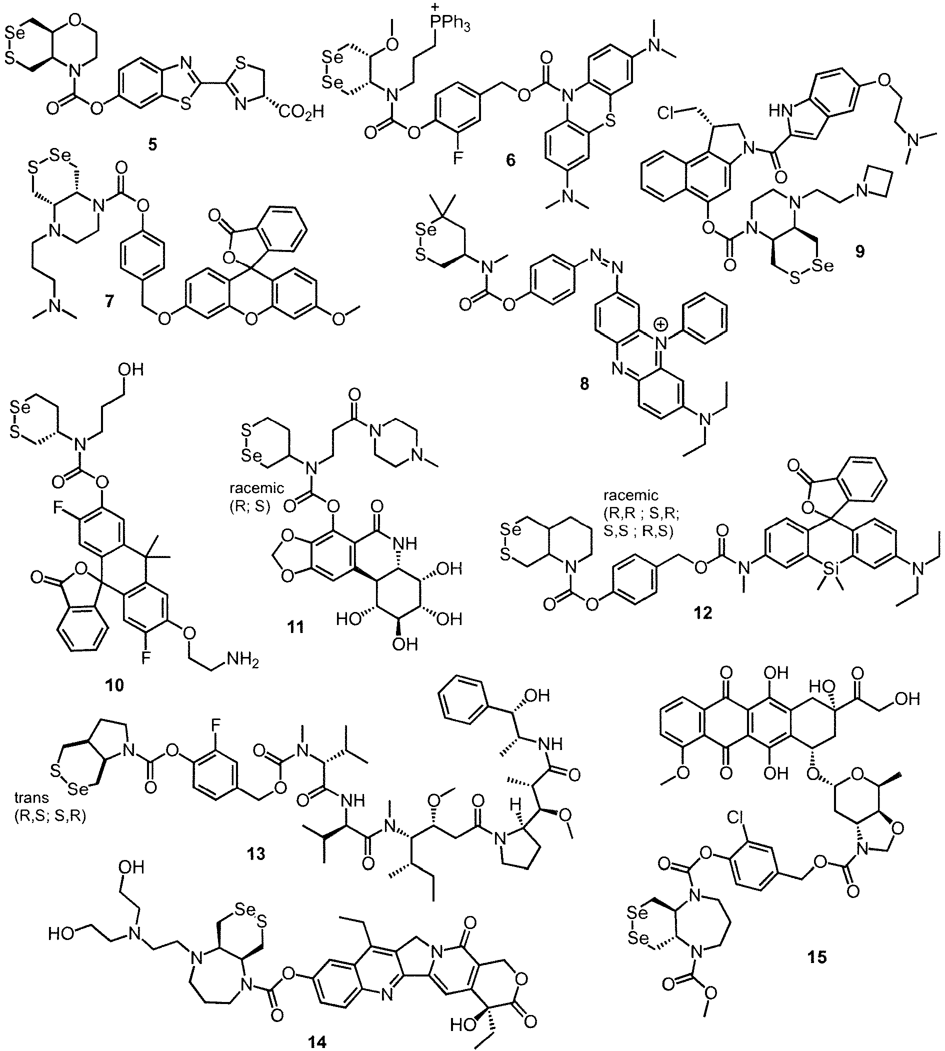

It is understood that all combinations of the above definitions and preferred definitions are envisaged by the present inventors.

### Synthesis of the compounds having the formula (I)

### A) General manufacturing of the dichalcogenide-containing moiety A

In some embodiments, general manufacturing of the compound of the invention can begin from a diol precursor similar to diol **A1**, which itself can be prepared from commercially available starting materials using literature known procedures.

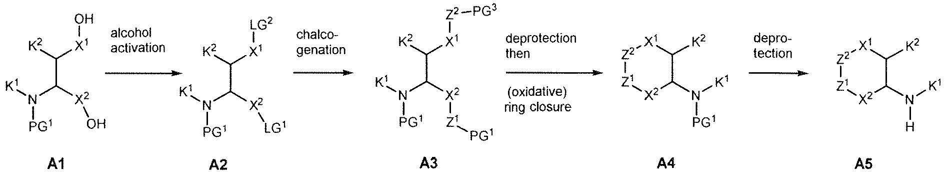

PG¹ represents any protecting group for the heterocyclic nitrogen atom, which intermediately masks the nitrogen's nucleophilic reactivity and can be cleaved without affecting other functional groups in the molecule. Preferably PG¹ can be selected from any of the following N-protecting groups: acetamide (Ac), trifluoroacetamide, *tert*-butyl carbamate (Boc), benzyl carbamate (Cbz), trityl (Trt), dimethoxy-trityl (DMT), toluenesulfonyl (Ts), *o*-nitro benzenesulfonyl (Ns), 9-fluorenmethyl carbamate (Fmoc), *p*-methoxy benzyl carbamate (Moc), benzoyl (Bz), 3,4-dimethoxybenzyl (DMPM), *p*-methoxybenzyl (PMB), *p*-methoxyphenyl (PMP), and trichloroethyl carbamate (Troc). More preferably PG¹ can be selected from one of the following *N*-protecting groups with preferred compatibility: acetamide (Ac), trifluoroacetamide, *tert*-butyl carbamate (Boc), trityl (Trt), dimethoxy-trityl (DMT), 9-fluorenmethyl carbamate (Fmoc), and trichloroethyl carbamate (Troc). Most preferably PG¹ can be selected from of following *N*-protecting groups with preferred compatibility and accessibility: acetamide (Ac), and *tert*-butyl carbamate (Boc).

Oxo-sulfur or oxo-selenium substitution can be generally achieved by any substitution reaction using any sulfur or selenium nucleophile and any activation method for alcohols. Preferably, **oxo-sulfur substitution** can be achieved by using (1) one of the following activation reagents transforming the alcohols into reactive LG¹ and LG²: *p*-toluenesulfonyl chloride (TsCl), methanesulfonyl chloride (MsCl), trifluoromethanesulfonic anhydride (Tf₂O), dialkyl azodicarboxylate activated with a trialkyl- or triarylphosphine (Mitsunobu conditions: e.g. PPh₃/DIAD), tetrahalogenmethane activated with a trialkyl- or triarylphosphine (e.g. PPh₃/CBr₄); and (2) one of the following nucleophiles: thioacetic acid (HSAc) or its sodium or potassium salt (NaSAc or KSAc), thiobenzoic acid (HSBz) or its sodium or potassium salt (NaSBz or KSBz), *p*-nitro-thiobenzoic acid or its sodium or potassium salt, *tert*-butyl mercaptan (HS-tBu), benzyl mercaptan (HS-Bn), *p*-methoxybenzyl mercaptan (HS-PMB), methanethiol (HS-Me), sodium or potassium thiocyanate or any alkyl thiol (HS-alkyl). Preferably oxo-sulfur substitution can be achieved by using MsCl or TsCl then KSAc in two separate steps or using thio-Mitsunobu conditions (PPh₃/DIAD followed by thioacetic acid, thiobenzoic acid, or *p*-nitro-thiobenzoic acid in one step). More preferably oxo-sulfur substitution can be achieved by using thio-Mitsunobu conditions of PPh₃/DIAD followed by thioacetic acid in one step.

Preferably, **oxo-selenium substitution** can be achieved by using (1) one of the following activation reagents transforming the alcohols into reactive LG¹ and LG²: TsCl, MsCl, Tf₂O, dialkyl azodicarboxylate activated with a trialkyl- or triarylphosphine (Mitsunobu conditions: e.g. PPh₃/DIAD), tetrahalogenmethane activated with a trialkyl- or triarylphosphine (e.g. PPh₃/CBr₄); and (2) one of the following nucleophiles: selenoacetic acid (HSeAc) or its sodium or potassium salt (NaSeAc or KSeAc), selenobenzoic acid (HSeBz) or its sodium or potassium salt (NaSeBz or KSeBz), *p*-nitro-selenobenzoic acid or its sodium or potassium salt, *tert*-butyl selenol (HSe-tBu), benzyl selenol (HSe-Bn), *p*-methoxybenzyl selenol (HSe-PMB) or its sodium or potassium salt (NaSePMB or KSePMB), methaneselenol (HS-Me), sodium or potassium thiocyanate or any alkyl thiol (HS-alkyl). Preferably, oxo-selenium substitution can be achieved by using mesylation or tosylation followed by sodium or potassium selenocyanate, sodium or potassium p-methoxybenzyl selenolate (NaSePMB or KSePMB) or selenium thioacetate in two separate steps or using seleno-Mitsunobu conditions (PPh₃/DIAD followed by selenoacetic acid, selenobenzoic acid, or p-nitro-selenobenzoic acid in one step). More preferably oxo-selenium substitution can be achieved by using mesylation or tosylation followed by sodium or potassium selenocyanate.

The groups PG² and PG³ represent the protecting groups for the selenium and/or sulfur atoms which are installed in **A3**.

The groups PG² and PG³ and Z¹ and Z² which are installed in **A3** by these reactions depend on the reagent or sequence of reagents and the number of equivalents of those reagents used for the oxo-sulfur or oxo-selenium substitutions, as will be known to those skilled in the art. For example, when oxo-selenium substitution is performed on both alcohol groups simultaneously with excess NaSePMB as the selenium nucleophile, the resulting **A3** will have PG² = PG³ = PMB and Z¹ = Z² = Se. Alternatively, when both alcohol groups are activated in one step but nucleophilically substituted in two separate steps with two different nucleophiles, regioisomeric products that may be separable may be obtained. For example, activating both alcohols with MsCl, and treating the product with 1 equivalent of KSAc, can depending on the conditions and the protecting groups used, allow isolation of a pair of regioisomers of the mono-S-acetylated product, and these may then be converted by reaction with e.g. KSeCN to a pair of products where one has PG² = Ac, Z¹ = S, PG³ = CN, Z² = Se and the other has PG² = CN, Z¹ = Se, PG³ = Ac, Z² = S.

Depending on the selected conditions **for oxo-sulfur substitution,** the following sulfur protecting groups can be obtained: acetate (Ac), benzoate (Bz), *p*-nitro benzoate, *tert*-butyl, methyl, alkyl, cyanide (CN) or *p*-methoxybenzyl; preferably Ac, Bz, *p*-nitro benzoate; more preferably Ac.

Depending on the selected conditions **for oxo-selenium substitution,** the following selenium protecting groups can be obtained: cyanide (CN), Ac, Bz, *p*-nitro benzoate, *tert*-butyl, methyl, alkyl or *p*-methoxy benzyl; preferably CN, Ac, Bz, *p*-nitro benzoate or *p*-methoxy benzyl; more preferably CN.

Deprotection of PG² and/or PG³ in **A3** can generally be achieved applying any appropriate deprotection conditions, which depending on PG¹ and/or PG²/PG³ may be selected from the following list of general deprotection conditions: (1) acidic deprotection: solutions of HCl in organic solvents (HCl/MeOH, HCl/EtOH, HCl/dioxane, HCl/Et₂O), or carboxylic acids (trifluoroacetic acid (TFA), trichloroacetic acid); (2) basic deprotection: piperidine, NaOH, KOH, Na₂CO₃, K₂CO₃, or Cs₂CO₃; (3) oxidative deprotection: 1,2-dichloro-4,5-dinitro-quinone (DDQ), cerium(IV) ammonium nitrate (CAN), tetrapropylammonium perruthenate, potassium perruthenate; (4) reductive deprotection: hydrogenolysis (H₂ in presence of Pd/C), diisobutylaluminium hyride (DIBAL-H), Na/NH₃, Li/naphthalene, Zn/HCl, LiAlH₄, NaBH₄, NaCNBH₃. Preferred conditions are: (1) acidic deprotection: solutions of HCl in organic solvents (HCl/MeOH, HCl/EtOH, HCl/dioxane, HCl/Et₂O) or carboxylic acids (trifluoroacetic acid (TFA), trichloroacetic acid); (2) basic deprotection: piperidine, KOH, or K₂CO₃. More preferably, acidic deprotection conditions are HCl/dioxane and basic deprotection conditions are KOH.

Deprotection conditions depending on PG¹ and/or PG² and/or PG³ can be selected, so that (1) PG¹, PG² and PG³ are cleaved consecutively giving the free dichalcogenol with a free amine, (2) so that only PG² and PG³ are cleaved giving the free dichalcogenol leaving the *N*-protecting group PG¹ intact. Alternatively, (3) only one of PG² or PG³ are cleaved, giving an *N*-protected dichalcogenol with one chalcogenol (either Z¹ or Z²) still protected. Alternatively, (4) PG¹ is cleaved and only one of PG² or PG³ are cleaved, giving a dichalcogenol with a free amine and one chalcogenol (either Z¹ or Z²) still protected.

Ring closure (formation of the dichalcogenide) can be accomplished in two ways, and based on the deprotection method employed to cleave PG¹ and/or PG² and/or PG³.

**If deprotection conditions (1) apply,** ring closure can be executed by any 2-electron oxidation conditions suitable for dichalcogenol-to-dichalcogenide oxidation to give dichalcogenide **A5** directly. Preferably these 2-electron oxidation conditions are selected from the following reaction conditions: I₂/MeOH, I₂/EtOH, I₂/DCM, O₂/MeOH, O₂/EtOH, O₂/DCM, DMSO. More preferably these are selected from the following conditions: I₂/MeOH or I₂/DCM. **If deprotection conditions (2) apply,** ring closure can be executed by any 2-electron oxidation conditions as defined above to give dichalcogenide **A4** directly.

**If deprotection conditions (3) apply,** ring closure can occur under the applied deprotection conditions to give dichalcogenide **A4** directly from **A3.**

**If deprotection conditions (4) apply,** ring closure can occur under the applied deprotection conditions to give dichalcogenide **A5** directly from **A3.**

Deprotection of **A4** can generally be achieved applying any appropriate deprotection conditions to give **A5,** which depending on PG¹ may be selected from the List of General Deprotection Conditions outlined above.

**A5** is obtained as a free amine or any acceptable ammonium salt of the same which may be selected from, but not limited to the following: acetate, benzoate, bromide, chloride, formate, iodide, nitrate, sulfate, or trifluoroacetate salt.

In some embodiments, general manufacturing of the compound of the invention can begin from diamine **A18,** which may be transformed to **A19** by any appropriate conditions, such as by reductive amination. Depending on the nature of X and Y, 6-membered piperazine or 7-membered 1,4-diazepane ring structures can be prepared by applying the respective optionally substituted dialdehyde, diketone, or ketoaldehyde precursors, which may be selected, but are not limited to the following: glyoxal, malondialdehyde, butanedione, and pentane-2,4-dione, or technically acceptable formulations which release them *in situ*. For reduction of the intermediate cyclic diimine structure any reducing agent may be used.

Preferably, a reducing agent may be selected from but is not limited to: LiAlH₄, LiBH₄, NaBH₄, NaCNBH₃, NaBH(OAc)₃, KBH₄, LiBHEt₃ or Ti(BH₄)₄(OEt₂). More preferably the cyclic diimine structure may be reduced using NaBH₄ or NaCNBH₃, most preferably using NaCNBH₃.

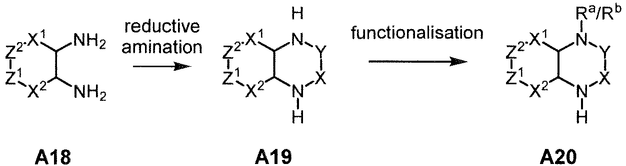

Monofunctionalisation of **A19** to achieve **A20** may be achieved by any *N*-alkylation, *N*-acylation or *N*-sulfonylation reaction with appropriate reagents, optionally with additional use of protecting groups, as necessary, and as known to those skilled in the art. Depending on the nature of R^{a}, **A19** may be treated with commercially available reagents, including but not limited to the following: For acylation: acetyl chloride, pivaloyl chloride, iPrC(O)-Cl, *n*BuC(O)Cl, methyl chloroformate, dimethyl carbamoylchloride; for sulfonylation: methanesulfonyl chloride; for alkylation: 3-(dimethylamino)propyl chloride, 3-(piperidin-1-yl)propyl chloride, 4-(azetidin-1-yl) butyl chloride, 1-(4-chlorobutyl)azetidine, 1-(3-chloropropyl)pyrrolidine, 2-(2-chloro-ethoxy)ethan-1-ol, methyl iodide, ethyl iodide. Depending on the nature of R^{b}, **A20** may be coupled with a proteinogenic amino acid to prepare a monopeptide thereof. Peptide coupling may be achieved by any appropriate coupling conditions for coupling of secondary amines with carboxylic acids. Preferably, peptide coupling may be achieved by using one of the following coupling reagents: DCC, DIC, EDCI, HATU, HBTU, PyBOP or 1-hydroxybenzotriazole. Preferably, peptide coupling may be achieved by using DCC or EDCI, most preferably by using EDCI.

### B) General manufacturing: compounds with no self-immolative linker

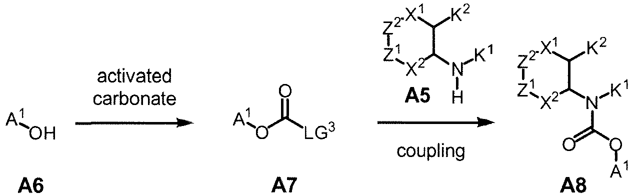

The phenolic structure **A6** represents a phenolic substructure of a motif which may be a therapeutic, diagnostic or theranostic cargo. Transformation of the phenolic alcohol function of **A6** into an activated carbonate derivative may be achieved by any appropriate method for the transformation of alcohols into activated carbonate derivatives.

Methods for the transformation of alcohols into activated carbonate derivatives include using one of the following reagents: phosgene, diphosgene (DP), triphosgene (TP), 1,1'-carbodiimidazole (CDI), *N*,*N'*-disuccinimidyl carbonate (DSC), di-2-pyridyl carbonate (DPC), dimethyl carbonate (DMC), bis(*p*-nitrophenyl) carbonate (DNPC), bis (*p*-chlorophenyl) carbonate (DCPC), phenyl chloroformate, *p*-nitrophenyl chloroformate, *p*-chlorophenyl chloroformate, or ethylene carbonate with chlorine. Preferably, the reagent is DP, TP, CDI, or p-chlorophenyl chloroformate. More preferably, the reagent is TP.

LG³ depends on the selected method for the transformation of the phenolic alcohol function of **A6** into an activated carbonate derivative and preferably may be one of the following: chloro, *N*-imidazolyl, *N'*-methyl-*N*-imidazoliumyl, succinimidyl, 2-pyridyl, methoxy, *p*-nitrophenyloxy, *p-*chlorophenyloxy, or phenyloxy. More preferably, LG³ may be one of the following: chloro, *N-*imidazolyl, or *p*-chlorophenyloxy. Most preferably, LG³ is chloro.

The activated carbonate derivative **A7** is coupled with the free amine **A5** or any acceptable ammonium salt of the same to yield **A8** by applying any appropriate reagent Suitable for Carbamate Coupling, which may be selected from the following: a trialkylamine (such as NMe₃, NEt₃, diisopropylethylamine (DIPEA), isopropyldiethylamine, triisopropylamine), pyridine, or 4-(N,N-dimethylamino)pyridine (DMAP); preferably, NEt₃ or DIPEA.

### C) General manufacturing: compounds including a benzylic self-immolative linker

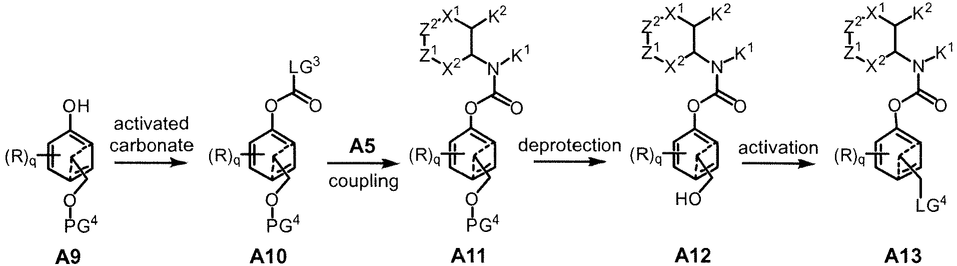

**A9** can be used to introduce an appropriate self-immolative spacer for the release of cargos. The protected benzylic alcohol can either be in *para* (1,4) or *ortho* (1,2) position relative to the phenolic alcohol. **A9** can be prepared from e.g. commercially available 2-hydroxybenzyl alcohol or 4-hydroxybenzyl alcohol using literature known methods.

PG⁴ represents any protecting group for the benzylic alcohol, which intermediately masks the oxygen's nucleophilic reactivity as needed. Preferably PG⁴ may be one of the following *O*-protecting groups: methoxymethyl ether (MOM), tetrahydropyranyl ether (THP), *tert*-butyl ether (tBu), allyl ether, benzyl ether (Bn), *tert*-butyldimethylsilyl ether (TBS), *tert-*butyldiphenylsilyl ether (TBDPS), benzoate (Bz), acetate (Ac), pivalate (Piv). More preferably, PG⁴ can be selected from any of the following *O*-protecting groups: MOM, THP, tBu, TBS.

Transformation of the phenolic alcohol function of **A9** into an activated carbonate derivative **A10** may be achieved by any appropriate method for the transformation of alcohols into activated carbonate derivatives, as outlined above. LG³ depends on the selected method, as outlined above.

The activated carbonate **A10** is coupled with the free amine **A5** or any acceptable ammonium salt of the same by applying any reagent suitable for carbamate coupling, as outlined above, yielding **A11**.

Deprotection of **A11** can generally be achieved by applying any appropriate deprotection conditions to give **A12**, which depending on PG⁴ may be selected from the General Deprotection Conditions as outlined above or from organic hydrogen fluoride solutions or any other hydrogen fluoride or fluoride source (HF/pyridine, alkali fluorides, alkaline earth fluorides, tetraalkylammonium fluorides). Preferred conditions are: organic hydrogen fluoride solutions or any other hydrogen fluoride or fluoride source (HF/pyridine, alkali fluoride, alkaline earth fluoride, tetraalkylammonium fluoride such as TBAF). More preferred are HF/pyridine or TBAF.

Activation of the benzylic alcohol **A12** for nucleophilic substitution may be achieved by any activation method for alcohols. Preferably, this may be achieved using an activation reagent selected from the following list of Reagents for the Activation of Benzylic Alcohols: *para-*toluenesulfonyl chloride (TsCl), methanesulfonyl chloride (MsCI), trifluoromethanesulfonic anhydride (Tf₂O), tetrachloromethane or tetrabromomethane or iodine activated with a trialkylphosphine or a triarylphosphine (e.g. PPh₃/CBr₄), thionyl chloride (SOCl₂), phosphoryl chloride (POCl₃), hydrochloric acid (HCl), or hydrobromic acid (HBr). Preferred are *p-*toluenesulfonyl chloride (TsCl), methanesulfonyl chloride (MsCl), thionyl chloride (SOCl₂), phosphoryl chloride (POCl₃), hydrochloric acid (HCl) or hydrobromic acid (HBr). Most preferred are methanesulfonyl chloride (MsCl) or hydrobromic acid (HBr).

Depending on the selected conditions for the activation of the benzylic alcohol, LG⁴ in **A13** is any acceptable leaving group suitable for nucleophilic substitution, such as -Cl, -Br, -I, *para-*toluenesulfonate (OTs), methylsulfonate (OMs), or trifluoromethylsulfonate (OTf). Preferably, LG⁴ is -Br, -Cl, or OMs. More preferably, LG⁴ is -Br.

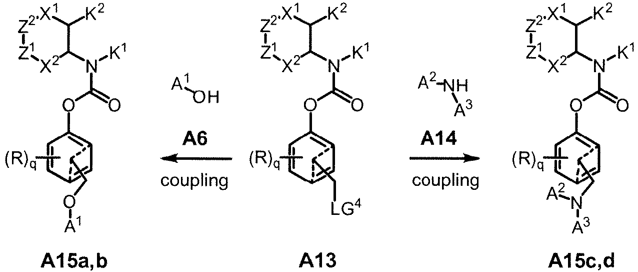

A¹ is selected, so that **A6** is a therapeutic, diagnostic or theranostic cargo of which the -OH group in A¹-OH is a phenolic or aromatic hydroxyl group. A² and A³ are selected, so that **A14** is a therapeutic, diagnostic or theranostic cargo of which the -NH- group in A²-NH-A³ is an amine or aniline group.

The reaction of **A13** with alcohol **A6** yielding benzylic ethers **A15a,b,** or the reaction of **A13** with amine **A14** yielding benzylic amines **A15c,d,** can be achieved by any appropriate method for nucleophilic substitution, which includes the use of appropriate reagents for nucleophilic substitution, which may be selected from the following: (1) alkali or alkaline earth hydrides (e.g. NaH, CaH₂), amides (e.g. NaNH₂, LDA, LHMDS, KHMDS), alkoxides (e.g. KO*t*Bu, NaOMe), hydroxides (e.g. NaOH, KOH), or carbonates (e.g. K₂CO₃, Cs₂CO₃); (2) trialkylamines (e.g. NMe₃, NEt₃, DIPEA, isopropyldiethylamine, triisopropylamine) or 4-dimethylaminopyridine (DMAP). Preferred are hydrides (NaH, CaH₂), hydroxides (NaOH, KOH), or carbonates (K₂CO₃, Cs₂CO₃). More preferred are NaH, KOH or K₂CO₃.

Alternatively, preparation of benzylic ethers **A15a,b** can be achieved from reaction of **A6** with **A12** using any appropriate conditions effecting *in situ* activation of the benzylic alcohol, including using dialkyl azodicarboxylate activated with a trialkylphosphine or triarylphosphine (Mitsunobu conditions: e.g. PPh₃/DIAD).

### D) General manufacturing: compounds including a benzyloxycarbonyl self-immolative linker

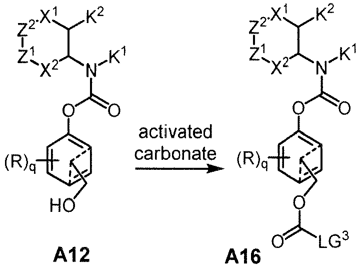

**A12** can also be used to introduce a benzyloxycarbonyl self-immolative spacer. Transformation of the benzylic alcohol function of **A12** into the activated carbonate **A16** may be achieved by any of the methods for the transformation of alcohols into activated carbonate derivatives outlined above. LG³ depends on the selected method, as outlined above.

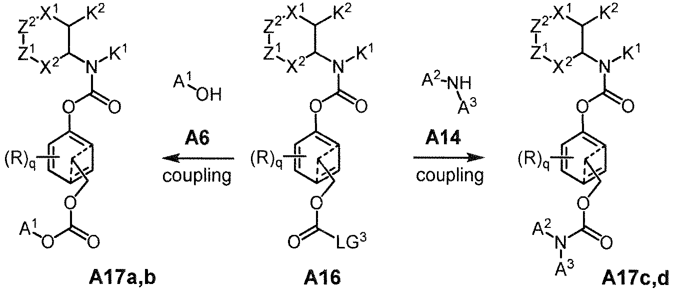

The activated carbonate **A16** is coupled with aromatic alcohol **A6** to yield **A17a,b,** or is coupled with amine **A14** or any acceptable ammonium salt of the same to yield **A17c,d,** by applying any Reagent Suitable for Carbamate Coupling as outlined above.

### Pharmaceutical or diagnostic compositions

The compounds of formula (I) can be administered to a patient in the form of a pharmaceutical or diagnostic composition which can optionally comprise one or more pharmaceutical carrier(s) or excipients.

The compounds of formula (I) can be administered by various well known routes, including oral, rectal and parenteral administration, e.g. intravenous, intramuscular, intradermal, subcutaneous, topical, and similar administration routes. Parenteral, oral and topical administration are preferred, particularly preferred is intravenous administration.

Particular preferred pharmaceutical or diagnostic forms for the administration of a compound of formula (I) are forms suitable for injectable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water buffered aqueous solutions, e.g. biocompatible buffers, ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like anti bacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum dried or freeze dried as necessary. Preferred diluents of the present invention are water, physiologically acceptable buffers, physiologically acceptable buffer salt solutions or salt solutions. Preferred carriers are cocoa butter and vitebesole. Excipients which can be used with the various pharmaceutical or diagnostic forms of a compound of the invention can be chosen from the following non limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

In one embodiment the formulation is for oral administration and the formulation comprises one or more or all of the following ingredients: pregelatinized starch, talc, povidone K 30, croscarmellose sodium, sodium stearyl fumarate, gelatin, titanium dioxide, sorbitol, monosodium citrate, xanthan gum, titanium dioxide, flavoring, sodium benzoate and saccharin sodium.

Other suitable excipients can be found in the Handbook of Pharmaceutical Excipients, published by the American Pharmaceutical Association, which is herein incorporated by reference.

The pharmaceutical or diagnostic compositions comprising a compound of the invention can be produced in a manner known per se to the skilled person as described, for example, in Remington's Pharmaceutical Sciences, 15th Ed., Mack Publishing Co., New Jersey (1991).

It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect.

The cargo is, as defined above, any therapeutic, diagnostic or theranostic agents. Depending on the therapeutic, diagnostic or theranostic agent, the mode of administration, amongst other factors, the appropriate dose spans a wide range. The determination of the appropriate dose lies within the discretion of the attending physician.

If desired, the pharmaceutical or diagnostic composition can comprise a second pharmaceutically active agent. The second pharmaceutically active agent is not limited and can be selected from a vascular disrupting agent, a cytotoxic chemotherapeutic agent and an immunomodulator. Examples thereof include combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.

### Mechanism of Reductive Release

The mechanism by which reductive cleavage of the dichalcogenide leads to release of the cargo agent, is shown in the following schemes depicting the release of A'-OH (phenol-type) or A²-NH-A³ (amine-type) cargos from compounds of the invention. Without wishing to be bound by theory, it is assumed that the reduction in cells of the dichalcogenide bond of the compounds of the invention could be performed by a reducing enzyme or protein with a dithiol or selenolthiol active site, for example, an oxidoreductase such as thioredoxin reductase 1 (TrxR1), or TrxR2, MsrB1, or GR; or an effector protein, such as thioredoxin 1 (Trx1), Trx2, Grx1, Grx2, PDIA1, DsbA, DsbB, or DsbC.
1. When L is a bond: 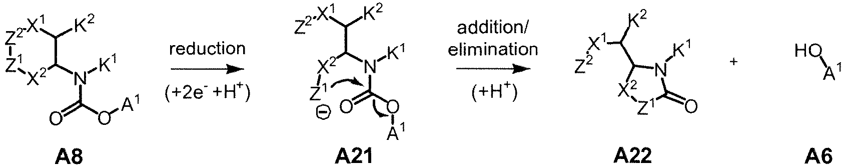 When L is a bond, the phenolic cargo A'-OH is directly linked to A as a carbamate, giving compounds of type **A8**. Upon reduction of the dichalcogenide bond in **A8,** the reduced thiol/selenolate (for Z₂=S and Z₁=Se) or selenol/thiolate (for Z₂=Se and Z₁=S) or selenol/selenolate (for Z₂=Z₁=Se) species **A21** is formed. **A21** can intramolecularly cyclise *via* an addition/elimination mechanism, which irreversibly releases the free phenolic cargo (i.e. therapeutic, diagnostic or theranostic agent) A¹-OH (**A6**) as well as the byproduct carbamoselenoate (for Z₁ = Se)/carbamothioate (for Z₁=S) species **A22**.
2. When L is a benzylic (1,4)- or (1-6)-self-immolative linker which is linked to A as a phenolic carbamate, and is connected to B as a benzylic ether or benzylamine

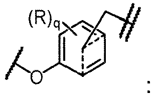

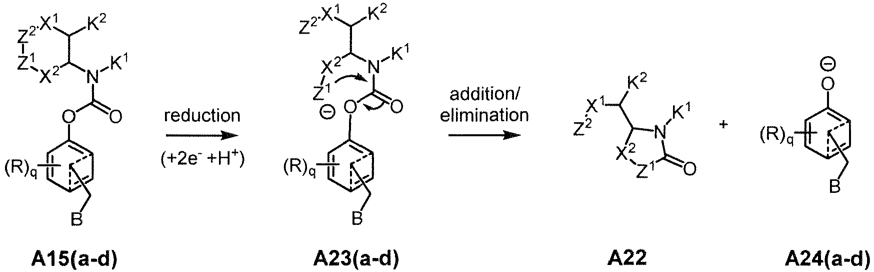

General Mechanism: When L is a benzylic (1,4)- or (1,6)-self-immolative linker which is linked to A as a carbamate, and is connected to B as a benzylic ether or benzylamine, then B can be selected from phenol-type A'-OH or amine-type A²-NH-A³ cargos, giving compounds **A15(a-d)** as defined below. Upon reduction of the dichalcogenide bond in **A15(a-d),** the corresponding reduced thiol/selenolate (for Z₂=S and Z₁=Se) or selenol/thiolate (for Z₂=Se and Z₁=S) or selenol/selenolate (for Z₂=Z₁=Se) species **A23(a-d)** is formed. **A23(a-d)** can intramolecularly cyclise *via* an addition/elimination mechanism, which irreversibly releases the corresponding intermediate phenolic species **A24(a-d)** as well as carbamoselenoate (for Z₁ = Se)/carbamothioate (for Z₁=S) byproduct **A22.** The nature of compounds **A15(a-d)** and how the corresponding intermediate **A24(a-d)** releases the cargo B are shown below:
When L is the (1,6)-self-immolative linker 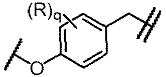 and B is the phenolic cargo

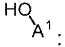

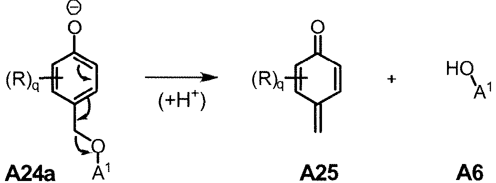

In compounds of type **A15a,** L is a benzylic (1,6)-self-immolative linker and B is a phenolic A¹-OH cargo which is connected to the linker as a benzylic ether. Reduction of **A15a** gives **A23a** which after cyclisation gives intermediate **A24a. A24a** can perform an intramolecular 1,6-elimination releasing the quinone-methide species **A25** and the free phenolic cargo **A6.**

When L is the (1,4)-self-immolative linker 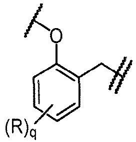 and B is the phenolic cargo

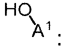

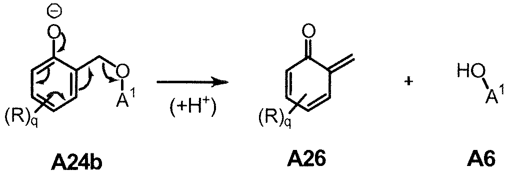

In compounds of type **A15b,** L is a benzylic (1,4)-self-immolative linker and B is a phenolic A'-OH cargo, which is connected to the linker as a benzylic ether. Reduction of **A15b** gives **A23b** which after cyclisation gives intermediate **A24b. A24b** can perform an intramolecular 1,4-elimination releasing the quinone-methide species **A26** and the free phenolic cargo **A6.**

When L is the (1,6)-self-immolative linker 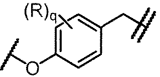 and B is the amine-type cargo

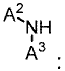

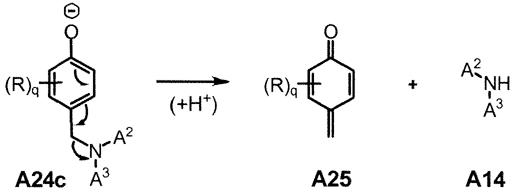

In compounds of type **A15c,** L is a benzylic (1,6)-self-immolative linker and B is an amine-type A²-NH-A³ cargo, which is connected to the linker as a benzylamine. Reduction of **A15c** gives **A23c** which after cyclisation gives intermediate **A24c. A24c** can follow an intramolecular 1,6-elimination releasing the quinone-methide species **A25** and the free amine-type cargo (i.e., therapeutic, diagnostic or theranostic agent) **A14.**

When L is the (1,4)-self-immolative linker 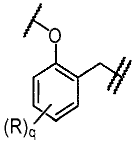 and B is the amine-type cargo

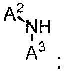

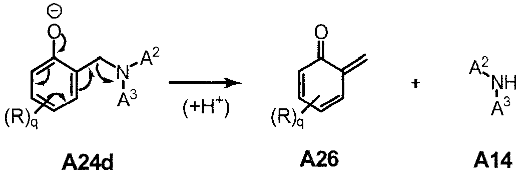

In compounds of type **A15d,** L is a benzylic (1,4)-self-immolative linker and B is an amine-type A²-NH-A³ cargo, which is connected to the linker as a benzylamine. Reduction of **A15d** gives **A23d** which after cyclisation gives intermediate **A24d. A24d** can perform an intramolecular 1,6-elimination releasing the quinone-methide species **A26** and the free amine-type cargo **A14.**

### 3. When L is a benzylic (1,4)- or (1-6)-self-immolative linker which is linked to A as a phenolic carbamate, and is connected to B as a benzylic carbonate or carbamate

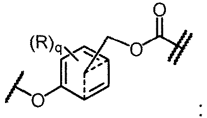

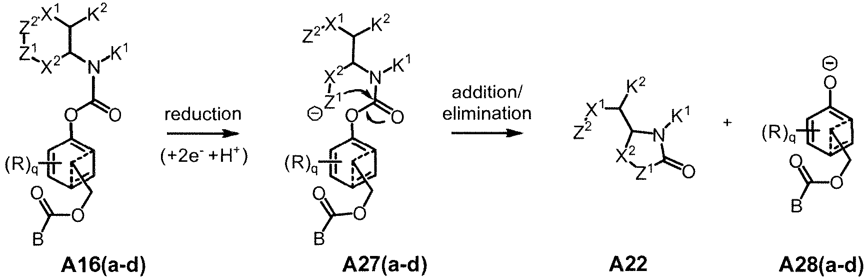

General Mechanism: When L is a benzylic (1,4)- or (1,6)-self-immolative linker which is linked to A as a carbamate, and is connected to B as a benzylic carbonate or carbamate, then B can be selected from phenol-type A'-OH or amine-type A²-NH-A³ cargos, giving compounds **A16(a-d)** as defined below. Upon reduction of the dichalcogenide bond in **A16(a-d),** the corresponding reduced thiol/selenolate (for Z₂=S and Z₁=Se) or selenol/thiolate (for Z₂=Se and Z₁=S) or selenol/selenolate (for Z₂=Z₁=Se) species **A27(a-d)** is formed. Depending on the redox microenvironment and local pH **A27(a-d)** can intramolecularly cyclise *via* an addition/elimination mechanism, which irreversibly releases the corresponding intermediate phenolic species **A28(a-d)** as well as carbamoselenoate (for Z₁ = Se)/carbamothioate (for Z₁=S) byproduct **A22**. The nature of compounds **A16(a-d)** and how the corresponding intermediate **A28(a-d)** releases the cargo B are shown below:

When L is the (1,6)-self-immolative linker 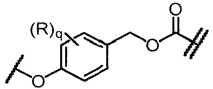 and B is the phenolic cargo

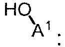

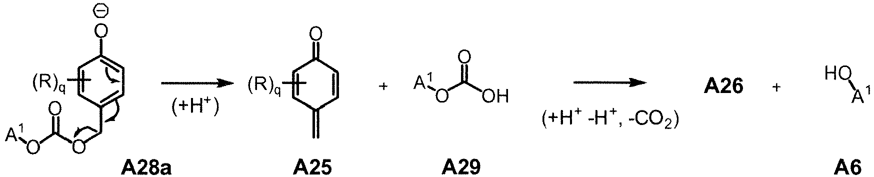

In compounds of type **A16a,** L is a benzylic (1,6)-self-immolative linker and B is a phenolic A¹-OH cargo which is connected to the linker as a benzylic carbonate. Reduction of **A16a** gives **A27a** which after cyclisation gives intermediate **A28a. A28a** can perform an intramolecular 1,6-elimination releasing the quinone-methide species **A25** as well as the carbonic acid species **A29** which further eliminates carbon dioxide to give the free phenolic cargo **A6.**

When L is the (1,4)-self-immolative linker 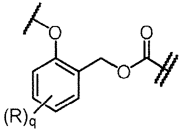 and B is the phenolic cargo

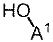

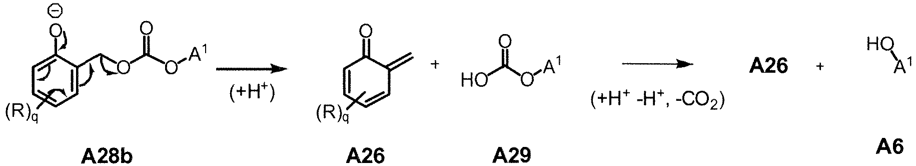

In compounds of type **A16b,** L is a benzylic (1,4)-self-immolative linker and B is a phenolic A'-OH cargo which is connected to the linker as a benzylic carbonate. Reduction of **A16b** gives **A27b** which after cyclisation gives intermediate **A28b. A28b** can perform an intramolecular 1,4-elimination releasing the quinone-methide species **A26** as well as the carbonic acid species **A29** which further eliminates carbon dioxide to give the free phenolic cargo **A6.**

When L is the (1,6)-self-immolative linker 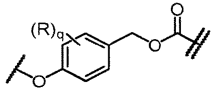 and B is the amine-type cargo

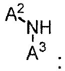

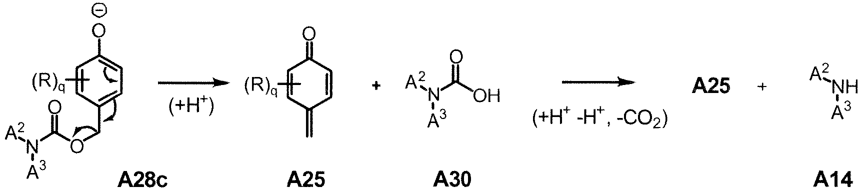

In compounds of type **A16c,** L is a benzylic (1,6)-self-immolative linker and B is an amine-type cargo A²-NH-A³ which is connected to the linker as a benzylic carbamate. Reduction of **A16c** gives **A27c** which after cyclisation gives intermediate **A28c. A28c** can perform an intramolecular 1,6-elimination releasing the quinone-methide species **A25** as well as the carbamic acid species **A30** which further eliminates carbon dioxide to give the free phenolic cargo **A14.** When L is the (1,4)-self-immolative linker 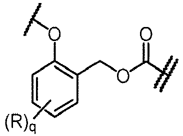 and B is the amine-type cargo

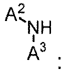

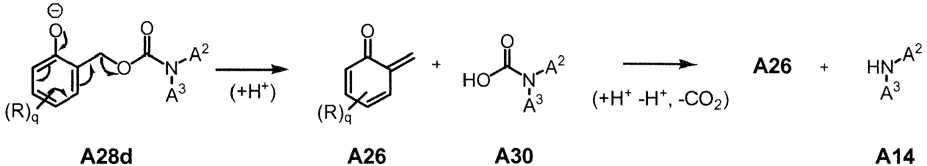

In compounds of type **A16d,** L is a benzylic (1,4)-self-immolative linker and B is an amine-type cargo A²-NH-A³ which is connected to the linker as a benzylic carbamate. Reduction of **A16d** gives **A27d** which after cyclisation gives intermediate **A28d. A29d** can perform an intramolecular 1,4-elimination releasing the quinone-methide species **A26** as well as the carbamic acid species **A30** which further eliminates carbon dioxide to give the free phenolic cargo **A14**.

Without wishing to be bound by theory, it is assumed that the specific cyclic structure common to the presently claimed compounds can ensure that the dichalcogenide bond is stabilized against nonspecific reduction e.g. by cellular monothiols such as GSH under physiological conditions.

It is furthermore assumed that in some of the claimed compounds this stabilised dichalcogenide is reductively cleaved selectively by certain reductants, such as one or more oxidoreductases or redox effector proteins, and therefore that cargo release can be used as a quantification method for the activity of those reductants, which is a goal of probe development in diagnostics and in research. Such compounds are also useful in the development of candidate inhibitor drugs for those reductants, as the compound can be used to read out the degree of inhibition of the reductant that the candidate causes.

It is furthermore assumed that in some of the claimed compounds this stabilised dichalcogenide is reductively cleaved selectively in cells with dysregulated redox properties (including cells located in hypoxic environments, and cells with upregulated oxidoreductases) such as tumor cells and cells located in inflammatory microenvironments. Therefore, the effect of the agent that is released after reductive cleavage can be localized to pathological cells and environments which ensures that healthy cells and tissues are not exposed to similar levels of the agent. This pathology-specificity allows the presently claimed compounds which include a therapeutic or theranostic agent to be used as therapeutics for pathological cells and environments; and allows the presently claimed compounds which include a diagnostic or theranostic agent to be used as diagnostics for pathological cells and environments. Such selectivity is a longstanding goal of prodrug and diagnostic development, and offers a significant advantage compared to prior art dichalcogenide-reduction-triggered compound designs which are activated by typical cellular conditions without specificity for pathological cells (see e.g. Hyman, L. M. et al. Coordination Chemistry Reviews 2012, 256, 2333-2356. https://doi.org/10.1016/j.ccr.2012.03.009).

### Utility, Diseases, and Examples

The compounds having the formula (I) are particularly useful as therapeutics or diagnostics.

In one embodiment, the present invention provides a (typically *in vitro*) method of determining (preferably quantifying) an inhibitory activity of a candidate inhibitor or candidate drug upon an oxidoreductase and/or a redox effector protein. The candidate inhibitor can be any compound which is to be tested to determine whether it can inhibit an oxidoreductase and/or a redox effector protein. The candidate drug is not particularly limited either. Examples of candidate drugs include any compound which is to be tested to determine whether it is suitable for treating, ameliorating, preventing or diagnosing a disorder selected from a neoplastic disorder; atherosclerosis; an autoimmune disorder; an inflammatory disease; a chronic inflammatory autoimmune disease; ischaemia; and reperfusion injury. Preferably the candidate drug is a compound which is assumed to be suitable for inhibiting an oxidoreductase and/or a redox effector protein.

In a first step, a compound having the formula (I), or a pharmaceutically acceptable salt, solvate, or ester thereof, wherein A¹ is selected such that A'-OH is a diagnostic or theranostic agent or A² and A³ are independently selected such that A²-NH-A³ is a diagnostic or theranostic agent, is contacted with the oxidoreductase and/or the redox effector protein as well as with the candidate inhibitor or candidate drug. The method of contacting the compound having the formula (I), or a pharmaceutically acceptable salt, solvate, or ester thereof, with the oxidoreductase and/or the redox effector protein as well as with the candidate inhibitor or candidate drug is not particularly limited. Typically the three components are mixed in a solvent such as water, typically with buffered pH, typically in the presence of a small amount of organic cosolvent (such as PBS, pH = 7, containing 1% DMSO).

Subsequently, the sample is imaged according to the imaging modality of the diagnostic or theranostic agent, for instance, by fluorescence imaging or luminescence imaging, to determine whether the compound (I) of the present invention, or a pharmaceutically acceptable salt, solvate, or ester thereof, has been reductively cleaved to release A'-OH or A²-NH-A³. Depending on the imaging method, the detection can be quantitative or qualitative. In a preferred embodiment, A'-OH or A²-NH-A³ is a fluorescent agent which can be imaged by fluorescence imaging.

The more the compound of the present invention, or a pharmaceutically acceptable salt, solvate, or ester thereof, has been reductively cleaved the less the candidate inhibitor is suitable for inhibiting the oxidoreductase and/or the redox effector protein. The less the compound of the present invention, or a pharmaceutically acceptable salt, solvate, or ester thereof, has been reductively cleaved the more the candidate drug is suitable for treating the recited disorders and/or for inhibiting the oxidoreductase and/or the redox effector protein.

The extent to which the compound (I) of the present invention is reductively cleaved to release A'-OH or A²-NH-A³ is not particularly limited. For instance, if at most about 30%, preferably at most about 5% of the compound (I) of the present invention, or a pharmaceutically acceptable salt, solvate, or ester thereof, is reductively cleaved, then it can be assumed that the candidate inhibitor will be suitable as an inhibitor of the oxidoreductase and/or the redox effector protein. For example, if at most about 50%, preferably at most about 10% of the compound (I) of the present invention, or a pharmaceutically acceptable salt, solvate, or ester thereof, are reductively cleaved, then it can be assumed that the candidate drug will be suitable for treating, ameliorating, preventing or diagnosing the recited disorder or will be suitable as an inhibitor of the oxidoreductase and/or the redox effector protein.

The method of the above embodiment can be used as part of e.g. biochemical and biological research in diverse settings, wherein such compounds of the invention are valuable probes.

The compounds having the formula (I) can be used in the diagnosis, treatment, amelioration or prevention of a disorder selected from a neoplastic disorder; atherosclerosis; an autoimmune disorder; an inflammatory disease; a chronic inflammatory autoimmune disease; ischaemia; and reperfusion injury.

Preferably, the disorder is a neoplastic disorder, in particular cancer. The cancer is not particularly limited, preferably the cancer is selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma.

The therapeutic, diagnostic or theranostic compounds having the formula (I) can be used in combination with one or more other therapeutics, diagnostics or theranostics. The type of the other therapeutics, diagnostics or theranostics is not particularly limited and will depend on the disorder to be treated and/or diagnosed. Preferably, other therapeutics can enhance the selectivity and/or the turnover of the compound of formula (I) in the targeted tissue type; for example, the other therapeutic may be an agent that can stimulate transient hypoxia in tumors and thereby enhance the tumor-specific release of the cargo of the compound of formula (I) (which may be a therapeutic, diagnostic, or theranostic). Preferably in the case of compounds of formula (I) which are therapeutics and theranostics releasing a cargo used to treat a disorder, the other medicaments will be further therapeutics which are used to treat the same disorder; for example, further therapeutics that can treat a neoplastic disorder such as cancer may be applied in combination with compounds of formula (I) that are therapeutic proagents releasing a cargo that can treat a neoplastic disorder such as cancer.

The one or more other medicament, diagnostic or theranostic can be administered prior to, after, or simultaneously with the compound having formula (I). In another preferred embodiment, the other medicament can be an agent that can stimulate transient hypoxia in tumors which can be administered prior to, after or simultaneously to administration of compound of formula (I).

Agents which are capable of stimulating hypoxia in diseased tissues include but are not limited to vascular disrupting agents (including colchicine domain tubulin inhibitors such as combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P), and pharmaceutically acceptable salts of the same), vascular-acting drugs such as vasodilator or antihypertensive drugs (including hydralazine), and inhibitors of prolyl hydroxylase or stabilizers of hypoxia-inducible factor 1 such as daprodustat, desidustat and molidustat.

For *in vivo* usage, a diagnostic compound of the invention is administered to a subject or patient by any acceptable route, preferably *i.v.*, *i.p.* or *per os*, and the subject or patient is imaged thereafter according to the imaging modality of the cargo, preferably by fluorescence imaging, photoacoustic imaging, luminescence imaging, or positron emission tomography. For usage *in vitro,* a diagnostic compound of the invention is administered to a test sample which, for instance, has been obtained from a subject, and the test sample is imaged thereafter according to the imaging modality of the cargo, preferably by fluorescence imaging, absorbance, or luminescence imaging; the sample may preferably be cells, a tissue section, a small animal model or a biopsy sample.

The compounds of the present invention can be used to predict the suitability of a compound of the invention for treating a patient who is suffering from a disorder selected from a neoplastic disorder; atherosclerosis; an autoimmune disorder; an inflammatory disease; a chronic inflammatory autoimmune disease; ischaemia; and reperfusion injury.

In a first step, a sample of the effected tissue or body fluid is obtained from the patient. The sample is then contacted with a compound (I*) of the present invention, wherein A is A*, L is L* and A¹ is selected such that A'-OH is a diagnostic or theranostic agent or A² and A³ are independently selected such that A²-NH-A³ is a diagnostic or theranostic agent. Subsequently, the sample is imaged according to the imaging modality of the diagnostic or theranostic agent, for instance, by fluorescence imaging, photoacoustic imaging, luminescence imaging, or positron emission tomography, to determine whether the compound (I*) of the present invention has been reductively cleaved to release A'-OH or A²-NH-A³. If compound of the present invention has been reductively cleaved then it can be assumed that a compound (I**) of the present invention which has the same moieties A* and L* as the compound (I*) but in which A'-OH is a therapeutic or theranostic agent or A² and A³ are independently selected such that A²-NH-A³ is a therapeutic or theranostic agent will be effective for treating the patient. The theranostic agent which is used in the compounds (I*) and (I**) can be the same or different.

The extent to which the compound (I*) of the present invention is reductively cleaved to release A'-OH or A²-NH-A³ is not particularly limited. It should be sufficient to provide a therapeutically effective amount of A¹-OH or A²-NH-A³ to the tissue or body fluid. For instance, 0.1-100 %, preferably 0.5-50 % of the compound (I*) of the present invention can be reductively cleaved.

In the following, examples of preferred compounds having the formula (I) are described, to illustrate some of the ways in which conjugation of a cargo H-B as the compound A-L-B masks its diagnostic and/or therapeutic activity, until the cargo is released following reduction.

These examples include the following therapeutic designs:
1 releases a DNA-alkylating nitrogen mustard cargo subunit of a type which is of interest especially for cancer therapy. The released bis(2-haloethyl)aniline species will feature similar activity to PR-104's bioactive alkylating metabolite PR-104M whereas the intact species 1 will not be as potently alkylating as the released cargo, since unmasking the electron-donating phenol in *para* to the aniline nitrogen increases electronic density and alkylating reactivity (similar principles are known for PR-104 and a range of related prodrugs; Sharma, A. et al. Chem. Soc. Rev. 2019, 48, 771-813. https://doi.org/10.1039/C8CS00304A). Note that a range of leaving groups (halogens -Cl, -Br, -I or other leaving groups such as -OS(O)₂CH₃) can be used as the halogen substituents of the 2-haloethyl groups without compromising functionality, and/or an aniline nitrogen (requiring the use of a spacer subunit) could be used instead of the phenolic hydroxyl as the unmasked group of the cargo, while remaining within the scope of a compound having the formula (I).
**4** and **9** are similar to compound **P8** in that they release cargo subunits that are analogues of 5-hydroxy-*seco*-cyclopropabenzaindole, which (when their phenolic hydroxyl is unmasked) can then undergo Winstein cyclisation to the active DNA-alkylating cyclopropabenzaindoles (CBIs), which are of interest especially for cancer therapy, whereas no Winstein cyclisation can take place until the phenol is unmasked. In this way only the released cargo (and not the proagents) will be bioactive (similar principles are known for a range of related prodrugs; Twum, E. A. et al. Bioorganic & Medicinal Chemistry 2015, 23, 3481-3489. https://doi.org/10.1016/j.bmc.2015.04.034). Note that a range of analogues of 5-hydroxy- or 5-amino-seco-cyclopropabenzaindoles or related cargos with various substitution motifs (such as the inclusion of the dimethylamino substituent on **9** to enhance solubility and DNA-binding potency) could be used, while remaining within the scope of a compound having the formula (I).
**11** releases a cargo subunit that is the Amaryllidaceae-type alkaloid pancratistatin. Amaryllidaceae-type alkaloids (such as pancratistatin or narciclasine) are metabolism-affecting drugs that have shown utility in disease indications featuring metabolic deregulation, from cancer to chronic inflammation (Lubahn, C. et al. Rheumatology International 2012, 32, 3751-3760. https://doi.org/10.1007/s00296-011-2217-z; McNulty, J. et al. J. Nat. Prod. 2008, 71, 357-363. https://doi.org/10.1021/np0705460). The cargo release process (following triggering of the dichalcogenide trigger subunit of **11**) removes steric hindrance and so allows binding of the lycorane to its molecular target, such that only the released cargo is bioactive. Note that a range of lycorane-type alkaloids with various substitution motifs could be used, while remaining within the scope of a compound having the formula (I).
**14** releases a cargo subunit that is SN-38, the active metabolite of the camptothecin class topoisomerase inhibitor irinotecan, a class which also contains the agent topotecan. As known for irinotecan, blocking the phenol removes bioactivity of the proagent so **14** will be biologically inactive except if triggered to release cargo; note that a range of similar cargo structures (e.g. modifying the ethyl group at the 7-position, or introduction of solubility and potency enhancing groups such as (dimethylamino)methyl as seen in topotecan) could be used, as is known for a range of related prodrugs (Sharma, A. et al. Chem. Soc. Rev. 2019, 48, 771-813. https://doi.org/10.1039/C8CS00304A), while remaining within the scope of a compound having the formula (I).

It is illustrated by **1**, **4**, **9**, **11** and **14** that a broad range of bioactive phenolic cargos with a range of different bioactivity mechanisms and disease indication scopes can therefore be used, including with extensive structural substitutions, within the scope of the invention to deliver cargo-release-based activation of proagent bioactivity, i.e. functional prodrugs dependent upon triggering of the key selenenylsulfide of a compound having the formula (I).

**13** releases an amine cargo subunit that is a tubulin-inhibiting auristatin derivative, of particular interest for cancer therapy and for neoangiogenic vasculopathies. A range of auristatins (and related peptide tubulin binders such as dolastatins and tubulysins) have been used in prodrugs where bioactivity is suppressed by conjugation of the agent to a large but cleavable trigger and/or trigger+spacer subunit (Singh, Y. et al. Curr Med Chem 2008, 15, 1802-1826), then restored upon its removal. Note that a range of such agents with various substitution patterns (including monomethyl auristatin E, monomethyl auristatin F, dolastatin or symplostatin) could be used; and/or that beyond the 3-fluoro-4-hydroxybenzyl alcohol spacer depicted in **13**, a range of spacers could instead be used, while remaining within the scope of a compound having the formula (I), to activate cytotoxic activity upon triggering of the proagent.

**15** releases an amine cargo subunit that is of the anthracycline class of topoisomerase-inhibitors. This class includes doxorubicin, daunorubicin and idarubicin, and is of particular interest for cancer therapy. Basicity of the aliphatic amine of doxorubicin and its analogues is important for bioactivity; its carbamylation in **15** blocks bioactivity, but bioactivity can be restored by diselenide triggering followed by spacer-mediated release of the doxorubicin analogue (Singh, Y. et al. Curr Med Chem 2008, 15, 1802-1826). Note that a range of related cargos with various substitution motifs and/or related spacers could instead be used, while remaining within the scope of a compound having the formula (I), to activate cytotoxic activity upon triggering of the proagent.

It is illustrated by **13** and **15** that a broad range of bioactive amine cargos with different bioactivity mechanisms and disease indication scopes can therefore be used in conjunction with a range of spacers, each with optional extensive structural substitutions, while remaining within the scope of the invention to deliver cargo-release-based activation of proagent bioactivity, i.e. functional prodrugs dependent upon triggering of the key dichalcogenide of a compound having the formula (I).

These examples include the following diagnostic designs:
**2** releases a fluorescent precipitating phenolic cargo subunit with large Stokes shift and green emission; **3, 7** and **10** release fluorescent phenolic cargos with small Stokes shifts and UV, cyan, or orange emission; all the proagents **2, 3, 7** and **10** are nonfluorescent due to masking of the phenol groups (either due to blocking excited-state intramolecular proton transfer in **2,** or reducing electron-donating capacity in **3,** or blocking the opening of the spirolactone in **7** and **10**); and all these designs provide a signal turn-on upon cargo release stimulated by triggering of the trigger subunits (and similar principles are known for a range of related probe cargos; Thorn-Seshold, O. et al. Chem. Commun. 2012, 48, 6253-6255. https://doi.org/10.1039/C2CC32227G); they are thus diagnostic fluorescence probes that may be useful in a variety of diagnostic purposes. It is also illustrated that spacer subunits can be optionally included (**7**), and that a range of cargos [e.g. **2** (2-hydroxyphenyl)quinazolin-4(3*H*)-one), **3** (coumarin), **7** and **10** (xanthene fluorophores of the fluorescein type)] with a range of structures and diagnostic properties are possible while remaining within the scope of a compound having the formula (I).
**5** releases a phenolic cargo subunit that is D-luciferin, the substrate of firefly luciferase enzyme, whereas the intact probe **5** is too bulky to be a substrate of luciferase. Therefore 5 is a dichalcogenide-reduction-triggered diagnostic luminescence probe allowing detection by bioluminescence in the presence of luciferase, which may be useful as a diagnostic for reductive probe turnover with ultra-low background signal (high sensitivity). Note that a range of luciferins for different luciferases and with different structural modifications are known and could also be used as cargo subunits (Kaskova, Z. M. et al. Chem. Soc. Rev. 2016, 45, 6048-6077. https://doi.org/10.1039/C6CS00296J) while remaining within the scope of a compound having the formula (I).
**8** releases a phenolic cargo subunit that is a functional analogue of Black Hole Quencher 3 (BHQ3) which has fluorescence quenching and photoacoustic signal generation properties (Chevalier, A. et al. Chem.-Eur. J 2013, 19, 1686-1699. https://doi.org/10.1002/chem.201203427). The proagent **8** is non-signal-generating and non-quenching due to masking of the key electron-donating phenol group, while the released cargo has no masking of the phenol and therefore has fluorescence quenching and photoacoustic signal generation activity, thus **8** functions as a multimodal diagnostic proagent. Note that similar unmasking-based activity turn-on principles are known for a range of related compounds including other quenchers, which could also be used as cargo subunits while remaining within the scope of a compound having the formula (I).
**12** releases a spacer-cargo system that fragments to release an aniline cargo subunit that is a silarhodamine derivative which has fluorescence and photoacoustic signal generation properties (Myochin, T. et al. J. Am. Chem. Soc. 2015, 137, 4759-4765. https://doi.org/10.1021/jacs.5b00246). The proagent **12** is non-signal-generating and nonfluorescent due to the masking of the amine group by carbamylation, while the released cargo silarhodamine is active in both diagnostic modalities. Note that a range of related or analogous cargos with various substitution motifs (such as a range of aniline-based fluorophores or photoacoustics dyes, including rhodols and rhodamines) could also be used as cargo subunits along the same design principles while remaining within the scope of a compound having the formula (I).

It is illustrated by **2**, **3**, **5**, **7**, **8**, **10** and **12** that a broad range of diagnostically active phenol or amine cargos, eabling a range of structures and diagnostic detection methods, can therefore be used, including with extensive structural substitutions, within the scope of the invention, to deliver cargo-release-based activation of proagent diagnostic activity, i.e. functional diagnostic probes dependent upon triggering of the key dichalcogenide of a compound having the formula (I).

These examples also include the theranostic design **6** that releases a cargo subunit that is leuco-methylene blue, a reduced form of the theranostic compound methylene blue which after release in biological media is spontaneously converted to methylene blue (similar principles are known for a range of related prodrugs). Methylene blue can be useful therapeutically including as a photosensitiser in photodynamic therapy (e.g. of the autoimmune disorder psoriasis, or of cancers) and/or as a redox-active selective toxin (e.g. against malaria parasite); as well as diagnostically (e.g. as a fluorescent agent or a photoacoustic dye); therefore being capable of theranostic activity (Schirmer, R. H. et al. Neurobiology of Aging 2011, 32, 2325.e7-2325.e16. https://doi.org/10.1016/j.neurobiolaging.2010.12.012). Note that a range of functionally and structurally related compounds (e.g. phenoxazines) can also be used as cargo subunits in theranostics following the same design principles while remaining within the scope of a compound having the formula (I).

Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention, as far as they fall under the claims.

### Examples

The invention will be more fully understood by reference to the following examples. They should not, however, be construed as limiting the scope of the invention.

### 1. Abbreviations

- A549:: human lung cancer cell line A549

- aq.:: aqueous (solution)
- 4T1:: Balb/c mouse breast cancer cell line
- Boc:: *tert*-butoxycarbonyl
- calc:: calculated
- DCM:: dichloromethane
- DIAD:: DIAD
- DMF:: dimethylformamide
- DMSO:: dimethylsulfoxide
- EDCI:: 1-(3-dimethylaminopropyl)-3-ethylcarbodiimide
- EI:: electron ionization
- ESI:: electron spray ionization
- FCC:: flash column chromatography
- HeLa:: *Henrietta Lacks,* human cervical cancer cell line
- (HP)LC:: (high performance) liquid chromatography
- HRMS:: high-resolution mass spectrometry
- DIPEA: diisopropylethylamine
- *J*:: coupling constant
- LC-MS:: liquid chromatography mass spectrometry
- LRMS:: low-resolution mass spectrometry
- MEF:: mouse embryonic fibroblast cell line
- MF-OH:: 3'-hydroxy-6'-methoxy-3*H*-spiro[isobenzofuran-1,9'-xanthen]-3-one
- NMR:: nuclear magnetic resonance
- Pd/C:: palladium on charcoal, 10%wt
- (δ) ppm:: chemical shift (parts per million)
- PMB:: *p*-methoxy benzyl
- PQ-OH:: 6-chloro-2-(5-chloro-2-hydroxyphenyl)quinazolin-4(3*H*)-one
- *p*-TSA:: *p*-toluenesulfonic acid
- R*_{f}*:: retention factor
- RP:: reversed phase
- r.t.:: room temperature
- sat.: saturated (solution)
- THF:: tetrahydrofuran
- TLC:: thin layer chromatography

### 2. General Synthesis Conditions

Unless stated otherwise, (1) all reactions and characterisations were performed with solvents and reagents, used as obtained, under closed air atmosphere without special precautions; (2) DCM, Et₂O, THF and DMF used for synthesis were distilled, then dried on activated molecular sieves; (3) isohexane and EtOAc used for chromatography was distilled from commercial crude iso-hexane fraction; (4) "column" and "chromatography" refer to FCC, performed on Merck silica gel Si-60 (40-63 µm); (5) procedures and yields are unoptimised; (6) yields refer to isolated chromatographically and spectroscopically pure materials, corrected for residual solvent content.

NMR: Standard NMR characterisation was carried out by ¹H- and ¹³C-NMR spectra. Avance III Bruker BioSpin 400 MHz, 500 MHz and 600 MHz spectrometers were used (¹H: 400 MHz, 500 MHz or 600 MHz, ¹³C: 101 MHz, 126 MHz and 151 MHz respectively) typically at 298 K, although with some carbamates spectroscopy was simplified by measuring at 373 K to induce a dynamic equilibrium between their rotameric species. Chemical shifts (δ) are reported in ppm calibrated to residual non-perdeuterated solvent as an internal reference. Peak descriptions singlet (s), doublet (d), triplet (t), quartet (q), pentuplet (p), and multiplet (m) are used. Coupling constants *J* are given in Hz.

Mass spectra: Unit mass measurements were performed on an AGILENT 1200 SL coupled LC-MS system with ESI mode ionisation, with binary eluent mixtures of H₂O:MeCN, with the water containing formic acid. HRMS was carried out by the Zentrale Analytik of the Ludwig-Maximilian-Univeristy (LMU), Munich (electron impact (EI) at 70 eV with a Thermo Finnigan MAT 95 or a Jeol GCmate II spectrometer; electrospray ionization (ESI) with a Thermo Finnigan LTQ FT Ultra Fourier Transform Ion Cyclotron resonance mass spectrometer) in positive or negative mode as stated.

### 3. General Synthesis Protocols

### General procedure A: selenocyanation of alkyl mesylates

The mesylate was dissolved in THF (0.1 M) and Nal (3.0 equiv.) was added. After 5 min, 18-crown-6 (1.1 equiv.) and KSeCN (2.2 equiv. per mesylate) were sequentially added and the colourless reaction mixture was heated to 50 °C for 16 h. The reaction mixture was diluted with brine and DCM, the organic layer was removed, and the aqueous layers were extracted with DCM (three times). The combined organic phases were dried over MgSO₄, filtered and concentrated *in vacuo.* Column chromatography allowed purification of the orange crude to afford alkyl selenocyanates as pale yellow solids.

### General procedure B: base-mediated selenenylsulfide or diselenide formation

A solution of (thioacetate) (selenocyanate) or bis(selenocyanate) starting material (1.0 equiv.) in THF (0.05 M), was charged with a methanolic solution of KOH (0.2 M, 1.4 equiv.) in a dropwise manner. When TLC control indicated full conversion of the starting material (typically within minutes) the reaction was quenched with NaHCO₃ (sat. aq.), and the aqueous layer was extracted with DCM (three times). The combined organic layers were dried over MgSO₄, filtered and concentrated *in vacuo.* The targeted selenenylsulfide or diselenide (respectively) was received as a deeply coloured solid and used without further purification.

### General procedure C: methylation of N-Boc protected amines

In a flame-dried Schlenk flask, *N*-Boc protected primary amine (1.0 equiv.) was dissolved in dry DMF (20 mM) under nitrogen atmosphere. The solution was cooled to 0°C and first Mel (1.1 equiv.) then NaH (60%, 1.1 equiv.) were added. The reaction mixture was allowed to slowly warm to r.t. and was stirred for 5 to 7 h, upon which TLC control confirmed full conversion of the starting material. The reaction was quenched with NaHCO₃ (aq. sat.) and diluted with DCM. The aqueous layer was extracted with DCM (three times) and the combined organic layers were dried over MgSO₄ and concentrated under reduced pressure. The crude product was purified by column chromatography to yield the target species as colourless or yellow oil.

### General procedure D: Monoalkylation of primary amines by 3-bromo-propionyl derivatives.

*Step 1:* To a solution of the primary amine (1.5 equiv.) in MeOH (0.3 M) was added the respective *N*-substituted 3-bromo-propenamide (1.0 eq). In case the starting amine was used as an HCl salt, NEt₃ (1.5 equiv.) was added for neutralisation. The clear solution was heated in a sealed tube using a laboratory microwave (120 °C, 25 W, 60 min), was then cooled to r.t. and concentrated under reduced pressure.

*Step 2:* The material obtained in step 1 was suspended in DCM (0.1 M) and charged with triethylamine (3.0 equiv.) and Boc₂O (1.8 equiv., 1 M in anhydrous DCM) at r.t., and the mixture was stirred at retained temperature for 15 h. The reaction was then concentrated under reduced pressure. Purification by flash column chromatography yielded the N-Boc protected alkylation product of the primary amine.

### General procedure E: Boc-deprotection of primary amines

Boc-protected amine (1.0 equiv.) was dissolved in DCM (0.1 M), and HCl (4 M in dioxane, 10 equiv.) was added at r.t. in a dropwise manner. In most cases and within minutes, formation of a precipitate was observed. Full conversion of the starting material was checked by TLC and observed within 4-12 hours. Excess HCl and DCM were removed under reduced pressure, yielding targeted amines as HCl salts. *Caution*: to remove HCl, use a leak-proof rotary evaporator in a well-ventilated hood.

### General procedure F: Carbamate formation with primary amine hydrochlorides and PQ-chloroformate

A trigger-amine hydrocholoride was suspended in DCM (50 mM) and solubilised by adding NEt₃ (1.1-2.2 equiv.), regenerating the free amine. Subsequently, to a suspension of preformed PQ-OC(O)CI **X22** in DCM (0.04 M) was slowly added the trigger amine and the resulting reaction mixture was stirred for 1 h. Once LCMS-control indicated full conversion of the starting material, the reaction mixture was concentrated *in vacuo* yielding a pale yellow crude oil. In most cases, flash chromatography provided the target carbamates in excellent purity and ensured no residual fluorescence of unreacted fluorophore, though additional purification by prepHPLC could be conducted as needed.

### 4. Synthesis

### Precursors to proagents

### N-Boc-dimethylaspartate (X1)

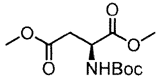

A methanolic suspension (0.3 M, 200 mL) of aspartic acid (10.0 g, 75.1 mmol, 1.0 equiv.) was reacted with SOCl₂ (21.8 mL, 301 mmol, 4.0 equiv.) and after evaporation of the solvent and gaseous byproducts, dimethylaspartate hydrochloride was received as a colourless solid. Without further purification, the obtained dimethyl ester was dissolved in a 1:1-mixture of water (75 mL) and dioxane (75 mL), cooled to 0 °C and was charged with NEt₃ (26.0 mL, 188 mmol, 2.5 equiv.) and Boc₂O (19.7 g, 90.1 mmol, 1.2 equiv.). The reaction mixture was stirred for 14 h, acidified and extracted to yield a colourless crude product. Purification was achieved running a short column (SiO₂, h = 3 cm, d = 6 cm, hexanes/EtOAc 3:1, 50 mL tubes, Fr8-20) giving target species **39** as a colourless solid (18.7 g, 71.6 mmol, 95%).

**TLC** R*_{f}* = 0.39 (hexanes/EtOAc 3:1). **¹H NMR** (400 MHz, CDCl₃) *δ* (ppm) = 5.48 (d, *J* = 8.7 Hz, 1H), 4.57 (dt, *J* = 9.2, 4.7 Hz, 1H), 3.75 (s, 3H), 3.69 (s, 3H), 2.99 (dd, *J* = 16.9, 4.7 Hz, 1H), 2.81 (dd, *J* = 16.9, 4.7 Hz, 1H), 1.44 (s, 9H). **¹³C NMR** (101 MHz, CDCl₃) *δ* (ppm) = 171.7, 171.5, 155.5, 80.3, 52.8, 52.1, 50.0, 36.8, 28.4. **HRMS** (EI+): m/z calc. for C₁₁H₁₉NO₆ [M]^{·+} 261.1207, found 261.1206.

### N-Boc-2-aminobutane-1,4-diol(X2)

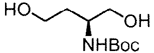

Diester **X1** (7.00 g, 26.8 mmol, 1.0 equiv.) in anhydrous THF (0.2 M, 135 mL) was carefully added to a suspension of LAH (3.05 g, 80.4 mmol, 3.0 equiv.) in dry THF (0.2 M, 135 mL) at 0 °C. After 3 h, Fieser-type work up was conducted giving a colourless crude oil. Flash chromatography (hexanes/EtOAc 1:1 → EtOAc) ultimately provided the target diol as a colourless solid (4.00 g, 19.5 mmol, 73%). NMR spectra of **X2** match reported data. (Lukesh; J. Am. Chem. Soc. 2012 134, 4057-4059)

TLC R*_{f}* = 0.30 (EtOAc). **¹H NMR** (400 MHz, CDCl₃) *δ* (ppm) = 4.99 (s, 1H), 3.91 - 3.82 (m, 1H), 3.78 - 3.62 (m, 4H), 2.24 (s, 2H), 1.81 (ddt, *J* = 14.1, 9.3, 4.6 Hz, 1H), 1.63 (tt, *J* = 10.1, 4.2 Hz, 1H), 1.45 (s, 9H). **¹³C NMR** (101 MHz, CDCl₃) *δ* (ppm) = 157.2, 80.2, 65.6, 58.9, 49.5, 35.0, 28.5. **HRMS** (EI+): m/z calc. for C₈H₁₆NO₃ [M-CH₃O)^{·+} 174.1125, found 174.1122.

### N-Boc-2-aminobutane-1,4-diyl dimethanesulfonate (X3)

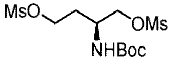

A solution of diol **X2** (0.18 g, 0.87 mmol, 1.0 equiv.) in anhydrous DCM (0.05 M, 20 mL) was charged with NEt₃ (0.61 mL, 4.3 mmol, 5.0 equiv.) and MsCl (0.15 mL, 2.0 mmol, 2.3 equiv.) at 0 °C. Acidic workup after 3 h and purification by column chromatography (hexanes/EtOAc 1:1 → hexanes/EtOAc 1:2) gave **X3** as a colourless solid (0.30 g, 0.82 mmol, 94%).

**TLC** R*_{f}* = 0.37 (hexanes/EtOAc 1:2) **¹H NMR** (400 MHz, CDCl₃) *δ* (ppm) = 4.78 (d, *J =* 8.7 Hz, 1H), 4.39 - 4.23 (m, 4H), 4.10 - 4.02 (m, 1H), 3.05 (s, 3H), 3.04 (s, 3H), 2.11 - 2.04 (m, 1H), 2.02 - 1.93 (m, 1H), 1.45 (s, 9H). **¹³C NMR** (101 MHz, CDCl₃) *δ* (ppm) = 155.3, 80.5, 71.0, 66.3, 46.9, 37.5 (2C), 31.0, 28.4 (3C). **HRMS** (El+): m/z calc. for C₁₁H₂₃NO₈S₂ [M]^{·+} 361.0860, found 361.0861.

### N-Boc-(3-amino-4-(methanesulfonyl)oxybutyl)ethanethioate (X4) &

### N-Boc-(2-amino-4-(methanesulfonyl)oxybutyl)ethanethioate (X5)

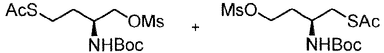

18-crown-6 (0.12 g, 0.33 mmol, 1.2 equiv.) and KSAc (32 mg, 0.277 mmol, 1.0 equiv.) were sequentially added to a solution of **X3** (0.10 g, 0.28 mmol, 1.0 equiv.) in dry DMF (0.05 M, 27 mL). After 15 h, the solvent was removed and the obtained crude was purified by column chromatography (hexanes/EtOAc 1:1) yielding **X4** as a colourless solid (55 mg, 0.16 mmol, 57%) along with its regioisomeric form **X5** (21 mg, 87 µmol, 31%).

***X4*: TLC** R*_{f}* = 0.40 (hexanes/EtOAc 1:1) **¹H NMR** (400 MHz, CDCl₃) *δ* (ppm) = 4.75 (s, 1H), 4.31 - 4.20 (m, 2H), 3.91 (br s, 1H), 3.04 (s, 3H), 3.03 - 2.95 (m, 1H), 2.89 - 2.81 (m, 1H), 2.34 (s, 3H), 1.85 (dt, *J* = 13.7, 6.9 Hz, 2H), 1.45 (s, 9H). **¹³C NMR** (101 MHz, CDCl₃) *δ* (ppm) = 195.8, 155.4, 71.0, 49.1, 37.5, 31.6, 30.8, 28.5, 25.6. **HRMS** (El+): m/z calc. for C₁₂H₂₃NO₆S₂ [M] ^{·+} 341.0961, found 341.0957.

***X5:* TLC** R_{f} = 0.44 (hexanes/EtOAc 1:1) **¹H NMR** (400 MHz, CDCl₃) *δ* (ppm) = 4.59 (d, *J* = 8.9 Hz, 1H), 4.33 - 4.25 (m, 2H), 3.90 (s, 1H), 3.08 (dd, *J* = 6.2, 2.9 Hz, 2H), 3.04 (s, 3H), 2.37 (s, 3H), 2.07 - 1.99 (m, 1H), 1.90 - 1.80 (m, 1H), 1.43 (s, 9H). **¹³C NMR** (101 MHz, CDCl₃) *δ* (ppm) = 195.7, 155.6, 66.9, 47.9, 37.5, 33.8, 30.7, 28.5. **HRMS** (EI+): m/z calc. for C₁₂H₂₃NO₆S₂ [M]^{·+} 341.0961, found 341.0930.

### N-Boc-(2-amino-4-selenocyanatobutyl)ethanethioate (X6)

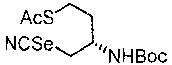

Selenocyanation was conducted based on general procedure A. Mesylate **X4** (0.60 g, 1.8 mmol, 1.0 equiv.) was dissolved in THF (0.1 M, 20 mL), subjected to Nal (0.79 mg, 5.3 mmol, 3.0 equiv.), 18-crown-6 (5.1 g, 1.9 mmol, 1.1 equiv.) and KSeCN (557 mg, 3.87 mmol, 2.2 equiv.), and the resulting mixture was heated to 50 °C for 10 h. Purification via column chromatography (hexanes/EtOAc 4:1 → hexanes/EtOAc 3:1) followed aqueous work up and ultimately provided **X6** as a pale yellow solid (552 mg, 1.57 mmol, 89%). *Note:* **X6** proved to be rather unstable being stored at ambient temperature and on air and turned purple within a few weeks.

**TLC** R*_{f}* = 0.20 (hexanes/EtOAc 3:1) **¹H NMR** (400 MHz, CDCl₃) *δ* (ppm) = 4.82 (s, 1H), 3.88 (h, *J* = 7.8, 7.3, 6.3, 6.3, 5.1 Hz, 1H), 3.44 - 3.31 (m, 1H), 3.31 - 3.22 (m, 1H), 3.04 - 2.97 (m, 1H), 2.85 (dt, *J* = 13.9, 7.7 Hz, 1H), 2.35 (s, 3H), 1.88 (q, *J =* 7.3 Hz, 2H), 1.46 (s, 9H). **¹³C NMR** (101 MHz, CDCl₃) *δ* (ppm) = 196.0, 155.5, 80.6, 50.3, 41.0, 35.1, 34.2, 30.8, 28.5, 25.7. **LRMS** (ESI+): m/z calc. for C₁₁H₁₈NO₃SSe [M-CH₂N]^{·+} 325.02, found 324.99.

### N-Boc-(2-amino-5-selenocyanatobutyl)ethanethioate (X7)

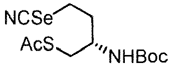

Following selenocyanation protocol A, mesylate **X5** (0.44 g, 1.3 mmol, 1.0 equiv.) was dissolved in THF (0.1 M, 13 mL) and subjected to Nal (0.58 g, 3.9 mmol, 3.0 equiv.), 18-crown-6 (0.38 g, 1.4 mmol, 1.1 equiv.) and KSeCN (0.41 g, 2.8 mmol, 2.2 equiv.) in a consecutive manner. After having stirred the resulting, colourless suspension at 50 °C for 10 h, aqueous work up gave an orange crude product which was purified by column (hexanes/EtOAc 3:1). **X7** was received as a pale yellow solid (0.43 g, 1.2 mmol, 92%).

**TLC** R_{f} = 0.55 (hexanes/EtOAc 1:1) **¹H NMR** (400 MHz, CDCl₃) *δ* (ppm) = 4.60 (d, *J* = 9.0 Hz, 1H), 3.91 (s, 1H), 3.19 (ddd, *J* = 12.6, 7.8, 5.1 Hz, 1H), 3.07 (d, *J* = 6.2 Hz, 2H), 2.99 (dt, *J =* 12.3, 7.9 Hz, 1H), 2.38 (s, 3H), 2.18 - 2.07 (m, 1H), 2.06 - 1.97 (m, 1H), 1.43 (s, 9H). **¹³C NMR** (101 MHz, CDCl₃) *δ* (ppm) = 195.8, 156.0, 80.3, 50.5, 41.0, 36.4, 33.6, 30.7, 28.5, 26.6. **HRMS** (El+): m/z calc. for C₁₂H₂₀N₂O₃SSe [M]^{·+} 352.0354, found 352.0352.

### N-Boc-1,4-diselenocyanatobutan-2-amine (X8)

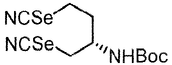

Nal (83 mg, 0.55 mmol, 1.0 equiv.), 18-crown-6 (0.32 g, 1.2 mmol, 2.2 equiv.) and KSeCN (0.20 g, 1.4 mmol, 2.5 equiv.) were added to a THF-solution (0.1 M, 6 mL) of dimesylate **X3** (0.20 g, 0.55 mmol, 1.0 equiv.). After heating for 20 h, aqueous work up and flash chromatography (hexanes/EtOAc 3:1 → hexanes/EtOAc 1:1) gave the title compound as a colourless solid (0.18 g, 0.46 mmol, 83%).

**TLC** R*_{f}* = 0.50 (hexanes/EtOAc 1:1) **¹H NMR** (400 MHz, CDCl₃) *δ* (ppm) = 4.74 (s, 1H), 4.06 (h, *J* = 7.5, 6.6 Hz, 1H), 3.43 - 3.13 (m, 3H), 3.01 (dt, *J* = 12.4, 7.8 Hz, 1H), 2.31 - 2.11 (m, 2H), 1.45 (s, 9H). **¹³C NMR** (101 MHz, CDCl₃) *δ* (ppm) = 155.5, 101.5, 101.4, 81.0, 50.7, 35.8, 34.5, 28.4, 25.8. **HRMS** (El+): m/z calc. for C₁₀H₁₆N₂O₂Se₂ [M-CH₂N]^{·+} 355.9537, found 355.9537.

### N-Boc-1,2-thiaselenan-4-amine (X9)

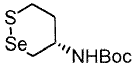

Thiaselenane-formation was achieved according to general procedure B. **X6** (0.70 g, 2.0 mmol, 1.0 equiv.) was dissolved in THF (0.05 M, 40 mL) and KOH was added (0.17 M in MeOH, 16 mL, 2.7 mmol, 1.4 equiv.). Within seconds, the colourless reaction mixture turned yellow and TLC control indicated full conversion of the starting material. Aqueous work up gave pure **X9** as a pale yellow solid (0.55 mg, 2.0 mmol, 100%).

**TLC** R*_{f}* = 0.61 (hexanes/EtOAc 3:1) **¹H NMR** (400 MHz, CDCl₃) *δ* (ppm) = 4.93 (s, 1H), 3.94 (s, 1H), 3.23 (ddd, *J* = 14.3, 7.8, 2.8 Hz, 2H), 3.00 (d, *J* = 13.2 Hz, 1H), 2.87 - 2.78 (m, 1H), 2.16 (d, *J* = 13.1 Hz, 1H), 1.77 (s, 1H), 1.45 (s, 9H). **¹³C NMR** (101 MHz, CDCl₃) *δ* (ppm) = 154.7, 80.0, 47.4, 35.1, 32.2, 30.1, 28.5. **HRMS** (EI+): m/z calc. for C₉H₁₇NO₂SSe [M]^{·+} 283.0140, found 283.0136.

### N-Boc-1,2-thiaselenan-5-amine (X10)

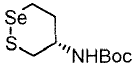

According to general procedure B, **X7** (0.40 g, 1.1 mmol, 1.0 equiv.) was dissolved in THF (0.05 M, 20 mL) and potassium hydroxide (0.17 M in MeOH, 10 mL, 1.7 mmol, 1.5 equiv.) was slowly added. The reaction mixture immediately changed from colourless to yellow and, within 5 min, full conversion of the starting material was indicated by TLC. Aqueous workup and concentration of the combined organic extracts gave the desired product as a pale yellow solid (0.32 g, 1.1 mmol, 100%).

**TLC** R*_{f}* = 0.64 (hexanes/EtOAc 3:1) **¹H NMR** (400 MHz, CDCl₃) *δ* (ppm) = 5.02 (s, 1H), 3.96 - 3.83 (m, 1H), 3.30 - 3.13 (m, 2H), 2.97 (s, 1H), 2.80 (dd, *J* = 13.5, 7.7 Hz, 1H), 2.25 (t, *J =* 11.8 Hz, 1H), 2.04 (s, 1H), 1.45 (s, 9H). **¹³C NMR** (101 MHz, CDCl₃) *δ* (ppm) = 154.8, 79.9, 47.2, 37.7, 28.5, 23.1. **HRMS** (El+): m/z calc. for C₉H₁₇NO₂SSe [M]^{·+} 283.0140, found 283.0133.

### N-Boc-1,2-diselenan-4-amine (X11)

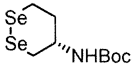

Diselenide formation was achieved in analogy to general protocol B: di-selenocyanate **X8** (0.50 g, 1.3 mmol, 1.0 equiv.) was provided in THF-solution (0.03 M, 30 mL) and KOH (0.17 M in MeOH, 9.4 mL, 1.6 mmol, 1.2 equiv.) was added. Within seconds, a deeply yellow species formed in the reaction vessel and full conversion was achieved according to TLC control. Subsequently conducted aqueous work up provided the title compound as a yellow solid (0.35 mg, 1.1 mmol, 85%).

**TLC** R*_{f}* = 0.58 (hexanes/EtOAc 3:1) **¹H NMR** (400 MHz, CDCl₃) *δ* (ppm) = 5.03 (s, 1H), 4.00 - 3.81 (m, 1H), 3.30 (tdd, *J* = 15.4, 9.1, 2.0 Hz, 2H), 3.10 - 3.00 (m, 1H), 2.89 (dd, *J* = 12.3, 8.2 Hz, 1H), 2.22 (ddt, *J* = 14.2, 8.8, 2.9 Hz, 1H), 2.03 - 1.91 (m, 1H), 1.43 (s, 9H). **¹³C NMR** (101 MHz, CDCl₃) *δ* (ppm) = 154.6, 79.8, 47.5, 35.0, 28.5, 28.5, 22.2. **HRMS** (El+): m/z calc. for C₉H₁₇NO₂Se₂ [M]^{·+} 330.9584, found 330.9588.

### N-Boc-N-methyl-1,2-thiaselenan-4-amine (X12)

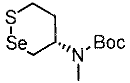

Following the general protocol C, Mel (79µL, 1.3 mmol, 1.2 equiv.) then NaH (47 mg, 1.2 mmol, 1.1 equiv.) were added to a solution of **X9** (0.30 g, 1.1 mmol, 1.0 equiv.) in dry DMF (0.02 M, 60 mL). After 5 h, basic, aqueous workup was followed by flash chromatography (hexanes/EtOAc 93:7) yielding **X12** as a pale yellow oil (88 mg, 0.30 mmol, 28%). *Note:* the title compound was observed as a mixture of rotameric species. Reported NMR data refer to the major rotamer.

**TLC** R*_{f}* = 0.30 (hexanes/EtOAc 93:7) **¹H NMR** (400 MHz, CDCl₃) *δ* (ppm) = 4.42 (s, 1H), 3.37 - 3.15 (m, 3H), 2.75 (s, 3H), 2.69 (ddd, *J* = 11.7, 3.1, 1.3 Hz, 1H), 2.09 (d, *J* = 13.2 Hz, 1H), 1.95 (m, 1H), 1.46 (s, 9H). **¹³C NMR** (101 MHz, CDCl₃) *δ* (ppm) = 155.2, 80.1, 54.4, 36.3, 34.0, 29.0, 28.6 (3C), 27.0. **HRMS** (ESI-): m/z calc. for C₁₀H₁₈NO₂SSe [M-H]⁻ 296.0229, found 296.0230.

### N-Boc-N-Methyl-1,2-thiaselenan-5-amine (X13)

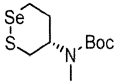

Methylation of 1,2-thiaselenan **X10** was achieved following general procedure C: The starting material (0.20 g, 0.71 mmol, 1.0 equiv.) was dissolved in dry DMF (0.03 M, 25 mL) and charged with Mel (53 µL, 0.85 mmol, 1.2 equiv.) and NaH (60%, 31 mg, 0.78 mmol, 1.1 equiv.) at 0 °C. After 5 h, aqueous work up was conducted and the obtained crude was purified by column chromatography (hexanes/EtOAc 93:7) yielding **X13** as a yellow oil (0.14 g, 0.48 mmol, 68%). *Note:* the title compound was observed as a mixture of rotameric species. Reported NMR data refer to the major rotamer.

**TLC** R*_{f}* = 0.30 (hexanes/EtOAc 9:1) **¹H NMR** (400 MHz, CDCl₃) *δ* (ppm) = 4.17 (s, 1H), 3.42 (t, *J* = 12.3 Hz, 1H), 3.14 (t, *J* = 12.5, 10.6 Hz, 1H), 3.04 (td, *J* = 12.8, 3.2 Hz, 1H), 2.79 (d, *J =* 3.0 Hz, 1H), 2.76 (s, 3H), 2.15 (d, *J* = 26.2 Hz, 2H), 1.46 (s, 9H). **¹³C NMR** (101 MHz, CDCl₃) *δ* (ppm) = 155.4, 80.2, 57.4 (minor), 55.5 (major), 35.5, 33.8, 29.4, 28.6, 28.2. **HRMS** (El+): m/z calc. for C₁₀H₁₉NO₂SSe [M]^{·+} 297.0296, found 297.0303.

### N-Boc-N-methyl-1,2-diselenane-4-amine (X14)

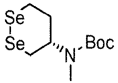

**X11** was methylated according to general procedure C: Starting material (200 mg, 0.608 mmol, 1.0 equiv.) in DMF (0.02 M, 40 mL) was sequentially charged with Mel (45 µL, 0.729 mmol, 1.2 equiv.) and NaH (27 mg, 0.668 mmol, 1.1 equiv.) at 0°C. Following basic, aqueous workup, flash chromatography (hexanes/EtOAc 20:1) enabled isolation of pure **X14** as a yellow solid (80 mg, 0.232 mmol, 38%). *Note:* the title compound was observed as a mixture of rotameric species. Reported NMR data refer to the major rotamer.

**TLC** R*_{f}* = 0.29 (hexanes/EtOAc 9:1) **¹H NMR** (400 MHz, CDCl₃) *δ* (ppm) = 4.37 (s, 1H), 3.52 - 3.42 (m, 1H), 3.30 (d, *J* = 13.0 Hz, 2H), 2.84 - 2.68 (m, 4H), 2.21 (d, *J* = 13.3 Hz, 1H), 2.16 - 2.05 (m, 1H), 1.46 (s, 9H). **¹³C NMR** (101 MHz, CDCl₃) *δ* (ppm) = 155.2, 80.1, 55.7, 34.6, 29.1, 28.6, 27.2, 25.6. **HRMS** (ESI-): m/z calc. for C₁₀H₁₈NO₂Se₂ [M-H]⁻ 343.9674, found 343.9677.

### 3-Bromo-1-morpholinopropan-1-one (X15)

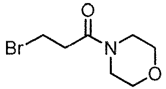

To a solution of morpholine (0.81 mL, 9.4 mmol, 1.0 eq, 0.1 M in DCM) and triethylamine (2.0 mL, 14 mmol, 1.5 eq) was carefully added a solution of 3-bromo-propionyl chloride (1.0 mL, 9.4 mmol, 1.0 eq, 1.0 M in DCM) at 0°C. The mixture was stirred for 15 min, was allowed to warm to r.t. and was further stirred for 15 min. The mixture was quenched by addition of sat. aq. NaCl, the aqueous layer was extracted with EtOAc (three times) and the combined organic layers were washed with aq. NaCl were dried with Na₂SO₄, filtered and concentrated under reduced pressure to yield **X15** as a colourless oil (1.51 g, 6.8 mmol, 72%). **TLC** R_{f} = 0.48 (EtOAc). **¹H-NMR** (400 MHz, DMSO-*d*₆): *δ* (ppm) = 3.64 (t, *J* = 6.7 Hz, 2H), 3.54 (dd, *J* = 10.1, 5.2 Hz, 4H), 3.43 (q, *J* = 5.0 Hz, 4H), 2.95 (t, *J* = 6.7 Hz, 2H). **¹³C-NMR** (101 MHz, DMSO-*d*₆): *δ* (ppm) = 168.2, 66.1, 66.1, 45.2, 41.6, 35.2, 28.9. **HRMS** (ESI+): m/z calc. for C₇H₁₃N₂OBr: [M+H]⁺ 222.01242, found 222.01256.

### 3-Bromo-1-(4-methylpiperazin-1-yl)propan-1-one hydrochloride (X16)

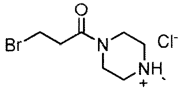

3-Bromo-propionyl chloride (3.0 mL, 28 mmol, 1.0 equiv.) was dissolved in dry DCM (30 mL, 1 M), and cooled to 0°C. N-Methyl-piperazine (3.1 mL, 28 mmol, 1.0 equiv.) in DCM (90 mL, 0.3 M), was added and the reaction mixture was stirred at retained temperature for 1 h. The colourless precipitates formed during the reaction were filtered, washed with ether and dried on air to give the title compound as a colourless solid (7.2 g, 26 mmol, 93%)
**TLC** R_{f} = 0.40 (DCM/MeOH 9:1). **¹H-NMR** (400 MHz, D₂O): *δ* (ppm) = 4.63 (br s, 1H), 4.23 (br s, 1H), 3.67 (t, J = 6.2 Hz, 2H), 3.59 (br s, 3H), 3.13 (br s, 5H), 2.95 (q, J = 1.8 Hz, 3H). **HRMS** (ESI+): m/z calc. for C₈H₁₆N₂OBr: [M+H]⁺ 235.04405, found 235.04418.

### (S)-3-((1,2-Thiaselenan-4-yl)amino)-1-morpholinopropan-1-one hydrochloride (X17)

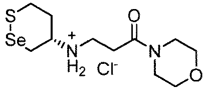

(*S*)-1,2-Thiaselenan-4-amine hydrochloride (0.10 g, 0.52 mmol, 1.2 equiv.), DIPEA (0.25 mL, 0.52 mmol, 1.2 equiv.) and **X15** (86 mg, 0.40 mmol, 1.0 equiv.) were reacted in MeOH (1.2 mL, 0.3 M) according to general procedure D. Boc-protection using Boc₂O (0.13 g, 0.60 mmol, 1.5 equiv.) and DIPEA (0.58 mL, 1.2 mmol, 3 equiv.) in DCM (2 mL, 0.2 M) gave **X17a** (49 mg, 0.12 mmol, 30%) after purification by flash chromatography (DCM→DCM/MeOH 9:1). **TLC** R_{f} = 0.56 (EtOAc). **HRMS** (ESI+): m/z calc. for C₁₆H₂₈NaN₂O₄SSe: [M+Na]⁺ 447.08272, found 447.08310.

Following general procedure E, hydrochloric acid (0.30 mL, 4 M in dioxane, 1.2 mmol, 10 equiv.) enabled Boc-deprotection of X6a in DCM (1.2 mL, 0.1 M). HCl-salt **X17** (42 mg, 0.12 mmol, 30% over 3 steps) was obtained as a solid after filtration from the reaction mixture. **¹H-NMR** (400 MHz, MeOD-*d₄*): *δ* (ppm) = 3.68 (dt, *J* = 10.6, 4.7 Hz, 6H), 3.62 - 3.57 (m, 2H), 3.54 - 3.46 (m, 3H), 3.44 - 3.34 (m, 3H), 3.26 - 3.14 (m, 2H), 2.85 (t, *J* = 5.9 Hz, 2H), 2.48 (d, *J* = 11.3 Hz, 1H), 1.94 (d, *J* = 10.9 Hz, 1H). **HRMS** (ESI+): m/z calc. for C₁₁H₂₁N₂O₄SSe: [M+H]⁺ 325.04835, found 325.04865.

### 3-((1,2-Thiaselenan-5-yl)amino)-1-morpholinopropan-1-one hydrochloride (X18)

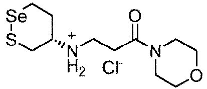

(*S*)-1,2-Thiaselenan-5-amine hydrochloride (0.11 g, 0.52 mmol, 1.3 equiv.), DIPEA (0.25 mL, 0.52 mmol, 1.3 equiv.) and **X15** (89 mg, 0.40 mmol, 1.0 equiv.) were dissolved in MeOH (1.2 mL, 0.3 M) and reacted under microwave irradiation (120°C, 60 min) according to general procedure D. Subsequent protection using Boc₂O (0.13 g, 0.60 mmol, 1.5 equiv.) and DIPEA (0.58 mL, 1.2 mmol, 3 equiv.) in DCM (2 mL, 0.2 M) followed by purification by flash chromatography (DCM→DCM/MeOH 9:1) gave **X18a** (49 mg, 0.12 mmol, 29%).

**TLC** R_{f} = 0.59 (EtOAc). **HRMS** (ESI+): m/z calc. for C₁₆H₂₈NaN₂O₄SSe: [M+Na]⁺ 447.08272, found 447.08318.

Boc-deprotection using HCl (0.30 mL, 4 M in dioxane, 1.2 mmol, 10 equiv.) in DCM (1.2 mL, 0.1 M) according to general procedure E gave **X18** (41 mg, 0.12 mmol, 29% over 3 steps) as a colourless solid.

**¹H-NMR** (400 MHz, MeOD-*d*₄): *δ* (ppm) = 3.71 - 3.64 (m, 6H), 3.62 - 3.58 (m, 2H), 3.55 (d, *J* = 9.7 Hz, 1H), 3.54 - 3.49 (m, 2H), 3.42 - 3.33 (m, 3H), 3.31 - 3.25 (m, 1H), 3.14 (dd, *J* = 13.8, 9.4 Hz, 1H), 2.86 (t, *J* = 6.0 Hz, 2H), 2.61 (d, *J* = 14.3 Hz, 1H), 2.25 (s, 1H). **¹³C-NMR** (101 MHz, MeOD-*d*₄): *δ* (ppm) = 170.4, 68.1, 67.6, 67.5, 46.9, 43.3, 42.2, 41.8, 35.1, 34.6, 29.8. **HRMS** (ESI+): m/z calc. for C₁₁H₂₁N₂O₄SSe: [M+H]⁺ 325.04835, found 325.04859.

### (S)-3-((1,2-Thiaselenan-4-yl)amino)-1-(4-methylpiperazin-1-yl)propan-1-one dihydrochloride (X19)

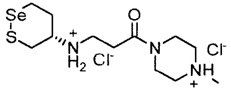

Prepared from (*S*)-1,2-thiaselenan-4-amine hydrochloride (0.31 g, 1.4 mmol, 1.4 equiv.), DIPEA (0.24 mL, 1.3 mmol, 1.3 equiv.) and **X16** (0.28 g, 1.0 mmol, 1.0 equiv.) in MeOH (3 mL, 0.3 M) according to general procedure D. Boc-protection was implemented using Boc₂O (0.34 g, 1.5 mmol, 1.5 equiv.) and DIPEA (0.55 mL, 3.1 mmol, 3 equiv.) in DCM (10 mL, 0.2 M). Purification by flash chromatography (DCM→DCM/MeOH 9:1) gave **X19a** as a colourless solid (0.15 g, 0.35 mmol, 35%).

**TLC** R_{f} = 0.54 (DCM/MeOH 9:1). **HRMS** (ESI+): m/z calc. for C₁₇H₃₂N₃O₃SSe: [M+H]⁺ 438.13241, found 438.13276.

Using hydrochloric acid (0.88 mL, 4 M in dioxane, 3.5 mmol, 10 equiv.) in DCM (3.5 mL, 0.1 M), Boc-deprotection of **X19a** according to general procedure E, gave **X19** (0.13 g, 0.32 mmol, 91%) as a pale yellow solid after filtration of the reaction mixture.

**¹H-NMR** (400 MHz, MeOD-*d*₄): *δ* (ppm) = 4.69 (d, *J* = 10.9 Hz, 1H), 4.15 (d, *J* = 15.8 Hz, 1H), 3.71 - 3.62 (m, 2H), 3.55 (dd, *J* = 18.0, 7.9 Hz, 3H), 3.39 (dd, *J* = 11.5, 5.8 Hz, 2H), 3.28 - 3.16 (m, 2H), 3.10 (d, *J* = 9.1 Hz, 2H), 3.06 - 2.98 (m, 1H), 2.95 (s, 3H), 2.91 - 2.80 (m, 1H), 2.51 (d, *J* = 8.9 Hz, 1H), 2.03 - 1.84 (m, 1H). **¹³C-NMR** (101 MHz, MeOD-*d₄*) *δ* (ppm) = 170.4, 54.5, 51.2, 43.6, 43.4, 41.9, 39.4, 33.4, 30.0, 25.4. **HRMS** (ESI+): m/z calc. for C₁₁H₂₁N₂O₄SSe: [M+H]⁺ 338.07998, found 338.08022.

### (S)-3-((1,2-Thiaselenan-5-yl)amino)-1-(4-methylpiperazin-1-yl)propan-1-one dihydrochloride (X20)

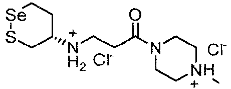

Following general procedure D, (*S*)-1,2-thiaselenan-5-amine hydrochloride (0.44 g, 2.0 mmol, 1.35 equiv.), DIPEA (0.35 mL, 1.9 mmol, 1.3 equiv.) and **X16** (0.40 g, 1.5 mmol, 1.0 equiv.), were dissolved in MeOH (5 mL, 0.3 M) and reacted under microwave irradiation (120 °C, 60 min). Boc-protection of the crude diamine was conducted using Boc₂O (0.48 g, 2.2 mmol, 1.5 equiv.) and DIPEA (0.79 mL, 4.4 mmol, 3 equiv.) in DCM (7.5 mL, 0.2 M). **X20a** was received as a colourless solid (0.17 g, 0.38 mmol, 25%) after purification via flash column chromatography (DCM→DCM/MeOH 9:1).

**TLC** R_{f} = 0.40 (DCM/MeOH 9:1). **HRMS** (ESI+): m/z calc. for C₁₇H₃₂N₃O₃SSe: [M+H]⁺ 438.13241, found 438.13263.

Deprotection of **X20a** was achieved according to general procedure E. Starting material (0.17 g, 0.38 mmol, 1.0 equiv.) in DCM (4 mL, 0.1 M) was charged with HCl (0.95 mL, 4 M in dioxane, 3.8 mmol, 10 equiv.) and the title compound was collected through filtration of the reaction mixture (0.15 g, 0.36 mmol, 95%).

**¹H-NMR** (400 MHz, MeOD-*d*₄): *δ* (ppm) = 4.68 (d, *J* = 11.2 Hz, 1H), 4.15 (d, *J* = 15.4 Hz, 1H), 3.71 - 3.65 (m, 1H), 3.63 - 3.49 (m, 5H), 3.38 (t, *J* = 5.7 Hz, 3H), 3.27 (d, *J* = 11.0 Hz, 2H), 3.18 - 3.04 (m, 4H), 2.95 (s, 3H), 2.61 (d, *J* = 9.1 Hz, 1H), 2.24 (d, *J* = 8.1 Hz, 1H). **¹³C-NMR** (101 MHz, MeOD-*d₄*) *δ*(ppm) = 170.5, 54.1, 51.2, 43.6, 43.4, 41.6, 40.4, 34.3, 32.1, 29.6. **HRMS** (ESI+): m/z calc. for C₁₁H₂₁N₂O₄SSe: [M+H]⁺ 338.04835, found 338.08024.

### 6-Chloro-2-(5-chloro-2-hydroxyphenyl)quinazolin-4(3H)-one (X21)

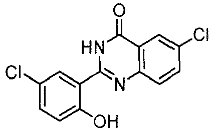

To a solution of 5-chloro-salicylaldehyde (5.00 g, 31.9 mmol, 1.0 equiv.) in anhydrous ethanol (0.05 M, 620 mL), 2-amino-5-chlorobenzamide (5.45 g, 31.9 mmol, 1.0 equiv.) and p-toluene sulfonic acid (607 mg, 3.19 mmol, 0.1 equiv.) were added and the resulting suspension was heated to 90 °C for 1 h. The reaction mixture was cooled to 0 °C and 2,3-dichloro-5,6-dicyano-1,4-benzoquinone (DDQ, 7.25 g, 31.9 mmol, 1.0 equiv.) was added in small batches. The reaction mixture was further stirred at retained temperature for 1 h upon which a yellow precipitate occurred. The solid was filtered, washed with ice-cold ethanol and dried to afford the title compound as a beige solid (8.40 g, 27.3 mmol, 86%). When excited with UV-light, **X21** exhibited bright green fluorescence. NMR spectra match reported data (Thorn-Seshold; *Chem. Commun.* **2012** *48*, 6253-6255).

**¹H NMR** (400 MHz, DMSO) *δ* (ppm) = 13.34 (s, 1H), 12.74 (s, 1H), 8.29 (d, *J* = 2.6 Hz, 1H), 8.09 (d, *J* = 2.4 Hz, 1H), 7.90 (dd, *J* = 8.7, 2.5 Hz, 1H), 7.84 (d, *J* = 8.7 Hz, 1H), 7.49 (dd, *J =* 8.9, 2.6 Hz, 1H), 7.04 (d, *J* = 8.8 Hz, 1H). **HRMS** (ESI-): m/z calc. for C₁₄H₇Cl₂N₂O₂ [M-H]⁻ 304.9890, found 304.9891.

### 4-Chloro-2-(6-chloro-4-oxo-3,4-dihydroquinazolin-2-yl)phenyl chloroformate (X22)

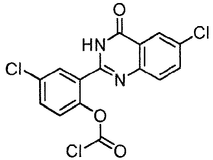

Under a nitrogen atmosphere, 6-chloro-2-(5-chloro-2-hydroxyphenyl) quinazolin-4-one (**X21**, 1.44 g, 4.70 mmol, 1.0 equiv.) was suspended in dry dichloromethane (0.25 M, 20 mL). NEt₃ (1.30 mL, 9.40 mmol, 2.0 equiv.) was added and the mixture was cooled to 0 °C. The mixture was then slowly charged with triphosgene (1.46 g, 4.94 mmol, 1.05 equiv.) in dry DCM (0.3 M, 18 mL) and an immediate formation of HCl could be observed. The reaction mixture was stirred at retained temperature for 30 min, was then allowed to warm to r.t. and was further stirred for 30 min. Exposure of the reaction mixture to 366 nm UV light allowed assessment of the reaction progress: starting material **X21** shows bright green fluorescence under these conditions, while the target species exhibits weak, blue fluorescence. Upon full conversion, which was indicated by LCMS, all volatile compounds were removed using two serially connected, external liquid nitrogen traps while carefully exposing the reaction vessel to reduced pressure. Excess phosgene which was caught in either of the liquid nitrogen traps was destroyed when still cold by carefully adding an excess of piperidine. Chloroformate **X22** was received as a beige solid and was used without further purification. For following carbamate coupling reactions, the crude solid was resuspended in dry DCM (0.04 M, 118 mL).

### Compounds of the invention: Fluorogenic Probes

### Me-SeS60-PQ (P1)

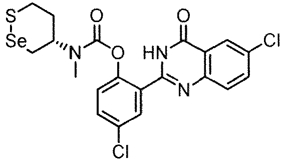

Boc-deprotection of **X12** was achieved following general procedure E. A solution of the starting material (70 mg, 0.236 mmol, 1.0 equiv.) in DCM (0.1 M, 2.5 mL) was charged with HCl (4 M in dioxane, 0.591 mL, 2.36 mmol, 10 equiv.) and the deprotected amine was obtained as its HCl salt and as a pale yellow solid. After re-dissolution in DCM (0.05 M, 5 mL) and NEt₃ (0.10 mL, 0.72 mmol, 3.0 equiv.), the amine was added to chloroformate **X22** (0.25 M in DCM, 2.0 mL, 0.50 mmol, 2.0 equiv.) according to general procedure F. Within 2 h, full conversion was achieved and target compound **P1** was isolated (92 mg, 0.174 mmol, 74%) after aqueous workup and column chromatography (hexanes:EtOAc 3:1). *Note:* the title compound was observed as a mixture of rotameric species. Reported NMR data refer to the major rotamer. **TLC** R*_{f}* = 0.15 (hexanes/EtOAc 3:1) **¹H NMR** (400 MHz, CDCl₃) *δ* (ppm) = 10.23 (s, 1H), 8.25 (d, *J =* 2.2 Hz, 1H), 7.98 (dd, *J* = 10.8, 2.6 Hz, 1H), 7.76 - 7.70 (m, 2H), 7.51 (dt, *J* = 8.8, 2.1 Hz, 1H), 7.18 (dd, *J* = 8.8, 1.6 Hz, 1H), 4.39 (tt, *J* = 11.9, 3.1 Hz, 1H), 3.35 - 3.20 (m, 3H), 3.03 (s, 3H), 2.69 (dd, 1H), 2.07 (dd, *J* = 13.3, 3.7 Hz, 1H), 2.02-1.90 (m, 1H). **¹³C NMR** (101 MHz, CDCl₃) *δ* (ppm) = 160.8, 153.9, 149.4, 147.6, 147.5, 135.5, 133.5, 132.5, 132.3, 130.6, 129.7, 128.1, 126.2, 125.3, 122.4, 56.7, 35.9, 33.5, 30.0, 26.1. **HRMS** (ESI+): m/z calc. for C₂₀H₁₈Cl₂N₃O₃SSe [M+H]⁺ 529.9606, found 529.9612.

### Me-SSe60-PQ (P2)

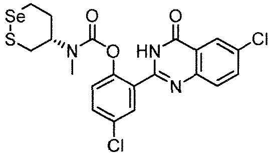

Combining general procedures E and F, thiaselenane **X13** (100 mg, 0.338 mmol, 1.0 equiv.) in DCM (0.1 M, 4 mL) was charged with HCl (4 M in dioxane, 0.84 mL, 3.4 mmol, 10 equiv.). Upon full conversion, the solvent was removed, and the residue was re-dissolved in DCM (0.05 M, 8 mL) and NEt₃ (0.140 mL, 1.01 mmol, 3.0 equiv.). A solution of chloroformate **X22** in DCM (0.25 M, 2.60 mL, 0.660 mmol, 2.0 equiv.) was then charged with the deprotected amine and, after workup and column chromatography (hexanes:EtOAc 3:1), **P2** was received as a pale yellow solid (83 mg, 0.157 mmol, 46%). *Note:* the title compound was observed as a mixture of rotameric species. Reported NMR data refer to the major rotamer.

**TLC** R_{f} = 0.14 (hexanes/EtOAc 3:1) **¹H NMR** (400 MHz, CDCl₃) *δ* (ppm) = 10.15 (s, 1H), 8.30 - 8.22 (m, 1H), 7.98 (t, *J* = 3.3 Hz, 1H), 7.79 - 7.67 (m, 2H), 7.51 (dd, *J* = 8.7, 2.5 Hz, 1H), 7.17 (dd, *J* = 8.8, 3.7 Hz, 1H), 4.23 - 4.15 (m, 1H), 3.47 - 3.35 (m, 1H), 3.19 (q, *J* = 11.5 Hz, 1H), 3.12 - 2.94 (m, 4H), 2.79 (dd, *J* = 13.0, 3.0 Hz, 1H), 2.36 - 2.14 (m, 2H). **¹³C NMR** (101 MHz, CDCl₃) *δ* (ppm) = 160.7, 153.9, 149.3, 147.6, 147.5, 135.5, 133.6, 132.5, 132.4, 130.6, 129.7, 128.0, 126.2, 125.3, 122.4, 57.8, 35.0, 33.1, 30.5, 27.7. **HRMS** (ESI+): m/z calc. for C₂₀H₁₈Cl₂N₃O₃SSe [M+H]⁺ 529.9606, found 529.9603.

### Me-SeSe60-PQ (P3)

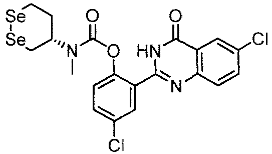

HPQ-coupling of **X14** was conducted according to general protocols E and F. Following deprotection of the starting material (70 mg, 0.204 mmol, 1.0 equiv.) by HCl (4 M in dioxane, 0.510 mL, 2.04 mmol, 10 equiv.), a solution of the amine-HCl salt in DCM (0.05 M, 6 mL) and NEt₃ (85 µL, 0.612 mmol, 3.0 equiv.) was reacted with chloroformate **X22** (0.25 M in DCM, 1.70 mL, 0.440 mmol, 2.0 equiv.) for 4 h. Upon completion, aqueous workup and flash chromatography (hexanes: EtOAc 3:1) gave **P3** as a yellow solid (67 mg, 0.116 mmol, 57%). *Note:* the title compound was observed as a mixture of rotameric species. Reported NMR data refer to the major rotamer.

**TLC** R*_{f}* = 0.18 (hexanes/EtOAc 3:1) **¹H NMR** (400 MHz, CDCl₃) *δ* (ppm) = 10.11 (s, 1H), 8.26 (dd, *J* = 2.1, 0.9 Hz, 1H), 7.99 (dd, *J* = 6.0, 2.6 Hz, 1H), 7.77 - 7.70 (m, 2H), 7.54 - 7.49 (m, 1H), 7.18 (d, *J* = 8.7 Hz, 1H), 4.33 (t, *J* = 11.8 Hz, 1H), 3.46 (t, *J* = 12.9 Hz, 1H), 3.42 - 3.24 (m, 2H), 3.02 (s, 3H), 2.78 (d, *J* = 11.9 Hz, 1H), 2.25 - 2.09 (m, 2H). **¹³C NMR** (101 MHz, CDCl₃) *δ* (ppm) = 160.7, 153.8, 149.3, 147.6, 147.5, 135.5, 133.6, 132.5, 132.4, 130.7, 129.8, 128.1, 126.2, 125.3, 122.4, 58.1, 34.2, 30.1 **HRMS** (ESI+): m/z calc. for C₂₀H₁₈Cl₂N₃O₃Se₂ [M+H]⁺ 577.9050, found 577.9048.

### M-SeS60-PQ (P4)

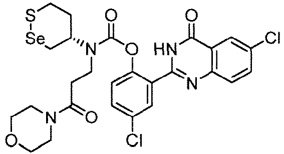

Prepared from **X17** (40 mg, 0.11 mmol, 1.0 equiv.) and **X22** (3.0 mL, 0.04 M in DCM, 0.12 mmol, 1.1 equiv.) according to general protocol F. Purification by flash chromatography (isohexane/EtOAc) gave **P4** as a colourless solid (43 mg, 0.065 mmol, 59%). Light blue solid-state fluorescence was observed under UV-light.

**TLC** R_{f} = 0.48 (EtOAc). **¹H-NMR** (400 MHz, CDCl₃): *δ* (ppm) = 10.46 (d, *J* = 64.1 Hz, 1H), 8.22 (d, *J* = 3.0 Hz, 1H), 7.92 (dd, *J* = 16.2, 2.3 Hz, 1H), 7.78 - 7.68 (m, 2H), 7.56 - 7.47 (m, 1H), 7.17 (dd, *J* = 8.6, 2.7 Hz, 1H), 4.49 - 4.12 (m, 1H), 3.73 (s, 1H), 3.68 (dd, *J* = 8.5, 3.7 Hz, 3H), 3.66 - 3.58 (m, 5H), 3.52 (d, *J* = 12.7 Hz, 3H), 3.50 - 3.46 (m, 1H), 3.43 - 3.35 (m, 1H), 3.33 (d, *J* = 13.5 Hz, 1H), 3.30 - 3.17 (m, 2H), 2.82 - 2.67 (m, 1H), 2.64 - 2.58 (m, 1H), 2.58 - 2.47 (m, 1H), 2.22 - 2.01 (m, 2H). ¹³C-NMR (101 MHz, CDCl₃) *δ* (ppm) = 168.9, 168.5, 160.8, 153.7, 149.6, 147.5, 147.1, 135.5, 133.6, 132.5, 130.7, 129.8, 128.6, 126.0, 125.1, 124.9, 122.3, 67.2, 67.0, 66.7, 66.5, 58.6, 46.0, 45.8, 42.2, 42.0, 40.0, 36.2, 36.0, 34.8, 33.6, 32.6, 27.4. **HRMS** (ESI+): m/z calc. for C₂₆H₂₇Cl₂N₅O₄SSe: [M+H]⁺ 657.02389, found 657.02417.

### M-SSe60-PQ (P5)

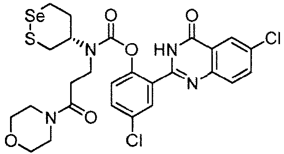

Prepared from **X18** (40 mg, 0.11 mmol, 1.0 equiv.) and **X22** (3.0 mL, 0.04 M in DCM, 0.12 mmol, 1.1 equiv.) according to general protocol F. Purification by flash chromatography (isohexane/EtOAc) gave **P5** as a colourless solid (58 mg, 0.088 mmol, 80%). Light blue solid-state fluorescence was observed under UV-light.

**TLC** R_{f} = 0.46 (EtOAc). **¹H-NMR** (400 MHz, CDCl₃): *δ* (ppm) = 10.48 (d, *J* = 52.1 Hz, 0H), 8.25 - 8.18 (m, 1H), 7.91 (dd, *J* = 24.5, 2.5 Hz, 1H), 7.80 - 7.68 (m, 2H), 7.51 (td, *J* = 7.9, 7.0, 2.5 Hz, 1H), 7.21 - 7.11 (m, 1H), 4.33 - 3.88 (m, 1H), 3.77 - 3.71 (m, 1H), 3.71 - 3.66 (m, 4H), 3.66 - 3.58 (m, 5H), 3.53 (d, *J* = 14.5 Hz, 4H), 3.50 - 3.45 (m, 2H), 3.43 - 3.20 (m, 4H), 3.07 - 2.98 (m, 1H), 2.88 (d, *J* = 10.6 Hz, 1H), 2.78 (d, *J* = 7.0 Hz, 1H), 2.59 (t, *J* = 7.3 Hz, 1H), 2.51 (q, *J* = 7.2 Hz, 1H), 2.40 - 2.27 (m, 2H), 2.21 (s, 1H). **HRMS** (ESI+): m/z calc. for C₂₆H₂₇Cl₂N₅O₄SSe: [M+H]⁺ 657.02389, found 657.02401.

### P-SeS60-PQ (P6)

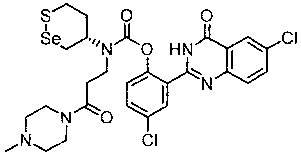

Prepared from **X19** (20 mg, 0.049 mmol, 1.0 equiv.) and **X22** (1.4 mL, 0.04 M in DCM, 0.054 mmol, 1.1 equiv.) according to general protocol F. Purification by flash chromatography (DCM→DCM/MeOH 9:1) gave **P6** as a colourless solid (20 mg, 0.030 mmol, 61%). Further purification by preparative HPLC (MeCN/H₂O, 0.1% FA) yielded **P6** as a colourless solid. Light blue solid-state fluorescence was observed under UV-light.

TLC R_{f} = 0.45 (DCM/MeOH 9:1). **¹H-NMR** (400 MHz, MeOD-*d*₄): δ (ppm) = ¹H NMR (500 MHz, MeOD-*d*₄) δ 8.22 (d, *J* = 2.0 Hz, 1H), 7.91 - 7.85 (m, 1H), 7.83 (d, *J* = 2.4 Hz, 1H), 7.76 (dd, J = 8.6, 6.8 Hz, 1H), 7.65 (dt, *J* = 8.5, 3.3 Hz, 1H), 7.42 - 7.34 (m, 1H), 4.48 (s, 0H), 3.89 (d, *J* = 12.1 Hz, 1H), 3.72 - 3.64 (m, 1H), 3.59 (s, 1H), 3.52 (s, 2H), 3.40 (d, *J =* 3.6 Hz, 1H), 3.36 (d, *J =* 11.1 Hz, 1H), 3.30 - 3.22 (m, 2H), 3.21 - 3.16 (m, 1H), 3.16 - 3.08 (m, 1H), 2.89 (d, *J* = 9.5 Hz, 1H), 2.83 - 2.74 (m, 1H), 2.56 (d, *J =* 11.2 Hz, 1H), 2.48 (d, *J =* 14.1 Hz, 2H), 2.41 (d, *J* = 4.5 Hz, 2H), 2.36 (d, *J* = 7.0 Hz, 3H), 2.32 (s, 1H), 2.20 (d, *J* = 13.2 Hz, 1H), 2.10 - 1.96 (m, 2H), 1.88 (d, *J* = 12.5 Hz, 1H). **¹³C-NMR** (101 MHz, MeOD-*d₄*) δ (ppm) = 169.9, 169.6, 162.0, 152.5, 147.5, 135.1, 132.7, 131.6, 131.0, 129.6, 128.9, 125.1, 124.7, 124.6, 122.1, 58.0, 56.1, 54.4, 54.3, 53.8, 44.4, 43.0, 40.7, 40.6, 35.8, 34.4, 33.2, 33.0, 32.0, 26.9, 25.9, 15.9, 15.7. **HRMS** (ESI+): m/z calc. for C₂₇H₃₀Cl₂N₅O₄SSe: [M+H]⁺ 670.05553, found 670.05587.

### P-SSe60-PQ (P7)

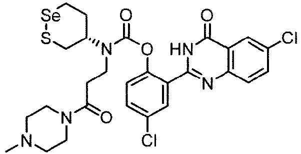

Prepared from **X20** (12 mg, 0.030 mmol, 1.0 equiv.) and **X22** (0.83 mL, 0.04 M in DCM, 0.033 mmol, 1.1 equiv.) according to general protocol F. Purification by flash chromatography (DCM→DCM/MeOH 9:1) gave **P7** as a colourless solid (13 mg, 0.020 mmol, 66%). Further purification by preparative HPLC (MeCN/H₂O, 0.1% FA) yielded **P7** as a colourless solid. Light blue solid-state fluorescence was observed under UV-light.

**TLC** R_{f} = 0.44 (DCM/MeOH 9:1). **¹H-NMR** (400 MHz, MeOD-*d*₄): δ (ppm) = δ 8.22 (d, *J* = 2.4 Hz, 1H), 7.88 (dt, *J* = 8.7, 3.1 Hz, 1H), 7.83 (d, *J* = 2.6 Hz, 1H), 7.79 - 7.74 (m, 1H), 7.65 (ddd, *J = 7.6,* 4.8, 2.6 Hz, 2H), 7.39 (t, *J* = 8.4 Hz, 1H), 4.27 (s, 1H), 3.73 - 3.63 (m, 2H), 3.58 (s, 1H), 3.54 - 3.48 (m, 2H), 3.48 - 3.42 (m, 1H), 3.39 (dd, *J* = 11.1, 4.9 Hz, 1H), 3.30 - 3.20 (m, 3H), 3.10 (d, *J =* 13.2 Hz, 1H), 3.02 - 2.91 (m, 1H), 2.79 - 2.73 (m, 1H), 2.58 (d, *J =* 10.9 Hz, 1H), 2.48 - 2.40 (m, 2H), 2.39 - 2.35 (m, 2H), 2.33 (d, *J* = 8.3 Hz, 3H), 2.27 (d, *J* = 22.8 Hz, 2H), 1.98 (d, *J* = 12.6 Hz, 1H). **¹³C-NMR** (101 MHz, MeOD-*d₄*) δ (ppm) = 169.9, 169.6, 152.6, 147.5, 135.0, 135.0, 132.7, 131.6, 131.0, 129.6, 129.0, 125.1, 124.7, 124.5, 122.1, 60.2, 56.2, 56.1, 54.5, 54.4, 54.0, 53.9, 44.5, 40.8, 40.7, 35.5, 34.5, 34.1, 32.9, 32.0, 27.6, 16.0, 15.9, 15.7. **HRMS** (ESI+): m/z calc. for C₂₇H₃₀Cl₂N₅O₄SSe: [M+H]⁺ 670.05553, found 670.05587.

### Reference fluorogenic probes

### Di-tert-butyl (disulfanediylbis(ethane-2,1-diyl))dicarbamate (X24)

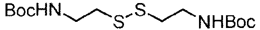

Cysteamine hydrochloride **X23** (1.00 g, 8.8 mmol) was dissolved in MeOH (30 mL) and the free base cysteamine was generated by addition of KOH (1.16 g, 17.6 mmol). Addition of methanolic I₂ until a slight colour persisted permanently, followed by evaporation of all volatiles gave 2-(2-aminoethyldisulfanyl)ethanamine that was N-Boc protected according using Boc₂O (2.2 equiv.) and DIPEA (2.5 equiv.) in dioxane/water (1:1, 0.5 M) gave **X24** (0.70 g, 2.0 mmol, 45%) as colourless crystalline needles after recrystallization from MeOH.

### 2,2'-Disulfanediylbis(N-methylethan-1-amine) dihydrochloride (X25)

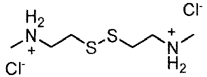

*Step 1:* **X24** (0.78 g, 2.21 mmol) was dissolved in anhydrous DMF (0.02 M) and solid NaH (2.2 eq) was added at 0 °C, followed by addition of Mel (2.3 eq) in one portion. The mixture was stirred for 0.5 h, then allowed to warm to r.t. and stirred for a further hour. The mixture was diluted with EtOAc and washed with sat. aq. NaCl (2×). The combined aq. layers were extracted with EtOAc (3×50 mL) and the combined organic layers were dried over Na₂SO₄. filtered and concentrated and purified by FCC to afford di-tert-butyl (disulfanediylbis(ethane-2,1-diyl))bis(methylcarbamate) as a colourless solid.

*Step 2:* Deprotection of the material obtained in step 1 was achieved according to general procedure E and the resulting diamine dihydrochloride **X25** (0.46 mg, 1.82 mmol, 82%) was obtained as a colourless solid.

**¹H-NMR** (400 MHz, DMSO-*d*₆): δ (ppm) = 9.27 (s, 4H), 3.19 (d, *J* = 7.4 Hz, 4H), 3.09 (dd, *J* = 8.1, 5.7 Hz, 4H), 2.56 (s, 6H). **¹³C-NMR** (101 MHz, DMSO-*d*₆): δ (ppm) = 47.0, 32.4, 32.3.

### 4-chloro-2-(6-chloro-4-oxo-3,4-dihydroquinazolin-2-yl)phenyl methyl(2-((2-(methyl amino)ethyl)disulfaneyl)ethyl)carbamate (X26)

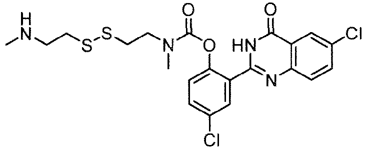

**X22** was coupled to **X25** (715 mg, 2.82 mmol) according to **general protocol F**. Purification by FCC (DCM/MeOH) gave **X26** as a colourless powder (245 mg, 0.477 mmol, 44%).

**R*_{f}*** = 0.22 (DCM/MeOH 9:1). **¹H-NMR** (400 MHz, tetrachlorethane-*d*₂, 373 K): δ (ppm) = 8.20 (t, J = 1.5 Hz, 1H), 7.87 (d, *J* = 2.6 Hz, 1H), 7.71 (d, *J* = 1.5 Hz, 2H), 7.48 (dd, *J* = 8.7, 2.6 Hz, 1H), 7.24 (d, *J* = 8.7 Hz, 1H), 3.62 (s, 2H), 3.23 (d, *J* = 6.2 Hz, 2H), 3.18 (d, *J* = 6.1 Hz, 2H), 3.03 (s, 3H), 2.88 (s, 2H), 2.66 (s, 3H). **¹³C-NMR** (101 MHz, DMSO-*d*₆): δ (ppm) = 160.8, 153.1, 150.6, 147.9, 147.2, 134.9, 131.5, 131.4, 130.1, 129.6, 129.4, 128.5, 125.2, 125.0, 122.4, 47.7, 47.0, 35.1, 34.6, 32.4, 32.3. **HRMS** (ESI+) m/z calc. for C₂₁H₂₃Cl₂N₄O₃S₂ [M+H]⁺ 513.05831, found 513.05886.

### 3-(Cyclohexylamino)-1-(4-methylpiperazin-1-yl)propan-1-one dihydrochloride (X27)

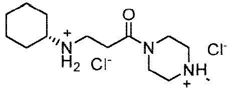

According to general procedure D, cyclohexylamine (57 µL, 0.50 mmol, 1.25 eq) and **X16** (77 mg, 0.40 mmol, 1.0 equiv.) were reacted in MeOH (1.2 mL, 0.3 M) under microwave irradiation (120 °C, 60 min). Boc-protection using Boc₂O (0.13 g, 0.60 mmol, 1.5 equiv.) and DIPEA (0.58 mL, 1.2 mmol, 3 equiv.) in DCM (2 mL, 0.2 M) gave **X27a** (0.12 g, 0.32 mmol, 81%) upon isolation by flash chromatography (DCM→DCM/MeOH 9:1).

TLC R_{f} = 0.45 (DCM/MeOH 9:1). **HRMS** (ESI+): m/z calc. for C₁₉H₃₆N₃O₃: [M+H]⁺ 354.27512, found 354.27544.

Boc-deprotection was conducted following general procedure E. Intermediate **X27a** (46 mg, 0.13 mmol, 1.0 equiv.) was dissolved in DCM (1.3 mL, 0.1 M) and reacted with HCl (0.33 mL, 4 M in dioxane, 1.3 mmol, 10 equiv.). Filtration of the precipitates formed during the reaction gave compound **X27** (42 mg, 0.13 mmol, 80% over 3 steps) as a colourless solid which was used without further analysis or purification.

### P-CC60-PQ (X28)

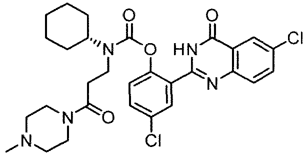

Prepared from **X27** (42 mg, 0.13 mmol, 1.0 equiv.) and **X22** (3.5 mL, 0.04 M in DCM, 0.14 mmol, 1.1 equiv.) according to general protocol F. Purification by flash chromatography (DCM→DCM/MeOH 9:1) gave **X28** as a colourless solid (55.0 mg, 0.088 mmol, 68%). Further purification by preparative HPLC (MeCN/H₂O, 0.1% FA) yielded **X28** as a colourless solid. Light blue solid-state fluorescence was observed under UV-light.

**TLC** R_{f} = 0.55 (DCM/MeOH 9:1). **¹H-NMR** (400 MHz, MeOD-*d*₄): *δ* (ppm) = 12.73 (s, 1H), 8.08 (d, *J* = 4.4 Hz, 1H), 7.88 (t, *J* = 7.7 Hz, 1H), 7.85 - 7.78 (m, 1H), 7.65 (dt, *J* = 14.4, 8.3 Hz, 2H), 7.37 (dd, *J* = 8.7, 3.7 Hz, 1H), 3.89 (s, 1H), 3.53 - 3.43 (m, 2H), 3.38 (s, 2H), 3.25 (s, 1H), 3.22 - 3.14 (m, 1H), 2.97 (s, 1H), 2.64 - 2.54 (m, 1H), 2.16 (d, *J* = 3.5 Hz, 6H), 2.09 - 2.02 (m, 1H), 1.66 (s, 2H), 1.60 (d, *J* = 12.8 Hz, 1H), 1.57 - 1.45 (m, 1H), 1.35 (t, *J* = 10.5 Hz, 3H), 1.30 - 0.88 (m, 4H). **¹³C-NMR** (101 MHz, DMSO-d₆) δ (ppm) = 168.8, 168.3, 152.6, 152.2, 148.0, 147.7, 134.6, 131.1, 130.2, 130.0, 129.2, 125.4, 124.9, 122.4, 56.4, 56.1, 54.8, 54.7, 54.3, 54.2, 45.7, 44.6, 44.3, 40.8, 40.7, 33.2, 31.9, 30.6, 29.7, 25.4, 24.8. **HRMS** (ESI+): m/z calc. for C₂₉H₃₄Cl₂N₅O₄: [M+H]⁺ 586.19824, found 586.19933.

### Precursors to Therapeutic Prodrugs

### 5-(3-(Azetidine-1-yl)propoxy-1H-indole-2-carboxylic acid (X29)

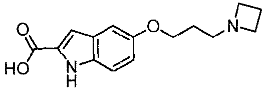

Step 1: 5-hydroxy-1*H*-indole-2-carboxylic acid (1.27 g, 7.15 mmol) was dissolved in MeOH (14 mL) and cooled to 0 °C. SOCl₂ (0.78 mL, 1.28 g, 10.7 mmol) was added dropwise and the resulting mixture was heated to 75 °C for 2 h, then cooled to 25 °C and diluted with EtOAc (100 mL). The organic layer was washed with sat. aq. NaHCO₃ and sat. aq. NaCl and the combined aq. layers were extracted with EtOAc (3×50 mL). The combined organic layers were dried over Na₂SO₄, filtered, concentrated under reduced pressure and purified by FCC (isohexane/EtOAc) to give methyl 5-hydroxy-1H-indole-2-carboxylate as a colourless powder (1.32 g, 6.9 mmol, 97%).

Step 2: PPh₃ (125 mg, 0.52 mmol) was dissolved in THF (0.25 M) and cooled to 0 °C. DIAD (94 µL, 0.48 mmol) was added dropwise and the mixture was stirred at r.t. for 30 min. A solution of 3-(azetidin-1-yl)propan-1-ol (0.5 M) in THFand a solution of methyl 5-hydroxy-1H-indole-2-carboxylate (0.5 M) in THF were added subsequently. The mixture was allowed to warm to r.t. for 1 h, was then further stirred for 15 h, before being concentrated under reduced pressure and purified using FCC to give methyl 5-(3-(azetidin-1-yl)propoxy)-1H-indole-2-carboxylate (76 mg, 0.27 mmol, 62%) as a colourless oil.

Step 3: Methyl 5-(3-(azetidin-1-yl)propoxy)-1H-indole-2-carboxylate (222 mg, 0.77 mmol) was dissolved in MeOH:H₂O (3:1, 40 mL) and solid KOH (61 mg, 0.92 mmol) was added. The resulting solution was heated to 75 °C for 15h, was cooled to r.t. and a solution of HCl (0.74 mL, 1.25 M in MeOH) was added. The resulting heterogenous mixture was concentrated under reduced pressure to obtain compound **X29** (303 mg, 99%. 68% w/w calc. purity) contaminated with KCI. An analytically pure sample was separately obtained from RP chromatography (H₂O/MeCN) giving **X29** as a colourless powder.

**¹H-NMR** (400 MHz, DMSO-*d*₆): δ (ppm) = 11.14 (d, *J* = 2.2 Hz, 1H), 7.25 (d, *J* = 8.8 Hz, 1H), 7.08 (d, *J* = 2.4 Hz, 1H), 6.76 (d, *J* = 2.1 Hz, 2H), 6.74 (d, *J* = 2.4 Hz, 1H), 4.02 (t, *J* = 6.8 Hz, 2H), 3.63 (t, *J* = 7.5 Hz, 4H), 2.91 (t, *J* = 6.5 Hz, 2H), 2.20 (p, *J* = 7.5 Hz, 2H), 1.86 (p, *J* = 6.7 Hz, 2H). **¹³C-NMR** (101 MHz, DMSO-*d*₆): δ (ppm) = 165.1, 152.6, 134.1, 131.7, 127.6, 114.3, 112.9, 104.3, 102.6, 65.3, 53.2, 53.0, 25.4, 16.2. **HRMS** (ESI-) m/z calc. for C₁₃H₁₃NO₂Cl [M-H]⁻: calc. 275.13902, found 275.13910.

### tert-Butyl (1S)-1-(chloromethyl)-5-((methyl((S)-1,2-thiaselenan-4-yl)carbamoyl)oxy)-2,3-dihydro-1H-3λ⁴-benzo[e]indole-3-carboxylate (X30)

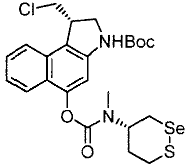

*Step* 1: Following Tietze *et al.* (Tietze, L. F. et al. ChemMedChem 2008, 3, 1946-1955. https://doi.org/10.1002/cmdc.200800250), to commercial (*S*)-3-(Boc)-5-(benzyloxy)-1-(chloromethyl)-1,2-dihydro-3H-benzo[e]indole (591 mg, 1.39 mmol) were added dry THF (0.05 M), Pd/C (297 mg, 10% on charcoal), and aq. NH₄HCO₂ (2.8 mL of a 4 M aq. solution). The mixture was stirred for 80 min, filtered over Kieselgur and washed with EtOAc (20 mL) and the filtrates were concentrated under reduced pressure to obtain (*S*)-3-(Boc)-1-(chloromethyl)-5-hydroxy-1,2-dihydro-3*H*-benzo[e]indole as a colourless solid (460 mg, 1.38 mmol, 99%).

*Step* 2: An oven-dried Schlenk flask was charged with (*S*)-3-(Boc)-1-(chloromethyl)-5-hydroxy-1,2-dihydro-3*H*-benzo[e]indole (88 mg, 0.30 mmol, 1.0 equiv.) under inert gas atmosphere, the compound was dissolved in anhydrous DCM (0.1-0.2 M) and the resulting solution was cooled to 0 °C. Triphosgene (1.2 eq, 0.05 M solution in anhydrous DCM) was added in one portion and DIPEA (4.0 eq, 0.05 M solution in anhydrous DCM) was added. Amine mediated *in-situ* formation of phosgene and hydrochloride was indicated, the resulting mixture was further stirred at 0°C for 0.5 h, was then allowed to warm to r.t. and was further stirred for 0.5h. All volatile compounds were removed using an external liquid nitrogen trap to afford the corresponding chloroformate derivative as a solid. The trapped residue mixture containing residual phosgene was quenched by subsequently adding an aq. solution of NaOH (2 M) and diisopropylamine (10 eq).

*Step 3:* **X12** (95 mg, 0.32 mmol, 1.0 equiv.) was deprotected according to general procedure E, using HCl (4 M in dioxane, 3.2 mmol, 10 equiv.) in DCM (0.1 M, 3 mL). The intermediary amine hydrochloride **X30a** was isolated through filtration of the reaction mixture and used without further purification.

Step 4: An oven-dried round-bottom flask was charged with (*R*)-N-methyl-1,2-thiaselenan-4-amine hydrochloride **X30a** (69 mg, 0.30 mmol, 1.0 equiv.) and the compound was suspended in anhydrous DCM (0.02 M). DIPEA (2.4 eq, 0.05 M solution in anhydrous DCM) was added to afford a clear solution of the corresponding free amine. A solution (0.01 M in anhydrous DCM) of the chloroformate synthesised in *Step 2* was added dropwise to the reaction flask at 0°C and the resulting mixture was further stirred for 0.5 h, was then allowed to warm to r.t. and was further stirred for 1-2 h. The reaction was stopped by adding a sat. aq. solution of NaHCO₃ (50 mL), the mixture was diluted with DCM. The organic layer was seperated and washed with a sat. aq. solution of sodium chloride (2×50 mL). The combined aq. layers were extracted with DCM (2×100 mL) and the combined organic layers were dried over Na₂SO₄, filtered and concentrated under reduced pressure to afford the crude product as a coloured solid. Purification was achieved using FCC and the desired product **X30** was obtained as a colourless solid (84 mg, 0.15 mmol, 51%).

NMR experiments at r.t. showed two rotameric species - only signals for the major rotamer are reported. **¹H NMR** (500 MHz, CDCl₃) *δ* (ppm) = 8.05 (br-s, 1H), 7.78 (dd, *J* = 20.5, 8.9 Hz, 1H), 7.70 (d, *J* = 8.4 Hz, 1H), 7.48 (dt, *J* = 24.5, 7.7 Hz, 1H), 7.42 - 7.30 (m, 1H), 4.50 (t, *J =* 10.7 Hz, 1H), 4.33 - 4.24 (m, 1H), 4.24 - 4.10 (m, 1H), 4.02 (t, *J* = 9.9 Hz, 1H), 3.93 (d, *J =* 10.8 Hz, 1H), 3.47 (t, *J* = 10.9 Hz, 1H), 3.42 - 3.29 (m, 2H), 3.14 - 2.78 (m, 2H) 3.13 (s, 2H), 2.47 - 1.92 (m, 2H), 1.58 (s, 9H). **¹³C NMR** (126 MHz, CDCl₃) *δ* (ppm) = 154.1, 152.5, 148.5, 141.4, 130.2, 127.7, 124.4, 122.7, 122.5, 120.3, 109.4, 81.5, 56.2, 53.1, 46.4, 41.8, 36.1, 33.8, 30.1, 28.6, 27.2.

### Compounds of the invention: Therapeutic prodrugs

### Me-SeS60-CBI-API (P8)

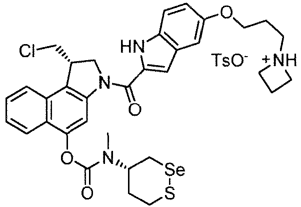

An oven-dried round-bottom flask was charged with **X30** (41 mg, 0.074 mmol, 1.0 equiv.) and the compound was dissolved in anhydrous DCM (0.05 M, 1.5 mL). The solution was cooled to 0 °C and a solution of HCl (1.5 mL, 4 M in dioxane, 6.0 mmol, 80 equiv.) was added in one portion. The mixture was stirred for 0.5 h, was then allowed to warm to r.t. and was further stirred for 4 h until quantitative turnover was confirmed. All volatile compounds were removed under reduced pressure to afford the solid amine hydrochloride without further purification.

An oven-dried round-bottom flask was charged with the amine hydrochloride and the compound was dissolved in anhydrous DMF (0.03 M). The solution was cooled to 0 °C and 5-(3-(azetidine-1-yl)propoxy-1*H*-indole-2-carboxylic acid (**X29**, 35.7 mg, 68% w/w, 0.088 mmol, 1.1 equiv.), EDCI (4 eq) and p-TsOH (1.1 eq) were added sequentially as solids. The mixture was stirred for 0.5 h, was then allowed to warm to r.t. and was further stirred for 15 h until quantitative turnover was confirmed. All volatile compounds were removed under reduced pressure to afford the crude mixture as a coloured solid. Purification by FCC (DCM/MeOH) gave the desired final amide as a yellow solid. Optional purification using a preparative HPLC system (H₂O/MeCN/HCO₂H) gave the title compound **P8** as a colourless solid (27 mg, 0.038 mmol, 51%).

**TLC** R_{f} = 0.57 (DCM/MeOH, 4:1). NMR experiments at r.t. showed two rotameric species - only signals for the major rotamer are reported. **¹H NMR** (400 MHz, DMSO-d₆) *δ* (ppm) = 11.64 (s, 1H), 9.86 (s, 1H), 8.21 (s, 1H), 8.04 (d, *J* = 8.5 Hz, 1H), 7.84 (d, *J* = 8.8 Hz, 1H), 7.61 (q, *J* = 7.3 Hz, 1H), 7.52 (t, *J* = 7.8 Hz, 1H), 7.47 (d, *J* = 7.9 Hz, 1H, TsO⁻), 7.43 (d, *J* = 9.0 Hz, 1H), 7.18 (s, 1H), 7.14 (s, 1H), 7.11 (d, *J* = 8.0 Hz, 1H, TsO⁻), 6.95 (dd, *J* = 8.9, 2.6 Hz, 1H), 4.96 - 4.78 (m, 2H), 4.61 (d, *J* = 9.8 Hz, 1H), 4.41 (s, 1H), 4.33 - 4.22 (m, 1H), 4.14 - 3.97 (m, 6H), 3.56 - 3.39 (m, 1H), 3.25 - 2.95 (m, 1H), 3.15 (s, 2H), 2.41 - 2.30 (m, 2H), 2.28 (s, 1H, TsO⁻), 2.23 - 2.00 (m, 2H), 1.95 (p, *J* = 6.5 Hz, 2H). **¹³C NMR** (126 MHz, DMSO-*d*₆) *δ* (ppm) = 160.2, 152.7, 150.7, 147.3, 145.8 (TsO⁻), 142.8, 141.4, 137.5, 135.6 (TsO⁻), 131.8, 130.8, 129.5, 128.0 (TsO⁻), 127.7, 127.5, 125.5 (TsO⁻), 124.3, 123.7, 122.4, 117.3 , 115.9, 113.3, 110.4, 105.5, 103.4, 64.9, 56.0, 54.8, 54.0, 51.8, 47.2, 41.1, 32.9, 30.0, 26.6, 24.3, 20.8 (TsO⁻), 15.9.

### 5. Biological Testing

### 5.1 Challenge with GR/GSH/Grx system reductants (see Figure 1)

This cell-free assay examined the stability of dichalcogenide-based reduction-triggered compounds of the invention when exposed to cellular monothiol reductant GSH, and its cellular partner reductants glutathione reductase (GR) and glutaredoxins (Grx1 and Grx2).

Assay Conditions: A black 96-well plate with black bottom was charged with solutions of fluorophore-releasing reduction-triggered probes that are compounds of the invention (**P1**, **P2**, **P3**, **P6** and **P7**) or that are not compounds of the invention and reveal the performance of prior art systems (disulfide **X26**), reaching 100 µL final volume, 1% final DMSO, 10 µM probe, aq. TE buffer (pH 7.4), with the indicated concentrations of reductant (in Fig 1a) or concentration ratios of reductant to probe (in Fig 1b-d). TCEP (100 µM) was used as a quantitative reference reductant; and in the GR/GSH/Grx assays (Fig 1c-d), GR was used at 20 nM, GSH at 100 µM, to maintain Grx in a reduced state suitable for challenging the probes without supplying a high concentration of either GSH or GR. Reaction mixtures were incubated at 37 °C for up to 6 h while measuring fluorescence intensity F over time, using a BMG Labtech FLUOstar Omega platereader (λ_{exc} = 355 nm, λₑₘ = 530 nm).

Data Representation: F^{TCEP} is the maximum possible fluorescence increase, corresponding to complete reduction-triggered cargo release by the TCEP incubation. F/F^{TCEP} thus gives the fluorescence intensity, as a fraction of the maximum possible fluorescence signal, at the indicated times.

Experimental Results and Discussion: In general, Figure 1 illustrates that **P1**, **P3** and **P6** (compounds of the invention) totally resisted releasing cargo when challenged with GSH system reductants at relevant cellular levels; **P2** and **P7** had high resistance to GSH challenge and total resistance to Grx challenge. Particularly, their slow, small response to high GSH levels (supraphysiological 10 mM in Fig 1a; titrations in Fig 1b), was compared to the rapid response by comparison probe **X26** to small, subphysiological GSH levels (1 mM GSH in Fig 1a; titration in Fig 1b). This reflected that compounds of the invention could have desirably very high resistance to ordinary cellular reducing conditions (such as 2 to 5 mM GSH); while prior art dichalcogenide-based proagents such as **X26** could offer no stability against non-enzymatic reduction by monothiols like GSH at cellular concentrations. The non-response of all compounds of the invention at for example 3 h treatment with 30 equiv. GSH also showed that compounds of the invention were stable to non-reductive cargo release mechanisms (including, being stable to hydrolysis) which is also desirable. By doing both, they can respond to the need described above (e.g. by resisting normal GSH levels and spontaneous hydrolysis mechanisms, they can instead be suitable for responding to reductive activity of specific reductive enzymes).

### 5.2 Enzymatic activation assay (see Figure 2)

This cell-free assay (see Figure 2) examined the processing of dichalcogenide-based fluorophore-releasing reduction-triggered probes that are compounds of the invention, by components of the cellular thioredoxin system.

Assay conditions and data representation: A black 96-well plate with black bottom was charged with solutions of probes (**P1**, **P2**, **P3**, **P6**, **P7**) to reach final reaction conditions: 100 µL of aq. TE buffer (pH 7.4), 10 µM probe concentration, 1 vol% DMSO, and 20 nM of human recombinant thioredoxin reductase 1 (TrxR1; in Fig 1a), or 20 nM of either TrxR1 or thioredoxin reductase Sec498Cys mutant (TrxR1 U498C or "mut"; in Fig 1b), or the indicated probe-to-reductant concentration ratios of TrxR1 (in Fig 1c) or human recombinant thioredoxin 1 (Trx1; in Fig 1d); with the final step being the addition of NADPH (100 µM final). The reaction mixtures were incubated at 37 °C for 3 to 6 h as shown with timecourse measurements of fluorescence as for Fig 1. Data representation as for Fig 1.

Experimental Results and Discussion: In general, Figure 2 illustrates that native, selenium-incorporating TrxR1 caused **P6** and **P7** (compounds of the invention) to release cargo very rapidly (Fig 2a) and sensitively (Fig 2c), and that adding increasing amounts of Trx1 did not affect the outcomes (Fig 2d), but that the U498C mutant did not cause cargo release (Fig 2b). This suggests that the probes were selectively processed by the highly distinctive selenolthiol active site of the enzyme TrxR1, for which it is highly desirable to develop efficient and selective probes and prodrugs. Also shown, is that compounds of the invention were created that were less responsive to TrxR (**P1** and **P2**) or nonresponsive to TrxR (**P3**) under ordinary conditions, and these compounds could therefore act as sensors for alternative reductase activities or for deregulated redox conditions, which is also of interest. In conclusion, the novel reduction-sensing motifs of molecules of the invention could therefore indeed be processed by a valuable endogenous redox enzyme with high sensitivity and selectivity for the native functional state, making compounds of the invention useful for selective cargo release triggered by activity of specific enzymes or potentially triggered by unusual redox conditions.

### 5.3 Cellular reduction assay (see Figure 3)

Assay Conditions: Cells were cultured under standard conditions (Gao, L. et al. Cell Chemical Biology 2021, 28, 1-14. https://doi.org/10.1016/j.chembiol.2020.11.007), left to adhere for 24 h, then compounds were added to 100 µM with 1%vol DMSO. Fluorescence timecourse measurements were acquired as in the chemoreductant assay, with all conditions in triplicates.

Data Representation: F.I.(t) is the fluorescence intensity signal increase as compared to time zero signal (fluorescence intensity immediately following compound addition, before probe activation could occur), in arbitrary units although with the same detection settings for all probes releasing the same cargo. This corresponds to reduction-triggered cargo release.

Experimental Results and Discussion: Figure 3a illustrates that compounds of the invention **P6** and **P7** generated strong fluorescent signal in HeLa cervical cancer cell line with the increase being time- and dose-dependent. Comparison probe **X26** (linear disulfide) also generated signal, indicating that linear disulfides are also reductively processed in HeLa cells. The control compound **X28** (non-reducible cyclohexyl control) did not generate signal, illustrating that the carbamate system used in the compounds of the invention was not responsible for their signal generation (i.e. the carbamate was robust to cellular hydrolysis).

Figure 3b illustrates that compound of the invention **P6** dose- and time-dependently generated signal also in A549 lung cancer cell line indicating that compounds of the invention could be usefully applied in diverse cell lines.

Figure 3c shows that the signal generation of **P6** was strongly suppressed by selenium starvation of the cells (Lacey; Biochemistry 2008 47, 12810-12821) indicating that its cellular processing depended on one or more selenium-dependent reductases; and since it was shown to be stable against the GR/GSH/Grx systems *in vitro* (Fig 1) yet strongly activated by the selenium-dependent reductase TrxR1 *in vitro* only when it incorporates selenium (Fig 2), this shows that **P6** was likely to have been processed by native (selenium-incorporating) TrxR1 in cells. The comparison disulfide probe **X26** (that was shown to be GSH labile *in vitro* in Fig 1) was not sensitive to selenium supplementation, which shows that its cellular processing is likely not to be TrxR-selective: indeed this is a common feature of prior art in dichalcogenide probes, since no satisfactory cellularly-TrxR-selective designs have been demonstrated until now.

Figure 3d-3e reinforce the conclusions from Fig 3c that compound of the invention **P6** is selectively processed in cells by TrxR1, since the signal of **P6** was almost entirely suppressed by knockout (Fig 3d; in MEF cells) or chemical inhibition (Fig 3e; in HeLa cells) of this enzyme.

### 5.4 in vitro cell viability assay (see Figure 4)

Assay Conditions: HeLa cells were cultured under standard conditions (Gao, L. et al. Cell Chemical Biology 2021, 28, 1-14. https://doi.org/10.1016/j.chembiol.2020.11.007), left to adhere for 24 h, then compounds were added from DMSO stock solution and adjusted to 1%vol DMSO. Cells were further incubated for 48 h, then 0.20 volume equivalents of a solution of resazurin in PBS (0.15 mg/mL) were added and incubated for 3 hours, then fluorescence (ex 544 nm, em 590 nm) was detected on a fluorescence platereader. Cell viability (%) is calculated from the fluorescence values, relative to a control experiment with no compound added.

Experimental Results and Discussion: Figure 4 illustrates that prodrug compound of the invention **P8** was significantly toxic to cells (EC₅₀ < 100 nM). This illustrates that it could release its bioactive cargo, the DNA alkylator CBI-OH, under the action of cellular reductive species. Taken together with Figures 1 to 3 (fluorogenic cargo release), this indicates that compounds according to the invention could usefully release a range of cargos in cell-free or cellular conditions, with tunable degree of release depending on the trigger.

### RX1 enables quantitative high-throughput cellular screening for TrxR1 inhibitors

Inhibitors of TrxR1 hold promise as therapeutics for treating cancer, autoimmune and inflammatory diseases. The chalcophilic gold complex auranofin (Ridaura) is one effective though poorly selective TrxR inhibitor, clinically used against the autoimmune inflammatory disease, rheumatoid arthritis. It and many analogue complexes have reached late-stage clinical trials in cancer, motivated by tumoral upregulation and reliance upon redox systems (Abdalbari, F. H. et al., Discover Oncology 12, 42 (2021)). Other TrxR inhibitor classes include redox-active species and organic electrophiles. Until now, TrxR1 assays to guide inhibitor development were enzymatic ("cell-free") or utilised cell lysates. The technical and cost challenges of expressing and purifying mTrxR with near-quantitative selenium-incorporation on a sufficient scale for large screening, have limited enzymatic TrxR screening: only one high-throughput screen (HTS) has been reported (Stafford, W. C. et al., Sci. Transl. Med. 10, eaaf7444 (2018); Prast-Nielsen, S. et al., Free Radical Biology and Medicine 50, 1114-1123 (2011)). Enzymatic or lysate assay hits can be irrelevant in cells (poor permeability, biolocalisation, or target specificity), report artifactual hits (e.g. fluorescence quenching or aggregation), and can identify promiscuous compounds that are unlikely to be useful in biology, rather than selective compounds. Biochemical assays also cannot identify compounds that are biotransformed into active inhibitors: a field that is recently emerging for selenoprotein targeting (Eaton, J. K. et al., Nature Chemical Biology 16, 497-506 (2020)).

Cellular HTS can be far simpler and cheaper to perform; and allows screening different cell lines, towards therapeutic TrxR inhibitors effective in target cells (with varied expression levels of TrxR and of likely off-target thiol/selenol species), while controlling for drug-relevant performance issues such as upregulated electrophile detoxification and drug efflux pumping, and cell-type-dependent uptake. If a selective probe/readout is used, the data can also be more likely to emphasise selectivity over nonspecific reactivity, making it more actionable in drug development. A HTS-suitable probe must be TrxR-selective; but it must also pass many additional criteria for automatic operation, including (a) broad dynamic range (negligible background and cellular crosstalk, high signal-to-noise); (b) broad linear range (for quantitative use); (c) minimal count of error-prone steps (no additional reagents or handling); (d) no manual tailoring of conditions or processing by compound classes; (e) high-quality data: stable signal, with high precision (small deviations) and high accuracy (confidence of TrxR quantification, minimum interference from test compounds).

The stability and constant environment of **P6**'s signal (crystallising fluorophore, protected from interferences), its low background (ESIPT quenched probe) and low crosstalk (high Stokes shift), and its ability to directly generate a readout, were promising features. We therefore set out to develop the first cellular quantitative HTS (qHTS) assay for TrxR1 inhibitors, using **P6,** and performing pilot screening with the 1280-compound LOPAC₁₂₈₀ library. LOPAC is intended to cover drugs and drug-like scaffolds with much comparative data on target selectivity, potency, cellular and in vivo bioactivity, without focus on any particular mechanism of action. The LOPAC library was used in the only previously reported qHTS enzymatic TrxR assay (in 2011), and there is 82% overlap in composition between the 2011 and 2021 version (Prast-Nielsen, S. et al., Free Radical Biology and Medicine 50, 1114-1123 (2011)).

Cellular screening using **P6**, optimized for HTS with 1536-well plates (assay volume 6 µL, 500 cells/well) over 4 h run-time, reproduced the strong performance seen in 96-well format. Untreated vs no-cell controls gave a 7-fold raw signal to background ratio [S:B] without background compensation, with a high Z' value of 0.64, suitable for HTS (Fig. 5a). **P6** signal was linear over the assay time, and linearly reflected turnover rate (varied **P6** concentration or cell count; Fig. 5b). Pre-incubating cells for 1 h with reference inhibitors prior to **P6** gave concentration-dependent inhibition consistent with reported values (Fig. 5c).

A LOPAC₁₂₈₀ screen using this assay protocol performed well (Z' = 0.63, S:B 7:1). Compounds with apparent toxicity within the assay time were excluded by a separate viability counter-assay. Moderate cut-off criteria (IC₅₀ <20 µM and curve form) gave a <1.5% hit rate (18 of 1278 compounds, plus TRi-1 and TRi-2; Fig. 5d). None of the LOPAC₁₂₈₀ compounds were expected to have truly TrxR-selective inhibitory activity in cells: the panel serves to demonstrate HTS assay performance and likely hit rates in larger-scale screening, and to identify trends among hit classes. Pleasingly, the hit rate was manageably low, and the hits are indeed likely selenol-reactive species: 3 heavy metal complexes, 13 organic electrophiles or redox-active species, and a known TrxR-inhibiting porphyrin which may act via redox (Prast-Nielsen, S. et al., Free Radical Biology and Medicine 50, 1114-1123 (2011)) encouragingly, only one non-obvious hit was present, the glutamate receptor ligand AIDA. pIC₅₀ values also followed likely cellular reactivity: no change despite ca. 1.1 LogP difference between lipophilic vinylsulfones BAY 11-7085 (methyl) and BAY 11-7082 (*tert*-butyl); but 10-fold lower for permeability-limited drug aurothioglucose than its ester prodrug auranofin; etc (Fig. 5d). Matching expectations, with the exception of auranofin, none of these species approached the potency of TRi-1 (Stafford, W. C. et al., Sci. Transl. Med. 10, eaaf7444 (2018)).

### RX1 enables comparing cellular to cell-free potencies, which may orient the development of effective and cellularly-selective TrxR1 inhibitors

One opportunity afforded by **P6**-enabled cellular screening is to compare, for the first time, cell-free to cellular TrxR inhibition by species, to assess trends for cellularly-useful or -less-useful inhibitors. 1047 LOPAC compounds were assessed across both the screens (Prast-Nielsen, S. et al., Free Radical Biology and Medicine 50, 1114-1123 (2011)), of which 1011 generated robust enough data quality for comparisons. Of these, 993 compounds were inactive in both assays, and 7 were active in both. This 99% overlap of conclusive results between **P6** and the purified enzyme screen speaks strongly to the precision of the cellular **P6** assay. Only 2 compounds, IPA-3 and chloro-APB, were active in the cellular screen despite inactivity in the enzymatic assay: and these are catechol-like species with plausible redox activity in cells. To us, this indicates that the cellular **P6** assay has excellent robustness against false positives. Given that hits were classed up to 20 µM, while **P6** relies on precipitation of >1 µM of a flat aryl fluorophore for signal generation, we had feared false positive inhibition from the many LOPAC PAINS (pan-assay interference compounds) that have aggregation effects, or could alkylate the reduced intermediate **C.** However, not even powerful cellular PAINS rottlerin, rhodanine and myricetin (Plemper, R. K. et al., PLOS Pathogens 14, e1007038 (2018)) gave apparent potencies above 3% of that of the reference inhibitor TRi-1 (Fig. 5d). Finally, 9 compounds were inactive in the **P6** assay despite activity in the enzymatic screen. These were also redox-actives and electrophiles (catechols, α-haloketone, nitrosyl donor, Michael acceptors, wortmannin), which may indicate filtering of low-quality hits by the more stringent cellular assay.

Interestingly, the ratios of cell-free to cellular potencies of the shared hits clustered with their reactivity (Fig. 5e-f). The S_{N}Ar-reactive species had near-identical cell-free and cellular potencies; heavy metals and nitrosylating agent Dephostatin had >10-fold potency loss in cells; and Michael system electrophiles up to ~100-fold potency loss. The on-target potency of irreversibly reactive compounds in cellular assays is strongly ruled by their biolocalisation and reaction rates with off-target species. We expect that our data comparison (Fig. 5f) reflects the degree of undesirable drug loss to off-targets according to compound class, suggesting how drug development can pursue both effective and selective inhibitors of this key oxidoreductase.

In conclusion, these strong results give confidence that cellular qHTS with **P6** is a valid and valuable TrxR inhibitor screening strategy, promising that focused libraries can be used with cell lines of choice to guide development of cellularly-acting TrxR inhibitors.

### 5.6 Summary

The compounds of the invention can have the unique feature of cellularly-selectively releasing their cargos under the activity of the key oxidoreductase TrxR1; they have comparable results across a range of cell lines; they are stable to hydrolysis; and they are entirely, or nearly entirely, stable to monothiol background from high levels of glutathione or monothiols which enables them to be cellularly-selective for redox-active proteins. Therefore the compounds of the invention can be selective probes or prodrugs to release a signal or a cargo such as a drug conditional upon the activity of a specific oxidoreductase or redox-active protein, a highly valuable feature for diagnostics and for prodrugs depending on the cargo used and the oxidoreductase or redox-active protein targeted.

## Claims

1. A compound having the formula (I)
A-L-B (I)
wherein
A is represented by 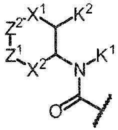 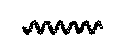
denotes the attachment point of A to L;
L is a bond or a self-immolative spacer
wherein the self-immolative spacer is selected from 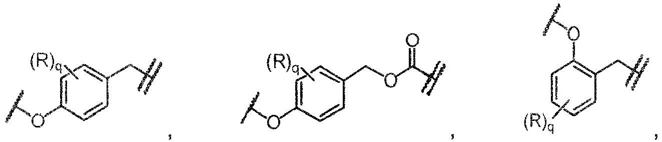 and 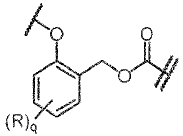
wherein
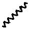 denotes the attachment point to A;
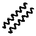 denotes the attachment point to B;
R is independently selected from halogen, -O(R^{r}), -N(R^{s})(R^{t}), -NO₂, -CN, and a heterocyclic group selected from azetidinyl, pyrrolidinyl, piperidinyl or morpholino, wherein the heterocyclic group is attached to the phenyl ring *via* the N atom;
q is 0, 1, 2, 3 or 4;
R^{r} is independently selected from -H, -C₁₋₄-alkyl and -(C₂₋₄-alkylene)-O-(C₁₋₄-alkyl), wherein -C₁₋₄-alkyl or -(C₂₋₄-alkylene)- can be optionally substituted by W;
R^{s} is independently selected from -H, -C(O)-C₁₋₄-alkyl and -C₁₋₄-alkyl; and R^{t} is independently selected from -H, -C(O)-C₁₋₄-alkyl and -C₁₋₄-alkyl;
B is represented by 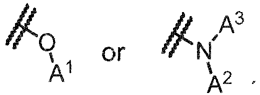 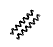
denotes the attachment point of B to L;
A' is selected such that A'-OH is a therapeutic, diagnostic or theranostic agent which contains an -OH group that is attached to a 5- or 6-membered aromatic or heteroaromatic ring;
A² and A³ are independently selected such that A²-NH-A³ is a therapeutic, diagnostic or theranostic agent which contains an -NH₂ or -NH- moiety;
K¹ is selected from -C₁₋₄-alkyl optionally substituted by W¹;
K² is selected from -H, -O-R^{j}, and -O-R^{k}; or
K¹ and K² are bonded together and K¹ and K² represent -X³-Y-X-, wherein X³ is bonded to N and X is bonded to C;
X is selected from -N(R^{a})-, -N(R^{b})-, -CR^{c}₂- and -O-;
X¹ is -(CR^{d}₂)ₘ-,
X² is -(CR^{e}₂)ₙ-;
X³ is -CR^{f}₂-;
Y is -(CR^{g}₂)ₚ-,
Z¹ and Z² are independently selected from S or Se such that either Z¹ is Se and Z² is S, or Z¹ is S and Z² is Se, or Z¹ and Z² are both Se;
W is independently from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or -(C₂₋₄-alkylene)- group via the N atom;
W¹ is selected from -OH, -C(O)-N(R^{h})(Rⁱ), -N(R^{h})(Rⁱ), -PR^{x}₃⁺, -C(O)-4-(morpholine), -C(O)-1-(piperazine), -C(O)-1-(4-methylpiperazine), -C(O)-1-(4-ethylpiperazine), and a heterocyclic group selected from azetidin-1-yl, pyrrolidin-1-yl, piperidin-1-yl, piperazin-1-yl, 4-methylpiperazin-1-yl, 4-ethylpiperazin-1-yl, 4-(2-hydroxyethyl)piperazin-1-yl, or morpholino, wherein that heterocyclic group is attached to the -C₁₋₄-alkyl or -(C₂₋₄-alkylene)- group via the N atom;
R^{a} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W;
R^{b} is an acyl group of a monopeptide selected from -proteinogenic amino acids attached via a carboxy group;
R^{c} groups are independently selected from -H and -C₁₋₄-alkyl;
R^{d} groups are independently selected from -H and -C₁₋₄-alkyl;
R^{e} groups are independently selected from -H and -C₁₋₄-alkyl;
R^{f} groups are independently selected from -H and -C₁₋₄-alkyl;
R^{g} groups are independently selected from -H and -C₁₋₄-alkyl;
R^{h} is independently selected from -H, -C₁₋₄-alkyl and -CH₂CH₂OH;
Rⁱ is independently selected from -H, -C₁₋₄-alkyl and -CH₂CH₂OH;
R^{j} is selected from -H, -C₁₋₄-alkyl, -C(O)-C₁₋₄-alkyl, -C(O)-O-C₁₋₄-alkyl, -C(O)-N(C₁₋₄-alkyl)₂, -S(O)₂-C₁₋₄-alkyl, and -(C₂₋₄-alkylene)-O-(C₁₋₄-alkyl), wherein -C₁₋₄-alkyl or -(C₂₋₄-alkylene)- can be optionally substituted by W¹,
R^{k} is an acyl group of a monopeptide selected from -proteinogenic amino acids attached via a carboxy group;
R^{x} groups are independently selected from phenyl- and 4-methoxyphenyl-;
m is 0, 1 or 2;
n is 1 or 2, provided that m+n is 2 or 3;
p is 0, 1, or 2, provided that when K¹ and K² are bonded together and K¹ and K² represent -X³-Y-X- and X represents -N(R^{a})- or -N(R^{b})-, then p = 1 or 2;
or any pharmaceutically acceptable salt, solvate or ester thereof.

2. The compound according to claim 1, wherein either Z¹ is Se and Z² is S, or Z¹ is S and Z² is Se.

3. The compound according to claim 1 or 2, wherein L is a bond.

4. The compound according to claim 1 or 2,
wherein L is a self-immolative spacer selected from

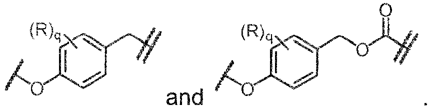

5. The compound according to any one of claims 1 to 4, wherein X¹ and X² are -CH₂-.

6. The compound according to any one of claims 1 to 5, wherein K¹ and K² are bonded together and K¹ and K² represent -X³-Y-X-.

7. The compound according to any one of claims 1 to 5, wherein K¹ is -C₁₋₄-alkyl optionally substituted by W¹ and K² is -H, -O-R^{j} or -O-R^{k}.

8. The compound according to any one of claims 1 to 7, wherein A¹-OH or A²-NH-A³ is selected from a diagnostically acceptable dye, a therapeutically acceptable DNA-alkylating agent, a therapeutically acceptable tubulin-inhibiting agent, and a therapeutically acceptable topoisomerase-inhibiting agent, or wherein A¹-OH or A²-NH-A³ is selected from 10-hydroxycamptothecin, 10-hydroxybelotecan, 10-hydroxygimatecan, 10-hydroxy-CKD-602, 10-hydroxy-BNP-1350, 10-hydroxy-sinotecan, topotecan, 7-ethyl-10-hydroxy-camptothecin (SN-38), 10-hydroxy-20-acetoxy-camptothecin, pyrrolobenzodiazepine, methotrexate, duocarmycin, CC-1065, doxorubicin, epirubicin, daunorubicin, pirarubicin, carminomycin, doxorubicin-N,O-acetal, 4-(bis(2-chloroethyl)amino)phenol, 4-(bis(2-bromoethyl)amino)phenol, 4-(bis(2-mesylethyl)amino)phenol, 4-((2-chloroethyl-2'-mesylethyl)amino)phenol, 5-hydroxy-seco-cyclopropabenzaindoles, 5-hydroxy-seco-(2-methyl-cyclopropa)benzaindoles, 5-hydroxy-seco-cyclopropamethoxybenzaindoles, 5-amino-seco-cyclopropabenzaindoles, etoposide, teniposide, GL331, NPF, TOP53, NK611, tubulysin A, tubulysin B, tubulysin C, tubulysin G, tubulysin I, monomethyl auristatin E, monomethyl auristatin F, dolastatin 10, dolastatin 15, symplostastin 1, symplostastin 3, narciclasine, pancratistatin, 2-epi-narciclasine, narciprimine, calicheamicin α1, calicheamicin β1, calicheamicin γ1, calicheamicin δ1, calicheamicin ε, calicheamicin θ, calicheamicin T, diclofenac, aceclofenac, mefenamic acid, clonixin, piroxicam, meloxicam, tenoxicam, lornoxicam, baricitinib, filgotinib, tofacitinib, upadacitinib, ruxolitinib, peficitinib, decemotinib, solcitinib, itacitinib, fostamatinib, SHR0302, leuco-methylene blue, leuco-methyl methylene blue, leuco-dimethyl methylene blue, leuco-toluidine blue, leuco-Azure A, leuco-Azure B, leuco-Azure C, leuco-Thionin, leuco-methylene violet, leuco-new methylene blue, leuco-Nile blue A, leuco-brilliant cresyl blue, firefly luciferin (D-Luciferin), umbelliferone, 4-trifluoromethylumbelliferone, 6,8-difluoro-4-methylumbelliferone, 7-hydroxycoumarin-3-carboxylic acid, 6,8-difluoro-7-hydroxy-5-methylcoumarin (DiFMU), 7-amino-4-methylcoumarin, 7-amino-4-chloromethylcoumarin, 3-*O*-methylfluorescein, 3-O-ethyl-5-carboxyfluorescein, 2,7-difluoro-3-O-methylfluorescein, 3-N-acetyl-rhodamine, 3-*N*-acetyl-dimethylsilarhodamine, 2,7-dibromo-3-*N*-acetyl-dimethylcarborhodamine, 3-N-acetyl-6-carboxyrhodamine, 2,7-difluoro-3-*N*-acetylrhodol, *3-O-*(*N,N-*dimethyl-2-aminoethyl)-6-carboxyfluorescein, 2,7-dichloro-3-O-(N,N-dimethyl-2-aminoethyl)fluorescein, blackberry quencher (BBQ), black hole quencher 3 (BHQ3), 2-(2-hydroxyphenyl)quinazolin-4-one, 6-chloro-2-(5-chloro-2-hydroxyphenyl)quinazolin-4-one, and 6-bromo-2-(5-bromo-2-hydroxyphenyl)quinazolin-4-one.

9. A pharmaceutical or diagnostic composition comprising the compound according to any one of claims 1 to 8, or a pharmaceutically acceptable salt, solvate, or ester thereof, and optionally a pharmaceutically acceptable carrier or excipient.

10. The pharmaceutical or diagnostic composition according to claim 9, further comprising a second pharmaceutically active agent selected from a vascular disrupting agent, a cytotoxic chemotherapeutic agent and an immunomodulator.

11. The pharmaceutical or diagnostic composition according to claim 10, wherein the second pharmaceutically active agent is selected from combretastatin A-4 (CA4), 3'-aminocombretastatin A-4, BNC105, ABT-751, ZD6126, combretastatin A-1, or prodrugs of the same (which includes but is not limited to combretastatin A-4 phosphate (CA4P), 3'-aminocombretastatin A-4 3'-serinamide (ombrabulin), combretastatin A-1 bisphosphate (CA1P), and BNC105 phosphate (BNC105P)), and pharmaceutically acceptable salts, solvates or esters of the same.

12. A compound according to any one of claims 1 to 8, or a pharmaceutically acceptable salt, solvate, or ester thereof, for use in medicine.

13. A compound according to any one of claims 1 to 8, or a pharmaceutically acceptable salt, solvate, or ester thereof, for use in the treatment, amelioration, prevention or diagnosis of a disorder selected from a neoplastic disorder; atherosclerosis; an autoimmune disorder; an inflammatory disease; a chronic inflammatory autoimmune disease; ischaemia; and reperfusion injury, wherein preferably the neoplastic disorder is cancer which is preferably selected from acoustic neuroma, adenocarcinoma, angiosarcoma, basal cell carcinoma, bile duct carcinoma, bladder carcinoma, breast cancer, bronchogenic carcinoma, cervical cancer, chondrosarcoma, chordoma, choriocarcinoma, craniopharyngioma, cystadenocarcinoma, embryonal carcinoma, endotheliosarcoma, ependymoma, epithelial carcinoma, Ewing's tumor, fibrosarcoma, hemangioblastoma, leiomyosarcoma, liposarcoma, Merkel cell carcinoma, melanoma, mesothelioma, myelodysplastic syndrome, myxosarcoma, oligodendroglioma, osteogenic sarcoma, ovarian cancer, pancreatic cancer, papillary adenocarcinomas, papillary carcinoma, pinealoma, prostate cancer, renal cell carcinoma, retinoblastoma, rhabdomyosarcoma, sebaceous gland carcinoma, seminoma, squamous cell carcinoma, sweat gland carcinoma, synovioma, testicular tumor, Wilms' tumor, adrenocortical carcinoma, urothelial carcinoma, gallbladder cancer, parathyroid cancer, Kaposi sarcoma, colon carcinoma, gastrointestinal stromal tumor, anal cancer, rectal cancer, small intestine cancer, brain tumor, glioma, glioblastoma, astrocytoma, neuroblastoma, medullary carcinoma, medulloblastoma, meningioma, leukemias including acute myeloid leukemia, multiple myeloma, acute lymphoblastic leukemia, liver cancer including hepatoma and hepatocellular carcinoma, lung carcinoma, non-small-cell lung cancer, small cell lung carcinoma, lymphangioendotheliosarcoma, lymphangiosarcoma, primary CNS lymphoma, non-Hodgkin lymphoma, and classical Hodgkin's lymphoma, preferably colon cancer, rectal cancer, small intestine cancer, brain tumor, leukemia, liver cancer, lung cancer, lymphoma, basal cell carcinoma, breast cancer, cervical cancer, melanoma, ovarian cancer, pancreatic cancer, and squamous cell carcinoma.

14. A method of predicting the suitability of a compound having the formula (I) as defined in any one of claims 1 to 8, or a pharmaceutically acceptable salt, solvate, or ester thereof, for treating a patient who is suffering from a disorder selected from a neoplastic disorder; atherosclerosis; an autoimmune disorder; an inflammatory disease; a chronic inflammatory autoimmune disease; ischaemia; and reperfusion injury, wherein the method comprises:
(i) obtaining a sample from the patient;
(ii) contacting the sample with a compound having the formula (I) as defined in any one of claims 1 to 8, or a pharmaceutically acceptable salt, solvate, or ester thereof, wherein A¹ is selected such that A¹-OH is a diagnostic or theranostic agent or A² and A³ are independently selected such that A²-NH-A³ is a diagnostic or theranostic agent; and
(iii) detecting the presence or absence of A¹-OH or A²-NH-A³.

15. An *in vitro* method of determining an inhibitory activity of a candidate inhibitor or candidate drug upon an oxidoreductase and/or a redox effector protein, wherein the method comprises:
(i) contacting a compound having the formula (I) as defined in any one of claims 1 to 8, or a pharmaceutically acceptable salt, solvate, or ester thereof, wherein A¹ is selected such that A¹-OH is a diagnostic or theranostic agent or A² and A³ are independently selected such that A²-NH-A³ is a diagnostic or theranostic agent, with the oxidoreductase and/or the redox effector protein as well as the candidate inhibitor or candidate drug; and
(ii) detecting the presence or absence of A¹-OH or A²-NH-A³.

## Patentansprüche

1. Eine Verbindung mit der Formel (I)
A-L-B (I)
wobei
A dargestellt ist durch 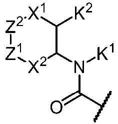 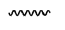
den Bindungspunkt von A an L bezeichnet;
L eine Bindung oder ein selbstimmolativer Spacer ist,
wobei der selbstimmolative Spacer ausgewählt ist aus 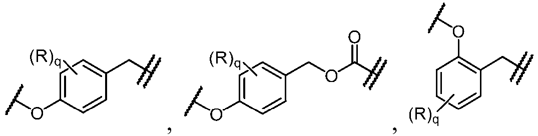 und 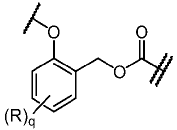 wobei
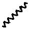 den Bindungspunkt an A bezeichnet;
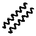 den Bindungspunkt an B bezeichnet;
R unabhängig ausgewählt ist aus Halogen, -O(R^{r}), -N(R^{s})(R^{t}), -NO₂, -CN und einer heterocyclischen Gruppe, ausgewählt aus Azetidinyl, Pyrrolidinyl, Piperidinyl oder Morpholino, wobei die heterocyclische Gruppe über das N-Atom an den Phenylring gebunden ist;
q 0, 1, 2, 3 oder 4 ist;
R^{r} unabhängig ausgewählt ist aus -H, -C₁₋₄-Alkyl und -(C₂₋₄-Alkylen)-O-(C₁₋₄-Alkyl), wobei -C₁₋₄-Alkyl oder -(C₂₋₄-Alkylen)- gegebenenfalls durch W substituiert sein kann;
R^{s} unabhängig ausgewählt ist aus -H, -C(O)-C₁₋₄-Alkyl und -C₁₋₄-Alkyl; und
R^{t} unabhängig ausgewählt ist aus -H, -C(O)-C₁₋₄-Alkyl und -C₁₋₄-Alkyl;
B durch 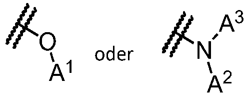 dargestellt ist; 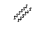
den Bindungspunkt von B an L bezeichnet;
A¹ derart ausgewählt ist, dass A¹-OH ein therapeutisches, diagnostisches oder theranostisches Mittel ist, das eine -OH-Gruppe enthält, die an einen 5- oder 6-gliedrigen aromatischen oder heteroaromatischen Ring gebunden ist;
A² und A³ unabhängig derart ausgewählt sind, dass A²-NH-A³ ein therapeutisches, diagnostisches oder theranostisches Mittel ist, das eine -NH₂- oder -NH-Gruppe enthält;
K¹ ausgewählt ist aus -C₁₋₄-Alkyl, das gegebenenfalls durch W¹ substituiert ist;
K² ausgewählt ist aus -H, -O-R^{j} und -O-R^{k}; oder
K¹ und K² aneinander gebunden sind und K¹ und K² -X³-Y-X- darstellen, wobei X³ an N gebunden ist und X an C gebunden ist;
X ausgewählt ist aus -N(R^{a})-, -N(R^{b})-, -CR^{c}₂- und -O-;
X¹ -(CR^{d}₂)m- ist;
X² -(CR^{e}₂)ₙ- ist;
X³ -CR^{f}₂- ist;
Y -(CR^{g}₂)ₚ- ist;
Z¹ und Z² unabhängig ausgewählt sind aus S oder Se, so dass entweder Z¹ Se ist und Z² S ist, oder Z¹ S ist und Z² Se ist, oder Z¹ und Z² beide Se sind;
W unabhängig ausgewählt ist aus -OH, -C(O)-N(R^{h})(Rⁱ), -N(R^{h})(Rⁱ), -PR^{x}₃⁺, -C(O)-4-(Morpholin), -C(O)-1-(Piperazin), -C(O)-1-(4-Methylpiperazin), -C(O)-1-(4-Ethylpiperazin) und einer heterocyclischen Gruppe, ausgewählt aus Azetidin-1-yl, Pyrrolidin-1-yl, Piperidin-1-yl, Piperazin-1-yl, 4-Methylpiperazin-1-yl, 4-Ethylpiperazin-1-yl, 4-(2-Hydroxyethyl)piperazin-1-yl oder Morpholino, wobei diese heterocyclische Gruppe über das N-Atom an die -C₁₋₄-Alkyl- oder -(C₂₋₄-Alkylen)-Gruppe gebunden ist;
W¹ ausgewählt ist aus -OH, -C(O)-N(R^{h})(Rⁱ), -N(R^{h})(Rⁱ), -PR^{x}₃⁺, -C(O)-4-(Morpholin), -C(O)-1-(Piperazin), -C(O)-1-(4-Methylpiperazin), -C(O)-1-(4-Ethylpiperazin) und einer heterocyclischen Gruppe, ausgewählt aus Azetidin-1-yl, Pyrrolidin-1-yl, Piperidin-1-yl, Piperazin-1-yl, 4-Methylpiperazin-1-yl, 4-Ethylpiperazin-1-yl, 4-(2-Hydroxyethyl)piperazin-1-yl oder Morpholino, wobei diese heterocyclische Gruppe über das N-Atom an die -C₁₋₄-Alkyl- oder -(C₂₋₄-Alkylen)-Gruppe gebunden ist;
R^{a} ausgewählt ist aus -H, -C₁₋₄-Alkyl, -C(O)-C₁₋₄-Alkyl, -C(O)-O-C₁₋₄-Alkyl, -C(O)-N(C₁₋₄-Alkyl)₂, -S(O)₂-C₁₋₄-Alkyl und -(C₂₋₄-Alkylen)-O-(C₁₋₄-Alkyl), wobei -C₁₋₄-Alkyl oder -(C₂₋₄-Alkylen)- gegebenenfalls durch W substituiert sein kann;
R^{b} eine Acylgruppe eines Monopeptids ist, ausgewählt aus -proteinogenen Aminosäuren, die über eine Carboxygruppe gebunden sind;
R^{c} Gruppen unabhängig ausgewählt sind aus -H und -C₁₋₄-Alkyl;
R^{d} Gruppen unabhängig ausgewählt sind aus -H und -C₁₋₄-Alkyl;
R^{e} Gruppen unabhängig ausgewählt sind aus -H und -C₁₋₄-Alkyl;
R^{f} Gruppen unabhängig ausgewählt sind aus -H und -C₁₋₄-Alkyl;
R^{g} Gruppen unabhängig ausgewählt sind aus -H und -C₁₋₄-Alkyl;
R^{h} unabhängig ausgewählt ist aus -H, -C₁₋₄-Alkyl und -CH₂CH₂OH;
Rⁱ unabhängig ausgewählt ist aus -H, -C₁₋₄-Alkyl und -CH₂CH₂OH;
R^{j} ausgewählt ist aus -H, -C₁₋₄-Alkyl, -C(O)-C₁₋₄-Alkyl, -C(O)-O-C₁₋₄-Alkyl, -C(O)-N(C₁₋₄-Alkyl)₂, -S(O)₂-C₁₋₄-Alkyl und -(C₂₋₄-Alkylen)-O-(C₁₋₄-Alkyl), wobei -C₁₋₄-Alkyl oder -(C₂₋₄-Alkylen)- gegebenenfalls durch W¹ substituiert sein kann,
R^{k} eine Acylgruppe eines Monopeptids ist, ausgewählt aus -proteinogenen Aminosäuren, die über eine Carboxygruppe gebunden sind;
R^{x} Gruppen unabhängig ausgewählt sind aus Phenyl- und 4-Methoxyphenyl-;
m 0, 1 oder 2 ist;
n 1 oder 2 ist, mit der Maßgabe, dass m+n 2 oder 3 ist;
p 0, 1 oder 2 ist, mit der Maßgabe, dass, wenn K¹ und K² aneinander gebunden sind und K¹ und K² -X³-Y-X- darstellen und X -N(R^{a})- oder -N(R^{b})- darstellt, dann p = 1 oder 2;
oder ein pharmazeutisch verträgliches Salz, Solvat oder Ester davon.

2. Die Verbindung nach Anspruch 1, wobei entweder Z¹ Se ist und Z² S ist, oder Z¹ S ist und Z² Se ist.

3. Die Verbindung nach Anspruch 1 oder 2, wobei L eine Bindung ist.

4. Die Verbindung nach Anspruch 1 oder 2,
wobei L ein selbstimmolativer Spacer ist, ausgewählt aus

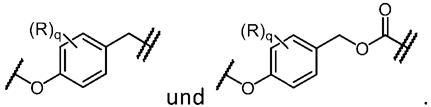

5. Die Verbindung nach einem der Ansprüche 1 bis 4, wobei X¹ und X² -CH₂- sind.

6. Die Verbindung nach einem der Ansprüche 1 bis 5, wobei K¹ und K² aneinander gebunden sind und K¹ und K² -X³-Y-X- darstellen.

7. Die Verbindung nach einem der Ansprüche 1 bis 5, wobei K¹ -C₁₋₄-Alkyl ist, das gegebenenfalls durch W¹ substituiert ist, und K² -H, -O-Rⁱ oder -O-R^{k} ist.

8. Die Verbindung nach einem der Ansprüche 1 bis 7, wobei A¹-OH oder A²-NH-A³ ausgewählt ist aus einem diagnostisch verträglichen Farbstoff, einem therapeutisch verträglichen DNA-Alkylierungsmittel, einem therapeutisch verträglichen Tubulin-Inhibitor und einem therapeutisch verträglichen Topoisomerase-Inhibitor, oder wobei A¹-OH oder A²-NH-A³ ausgewählt ist aus 10-Hydroxycamptothecin, 10-Hydroxybelotecan, 10-Hydroxygimatecan, 10-Hydroxy-CKD-602, 10-Hydroxy-BNP-1350, 10-Hydroxy-Sinotecan, Topotecan, 7-Ethyl-10-hydroxy-camptothecin (SN-38), 10-Hydroxy-20-acetoxy-camptothecin, Pyrrolobenzodiazepin, Methotrexat, Duocarmycin, CC-1065, Doxorubicin, Epirubicin, Daunorubicin, Pirarubicin, Carminomycin, Doxorubicin-N,O-Acetal, 4-(Bis(2-chlorethyl)amino)phenol, 4-(Bis(2-bromethyl)amino)phenol, 4-(Bis(2-mesylethyl)amino)phenol, 4-((2-Chlorethyl-2'-mesylethyl)amino)phenol, 5-Hydroxy-seco-cyclopropabenzaindolen, 5-Hydroxy-seco-(2-methyl-cyclopropa)benzaindolen, 5-Hydroxy-seco-cyclopropamethoxybenzaindolen, 5-Amino-seco-cyclopropabenzaindolen, Etoposid, Teniposid, GL331, NPF, TOP53, NK611, Tubulysin A, Tubulysin B, Tubulysin C, Tubulysin G, Tubulysin I, Monomethyl-Auristatin E, Monomethyl-Auristatin F, Dolastatin 10, Dolastatin 15, Symplostastin 1, Symplostastin 3, Narciclasin, Pancratistatin, 2-Epi-Narciclasin, Narciprimin, Calicheamicin α1, Calicheamicin β1, Calicheamicin γ1, Calicheamicin δ1, Calicheamicin ε, Calicheamicin θ, Calicheamicin T, Diclofenac, Aceclofenac, Mefenaminsäure, Clonixin, Piroxicam, Meloxicam, Tenoxicam, Lornoxicam, Baricitinib, Filgotinib, Tofacitinib, Upadacitinib, Ruxolitinib, Peficitinib, Decemotinib, Solcitinib, Itacitinib, Fostamatinib, SHR0302, Leuko-Methylenblau, Leuko-Methyl-Methylenblau, Leuko-Dimethyl-Methylenblau, Leuko-Toluidinblau, Leuko-Azur A, Leuko-Azur B, Leuko-Azur C, Leuko-Thionin, Leuko-Methylenviolett, Leuko-Neu-Methylenblau, Leuko-Nilblau A, Leuko-Brilliant-Kresylblau, Glühwürmchen-Luciferin (D-Luciferin), Umbelliferon, 4-Trifluormethylumbelliferon, 6,8-Difluor-4-methylumbelliferon, 7-Hydroxycumarin-3-carbonsäure, 6,8-Difluor-7-hydroxy-5-methylcumarin (DiFMU), 7-Amino-4-methylcumarin, 7-Amino-4-chlormethylcumarin, 3-O-Methylfluorescein, 3-O-Ethyl-5-carboxyfluorescein, 2,7-Difluor-3-O-methylfluorescein, 3-N-Acetyl-Rhodamin, 3-N-Acetyl-dimethylsilarhodamin, 2,7-Dibrom-3-N-acetyl-dimethylcarbo-rhodamin, 3-N-Acetyl-6-carboxyrhodamin, 2,7-Difluor-3-N-acetylrhodol, 3-O-(N,N-Dimethyl-2-aminoethyl)-6-carboxyfluorescein, 2,7-Dichlor-3-O-(N,N-Dimethyl-2-amino-ethyl)fluorescein, Blackberry Quencher (BBQ), Black-Hole-Quencher 3 (BHQ3), 2-(2-Hydroxyphenyl)chinazolin-4-on, 6-Chlor-2-(5-chlor-2-hydroxyphenyl)chinazolin-4-on und 6-Brom-2-(5-brom-2-hydroxyphenyl)chinazolin-4-on.

9. Eine pharmazeutische oder diagnostische Zusammensetzung, umfassend die Verbindung nach einem der Ansprüche 1 bis 8 oder ein pharmazeutisch verträgliches Salz, Solvat oder einen pharmazeutisch verträglichen Ester davon und gegebenenfalls einen pharmazeutisch verträglichen Träger oder Exzipienten.

10. Die pharmazeutische oder diagnostische Zusammensetzung nach Anspruch 9, ferner umfassend einen zweiten pharmazeutischen Wirkstoff, ausgewählt aus einem vaskulären Disruptor, einem zytotoxischen Chemotherapeutikum und einem Immunmodulator.

11. Die pharmazeutische oder diagnostische Zusammensetzung nach Anspruch 10, wobei der zweite pharmazeutische Wirkstoff ausgewählt ist aus Combretastatin A-4 (CA4), 3'-Aminocombretastatin A-4, BNC105, ABT-751, ZD6126, Combretastatin A-1 oder Prodrugs davon (welches Combretastatin A-4-Phosphat (CA4P), 3'-Aminocombretastatin A-4-3'-Serinamid (Ombrabulin), Combretastatin A-1-Bisphosphat (CA1P) und BNC105-Phosphat (BNC105P) beinhaltet, aber nicht darauf beschränkt ist) und pharmazeutisch verträgliche Salze, Solvate oder Ester davon.

12. Eine Verbindung nach einem der Ansprüche 1 bis 8 oder ein pharmazeutisch verträgliches Salz, Solvat oder einen pharmazeutisch verträglichen Ester davon zur Verwendung in der Medizin.

13. Eine Verbindung nach einem der Ansprüche 1 bis 8 oder ein pharmazeutisch verträgliches Salz, Solvat oder einen pharmazeutisch verträglichen Ester davon zur Verwendung bei der Behandlung, Linderung, Vorbeugung oder Diagnose einer Erkrankung, ausgewählt aus einer neoplastischen Erkrankung; Atherosklerose; einer Autoimmunerkrankung; einer entzündlichen Erkrankung; einer chronisch-entzündlichen Autoimmunerkrankung; Ischämie; und Reperfusionsschaden, wobei die neoplastische Erkrankung vorzugsweise Krebs ist, der vorzugsweise ausgewählt ist aus Akustikusneurinom, Adenokarzinom, Angiosarkom, Basalzellkarzinom, Gallengangskarzinom, Blasenkarzinom, Brustkrebs, Bronchialkarzinom, Gebärmutterhalskrebs, Chondrosarkom, Chordom, Choriokarzinom, Kraniopharyngeom, Zystadenokarzinom, Embryonalkarzinom, Endotheliosarkom, Ependymom, Epithelkarzinom, Ewing-Tumor, Fibrosarkom, Hämangioblastom, Leiomyosarkom, Liposarkom, Merkelzellkarzinom, Melanom, Mesotheliom, myelodysplastischem Syndrom, Myxosarkom, Oligodendrogliom, osteogenem Sarkom, Eierstockkrebs, Bauchspeicheldrüsenkrebs, papillären Adenokarzinomen, papillärem Karzinom, Pinealom, Prostatakrebs, Nierenzellkarzinom, Retinoblastom, Rhabdomyosarkom, Talgdrüsenkarzinom, Seminom, Plattenepithelkarzinom, Schweißdrüsenkarzinom, Synoviom, Hodentumor, Wilms-Tumor, Nebennierenrindenkarzinom, Urothelkarzinom, Gallenblasenkrebs, Nebenschilddrüsenkrebs, Kaposi-Sarkom, Kolonkarzinom, gastrointestinalem Stromatumor, Analkrebs, Rektumkarzinom, Dünndarmkrebs, Hirntumor, Gliom, Glioblastom, Astrozytom, Neuroblastom, medullärem Karzinom, Medulloblastom, Meningeom, Leukämien einschließlich akuter myeloischer Leukämie, Multiplem Myelom, akuter lymphoblastischer Leukämie, Leberkrebs einschließlich Hepatom und hepatozellulärem Karzinom, Lungenkarzinom, nicht-kleinzelligem Lungenkarzinom, kleinzelligem Lungenkarzinom, Lymphangioendotheliosarkom, Lymphangiosarkom, primäres ZNS-Lymphom, Non-Hodgkin-Lymphom und klassischem Hodgkin-Lymphom, vorzugsweise Dickdarmkrebs, Rektumkrebs, Dünndarmkrebs, Hirntumor, Leukämie, Leberkrebs, Lungenkrebs, Lymphom, Basalzellkarzinom, Brustkrebs, Gebärmutterhalskrebs, Melanom, Eierstockkrebs, Bauchspeicheldrüsenkrebs und Plattenepithelkarzinom.

14. Ein Verfahren zur Vorhersage der Eignung einer Verbindung mit der in einem der Ansprüche 1 bis 8 definierten Formel (I) oder eines pharmazeutisch verträglichen Salzes, Solvats oder Esters davon zur Behandlung eines Patienten, der an einer Erkrankung leidet, ausgewählt aus einer neoplastischen Erkrankung; Atherosklerose; einer Autoimmunerkrankung; einer entzündlichen Erkrankung; einer chronisch-entzündlichen Autoimmunerkrankung; Ischämie; und Reperfusionsschaden, wobei das Verfahren umfasst:
(i) Entnahme einer Probe von dem Patienten;
(ii) Inkontaktbringen der Probe mit einer Verbindung mit der in einem der Ansprüche 1 bis 8 definierten Formel (I) oder einem pharmazeutisch verträglichen Salz, Solvat oder Ester davon, wobei A¹ derart ausgewählt ist, dass A¹-OH ein diagnostisches oder theranostisches Mittel ist, oder A² und A³ unabhängig derart ausgewählt sind, dass A²-NH-A³ ein diagnostisches oder theranostisches Mittel ist; und
(iii) Nachweis des Vorhandenseins oder Fehlens von A¹-OH oder A²-NH-A³.

15. Ein *in* vitro-Verfahren zur Bestimmung einer inhibitorischen Aktivität eines Inhibitor-Kandidaten oder eines Arzneimittel-Kandidaten gegenüber einer Oxidoreduktase und/oder einem Redox-Effektorprotein, wobei das Verfahren umfasst:
(i) Inkontaktbringen einer Verbindung mit der in einem der Ansprüche 1 bis 8 definierten Formel (I) oder eines pharmazeutisch verträglichen Salzes, Solvats oder Esters davon, wobei A¹ derart ausgewählt ist, dass A¹-OH ein diagnostisches oder theranostisches Mittel ist, oder A² und A³ unabhängig derart ausgewählt sind, dass A²-NH-A³ ein diagnostisches oder theranostisches Mittel ist, mit der Oxidoreduktase und/oder dem Redox-Effektorprotein sowie dem Inhibitor-Kandidaten oder Arzneimittel-Kandidaten; und
(ii) Nachweis des Vorhandenseins oder Fehlens von A¹-OH oder A²-NH-A³.

## Revendications

1. Composé de formule (I)
A-L-B (I)
dans lequel
A est représenté par 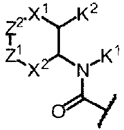
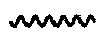 désigne le point de rattachement de A à L ;
L est une liaison ou un espaceur auto-immolable,
dans lequel l'espaceur auto-immolable est choisi parmi 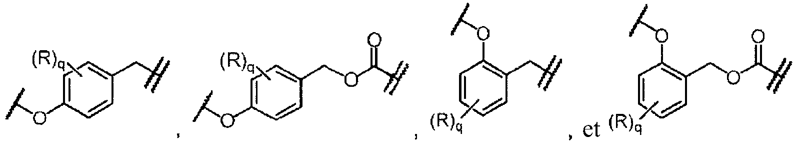
dans lesquels
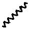 désigne le point de rattachement à A ;
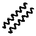 désigne le point de rattachement à B ;
R est indépendamment choisi parmi un halogène, -O(R^{r}), -N(R^{s})(R^{t}), -NO₂, -CN, et un groupe hétérocyclique choisi parmi azétidinyle, pyrrolidinyle, pipéridinyle ou morpholino, dans lesquels le groupe hétérocyclique est rattaché au cycle phényle via l'atome N ;
q vaut 0, 1, 2, 3 ou 4 ;
R^{f} est indépendamment choisi parmi -H, -alkyle en C₁ à C₄ et -(alkylène en C₂ à C₄)-O-(alkyle en C₁ à C₄), dans lesquels le -alkyle en C₁ à C₄ ou -(alkylène en C₂ à C₄) peut être éventuellement substitué par W ;
R^{s} est indépendamment choisi parmi -H, -C(O)-alkyle en C₁ à C₄ et -alkyle en C₁ à C₄ ; et
R^{t} est indépendamment choisi parmi -H, -C(O)-alkyle en C₁ à C₄ et -alkyle en C₁ à C₄ ;
B est représenté par 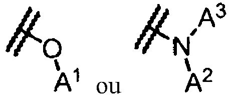 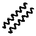
désigne le point de rattachement à B à L ;
A¹ est choisi de façon que A¹-OH soit un agent thérapeutique, diagnostique ou théranostique qui contient un groupe -OH qui est rattaché à un cycle hétéroaromatique ou aromatique à 5 ou 6 chaînons ;
A² et A³ sont indépendamment choisis de façon que A²-NH-A³ soit un agent thérapeutique, diagnostique ou théranostique qui contient un fragment -NH₂ ou -NH- ;
K¹ est choisi parmi un -alkyle en C₁ à C₄ éventuellement substitué par W¹ ;
K² est choisi parmi -H, -O-R^{j} et -O-R^{k} ; ou
K¹ et K² sont liés l'un à l'autre et K¹ et K² représentent -X³-Y-X- dans lequel X³ est lié à N et X est lié à C ;
X est choisi parmi -N(R^{a})-, -N(R^{b})-, -CR^{c}₂ et -O- ;
X¹ est -(CR^{d}₂)ₘ- ;
X² est -(CR^{e}₂)ₙ₋ ;
X³ est -CR^{f}₂- ;
Y est -(CR^{g}₂)ₚ- ;
Z¹ et Z² sont indépendamment choisis parmi S et Se de façon que soit Z¹ soit Se et Z² soit S, soit Z¹ soit S et Z² soit Se, ou soit Z¹ et Z² soient tous deux Se ;
W est indépendamment choisi parmi -OH, -C(O)-N(R^{h})(Rⁱ), -N(R^{h})(Rⁱ), -PR^{x}₃⁺, -C(O)-4-(morpholine), -C(O)-1-(pipérazine), -C(O)-1-(4-méthylpipérazine), -C(O)-1-(4-éthylpipérazine), et un groupe hétérocyclique choisi parmi azétidin-1-yle, pyrrolidin-1-yle, pipéridin-1-yle, pipérazin-1-yle, 4-méthylpipérazine-1-yle, 4-éthylpipérazin-1-yle, 4-(2-hydroxyéthyl)pipérazin-1-yle, ou morpholino, dans lesquels ce groupe hétérocyclique est rattaché au groupe -(alkylène en C₂ à C₄)- ou -alkyle en C₁ à C₄ via l'atome N ;
W¹ est choisi parmi -OH, -C(O)-N(R^{h})(Rⁱ), -N(R^{h})(Rⁱ), -PR^{x}₃⁺, -C(O)-4-(morpholine), -C(O)-1-(pipérazine), -C(O)-1-(4-méthylpipérazine), -C(O)-1-(4-éthylpipérazine), et un groupe hétérocyclique choisi parmi azétidin-1-yle, pyrrolidin-1-yle, pipéridin-1-yle, pipérazin-1-yle, 4-méthylpipérazine-1-yle, 4-éthylpipérazin-1-yle, 4-(2-hydroxyéthyl)pipérazin-1-yle, ou morpholino, dans lesquels ce groupe hétérocyclique est rattaché au groupe -(alkylène en C₂ à C₄)- ou -alkyle en C₁ à C₄ via l'atome N ;
R^{a} est choisi parmi -H, -alkyle en C₁ à C₄, -C(O)-alkyle en C₁ à C₄, -C(O)-O-alkyle en C₁ à C₄, -C(O)-N(alkyle en C₁ à C₄)₂, -S(O)₂-alkyle en C₁ à C₄ et -(alkylène en C₂ à C₄)-O-(alkyle en C₁ à C₄), dans lesquels le -alkyle en C₁ à C₄ ou -(alkylène en C₂ à C₄)- peut être éventuellement substitué par W ;
R^{b} est un groupe acyle d'un monopeptide choisi parmi les -acides aminés protéinogènes rattachés via un groupe carboxy ;
les groupes R^{c} sont indépendamment choisis parmi -H et -alkyle en C₁ à C₄ ;
les groupes R^{d} sont indépendamment choisis parmi -H et -alkyle en C₁ à C₄ ;
les groupes R^{e} sont indépendamment choisis parmi -H et -alkyle en C₁ à C₄ ;
les groupes R^{f} sont indépendamment choisis parmi -H et -alkyle en C₁ à C₄ ;
les groupes R^{g} sont indépendamment choisis parmi -H et -alkyle en C₁ à C₄ ;
R^{h} est indépendamment choisi parmi -H, -alkyle en C₁ à C₄ et -CH₂CH₂OH ;
Rⁱ est indépendamment choisi parmi -H, -alkyle en C₁ à C₄ et -CH₂CH₂OH ;
R^{j} est choisi parmi -H, -alkyle en C₁ à C₄, -C(O)-alkyle en C₁ à C₄, -C(O)-O-alkyle en C₁ à C₄, -C(O)-N(alkyle en C₁ à C₄)₂, -S(O)₂-alkyle en C₁ à C₄ et -(alkylène en C₂ à C₄)-O-(alkyle en C₁ à C₄), dans lesquels le -alkyle en C₁ à C₄ ou -(alkylène en C₂ à C₄)- peut être éventuellement substitué par W¹ ;
R^{k} est un groupe acyle d'un monopeptide choisi parmi les -acides aminés protéinogènes rattachés via un groupe carboxy ;
les groupes R^{x} sont indépendamment choisis parmi phényl- et 4-méthoxyphényl- ;
m vaut 0, 1 ou 2 ;
n vaut 1 ou 2, sous réserve que m+n vaille 2 ou 3 ;
p vaut 0, 1 ou 2, sous réserve que, lorsque K¹ et K² sont liés l'un à l'autre et K¹ et K² représentent -X³-Y-X- et X représente -N(R^{a})- ou -N(R^{b})-, alors p = 1 ou 2 ;
ou n'importe quel ester, solvate ou sel pharmaceutiquement acceptable d'un tel composé.

2. Composé selon la revendication 1, dans lequel soit Z¹ est Se et Z² est S, soit Z¹ est S et Z² est Se.

3. Composé selon la revendication 1 ou 2, dans lequel L est une liaison.

4. Composé selon la revendication 1 ou 2, dans lequel L est un espaceur auto-immolable choisi parmi

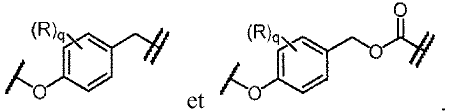

5. Composé selon l'une quelconque des revendications 1 à 4, dans lequel X¹ et X² sont -CH₂-.

6. Composé selon l'une quelconque des revendications 1 à 5, dans lequel K¹ et K² sont liés l'un à l'autre et K¹ et K² représentent -X³-Y-X-.

7. Composé selon l'une quelconque des revendications 1 à 5, dans lequel K¹ est un -alkyle en C₁ à C₄ éventuellement substitué par W¹ et K² est -H, -O-R^{j} ou -O-R^{k}.

8. Composé selon l'une quelconque des revendications 1 à 7, dans lequel A¹-OH ou A²-NH-A³ est choisi parmi un colorant acceptable en diagnostic, un agent d'alkylation d'ADN acceptable en thérapie, un agent inhibiteur de tubuline acceptable en thérapie, et un agent inhibiteur de topoisomérase acceptable en thérapie, ou dans lequel A¹-OH ou A²-NH-A³ est choisi parmi la 10-hydroxycamptothécine, le 10-hydroxybélotécan, le 10-hydroxygimatécan, le 10-hydroxy-CKD-602, le 10-hydroxy-BNP-1350, le 10-hydroxy-sinotécan, le topotécan, la 7-éthyl-10-hydroxycamptothécine (SN-38), la 10-hydroxy-20-acétoxy-camptothécine, la pyrrolobenzodiazépine, le méthotrexate, la duocarmycine, CC-1065, la doxorubicine, l'épirubicine, la daunorubicine, la pirarubicine, la carminomycine, le doxorubicine-N,O-acétal, le 4-(bis(2-chloroéthyl)amino)phénol, le 4-(bis(2-bromoéthyl)amino)phénol, le 4-(bis(2-mésyléthyl)amino)phénol, le 4-((2-chloroéthyl-2'-mésyléthyl)amino)phénol, les 5-hydroxy-séco-cyclopropabenzaindoles, les 5-hydroxy-séco-(2-méthyl-cyclopropa)benzaindoles, les 5-hydroxy-séco-cyclopropaméthoxybenzaindoles, les 5-amino-séco-cyclopropabenzaindoles, l'étoposide, le téniposide, GL331, NPF, TOP53, NK611, la tubulysine A, la tubulysine B, la tubulysine C, la tubulysine G, la tubulysine I, la monométhyl-auristatine E, la monométhyl-auristatine F, la dolastatine 10, la dolastatine 15, la symplostatine 1, la symplostatine 3, la narciclasine, la pancratistatine, la 2-épi-narciclasine, la narciprimine, la calichéamicine α1, la calichéamicine β1, la calichéamicine γ1, la calichéamicine δ1, la calichéamicine ε, la calichéamicine θ, la calichéamicine T, le diclofénac, l'acéclofénac, l'acide méfénamique, la clonixine, le piroxicam, le méloxicam, le ténoxicam, le lornoxicam, le baricitinib, le filgotinib, le tofacitinib, l'upadacitinib, le ruxolitinib, le péficitinib, le décémotinib, le solcitinib, l'itacitinib, le fostamatinib, SHR0302, le leuco-bleu de méthylène, le leuco-bleu de méthyl-méthylène, le leuco-bleu de diméthyl-méthylène, le leuco-bleu de toluidine, le leuco-Azure A, le leuco-Azure B, le leuco-Azure C, la leuco-thionine, le leuco-violet de méthylène, le leuco-nouveau bleu de méthylène, le leuco-bleu Nil A, le leuco-bleu de crésyle brillant, la luciférine de luciole (D-luciférine), l'umbelliférone, la 4-trifluorométhylumbelliférone, la 6,8-difluoro-4-méthylumbelliférone, l'acide 7-hydroxycoumarine-3-carboxylique, la 6,8-difluoro-7-hydroxy-5-méthylcoumarine (DiFMU), la 7-amino-4-méthylcoumarine, la 7-amino-4-chlorométhylcoumarine, la 3-O-méthylfluorescéine, la 3-O-éthyl-5-carboxyfluorescéine, la 2,7-difluoro-3-*O-*méthylfluorescéine, la 3-*N*-acétylrhodamine, la 3-*N*-acétyl-diméthylsilarhodamine, la 2,7-dibromo-3-*N*-acétyl-diméthylcarborhodamine, la 3-*N*-acétyl-6-carboxyrhodamine, le 2,7-difluoro-3-*N*-acétylrhodol, la 3-*O*-(*N*,*N*-diméthyl-2-aminoéthyl)-6-carboxyfluorescéine, la 2,7-dichloro-3-*O*-(*N*,*N*-diméthyl-2-aminoéthyl)fluorescéine, le Blackberry Quencher (BBQ), le Black Hole Quencher 3 (BHQ3), la 2-(2-hydroxyphényl)quinazolin-4-one, la 6-chloro-2-(5-chloro-2-hydroxyphényl)quinazolin-4-one, et la 6-bromo-2-(5-bromo-2-hydroxyphényl)quinazolin-4-one.

9. Composition pharmaceutique ou diagnostique comprenant le composé selon l'une quelconque des revendications 1 à 8, ou un ester, solvate ou sel pharmaceutiquement acceptable d'un tel composé, et éventuellement un véhicule ou excipient pharmaceutiquement acceptable.

10. Composition pharmaceutique ou diagnostique selon la revendication 9, comprenant en outre un deuxième principe actif pharmaceutique choisi parmi un agent de perturbation vasculaire, un agent chimiothérapeutique cytotoxique et un immunomodulateur.

11. Composition pharmaceutique ou diagnostique selon la revendication 10, dans laquelle le deuxième principe actif pharmaceutique est choisi parmi la combrétastatine A-4 (CA4), la 3'-aminocombrétastatine A-4, BNC105, ABT-751, ZD6126, la combrétastatine A-1, ou leurs promédicaments (qui englobent, mais sans s'y limiter, le phosphate de combrétastatine A-4 (CA4P), le 3'-sérinamide de 3'-aminocombrétastatine A-4 (ombrabuline), le bisphosphate de combrétastatine A-1 (CA1P) et le phosphate de BNC105 (BNC105P)), ainsi que leurs esters, solvates et sels pharmaceutiquement acceptables.

12. Composé selon l'une quelconque des revendications 1 à 8 ou un ester, solvate ou sel pharmaceutiquement acceptable d'un tel composé, pour son utilisation en médecine.

13. Composé selon l'une quelconque des revendications 1 à 8 ou un ester, solvate ou sel pharmaceutiquement acceptable d'un tel composé, pour son utilisation dans le traitement, l'amélioration, la prévention ou le diagnostic d'un trouble choisi parmi un trouble néoplasique ; une athérosclérose ; un trouble auto-immun ; une maladie inflammatoire ; une maladie auto-immune inflammatoire chronique ; une ischémie ; et une lésion par reperfusion, dans lequel de préférence le trouble néoplasique est un cancer qui est de préférence choisi parmi un neurinome de l'acoustique, un adénocarcinome, un angiosarcome, un carcinome à cellules basales, un carcinome des voies biliaires, un carcinome de la vessie, un cancer du sein, un carcinome bronchogénique, un cancer du col de l'utérus, un chondrosarcome, un chordome, un choriocarcinome, un craniopharyngiome, un cystadénocarcinome, un carcinome embryonnaire, un endothéliosarcome, un épendymome, un carcinome épithélial, une tumeur d'Ewing, un fibrosarcome, un hémangioblastome, un léïomyosarcome, un liposarcome, un carcinome à cellules de Merkel, un mélanome, un mésothéliome, un syndrome myélodysplasique, un myxosarcome, un oligodendrogliome, un sarcome ostéogénique, un cancer ovarien, un cancer pancréatique, des adénocarcinomes papillaires, un carcinome papillaire, un pinéalome, un cancer de la prostate, un carcinome à cellules rénales, un rétinoblastome, un rhabdomyosarcome, un carcinome des glandes sébacées, un séminome, un carcinome à cellules squameuses, un carcinome des glandes sudoripares, un synoviome, une tumeur testiculaire, une tumeur de Wilms, un carcinome corticosurrénalien, un carcinome urothélial, un cancer de la vésicule biliaire, un cancer parathyroïdien, un sarcome de Kaposi, un carcinome du côlon, une tumeur stromale gastro-intestinale, un cancer anal, un cancer rectal, un cancer de l'intestin grêle, une tumeur cérébrale, un gliome, un glioblastome, un astrocytome, un neuroblastome, un carcinome médullaire, un médulloblastome, un méningiome, des leucémies y compris une leucémie myéloïde aiguë, un myélome multiple, une leucémie lymphoblastique aiguë, un cancer du foie y compris un hépatome et un carcinome hépatocellulaire, un carcinome pulmonaire, un cancer du poumon non à petites cellules, un carcinome pulmonaire à petites cellules, un lymphangioendothéliosarcome, un lymphangiosarcome, un lymphome du CNS primaire, un lymphome non Hodgkinien, et un lymphome Hodgkinien classique, de préférence un cancer du côlon, un cancer rectal, un cancer de l'intestin grêle, une tumeur cérébrale, une leucémie, un cancer du foie, un cancer du poumon, un lymphome, un carcinome à cellules basales, un cancer du sein, un cancer du col de l'utérus, un mélanome, un cancer ovarien, un cancer pancréatique, et un carcinome à cellules squameuses.

14. Méthode de prédiction de la pertinence d'un composé de formule (I) tel que défini selon l'une quelconque des revendications 1 à 8, ou d'un ester, solvate ou sel pharmaceutiquement acceptable d'un tel composé, pour traiter un patient qui souffre d'un trouble choisi parmi un trouble néoplasique ; une athérosclérose ; un trouble auto-immun ; une maladie inflammatoire ; une maladie auto-immune inflammatoire chronique ; une ischémie ; et une lésion par reperfusion, dans laquelle la méthode comprend :
(i) l'obtention d'un échantillon provenant du patient ;
(ii) la mise en contact de l'échantillon avec un composé de formule (I) tel que défini selon l'une quelconque des revendications 1 à 8, ou un ester, solvate ou sel pharmaceutiquement acceptable d'un tel composé, dans lequel A¹ est choisi de façon que A¹-OH soit un agent diagnostique ou théranostique ou A² et A³ sont indépendamment choisis de façon que A²-NH-A³ soit un agent diagnostique ou théranostique ; et
(iii) la détection de la présence ou de l'absence de A¹-OH ou de A²-NH-A³.

15. Méthode *in vitro* de détermination d'une activité inhibitrice d'un inhibiteur candidat ou d'un médicament candidat sur une oxydoréductase et/ou une protéine effectrice rédox, dans laquelle la méthode comprend :
(i) la mise en contact d'un composé de formule (I) tel que défini selon l'une quelconque des revendications 1 à 8, ou d'un ester, solvate ou sel pharmaceutiquement acceptable d'un tel composé, dans lequel A¹ est choisi de façon que A¹-OH soit un agent diagnostique ou théranostique ou A² et A³ sont indépendamment choisis de façon que A²-NH-A³ soit un agent diagnostique ou théranostique, avec l'oxydoréductase et/ou la protéine effectrice rédox ainsi que l'inhibiteur candidat ou le médicament candidat ; et
(ii) la détection de la présence ou de l'absence de A¹-OH ou de A²-NH-A³.
